# EUROPEAN PATENT APPLICATION

(11) **EP 4 656 247 A2**
(43) Date of publication of application: **03.12.2025**
(21) Application number: 25194341.1
(22) Date of filing: 03.04.2019
(51) Int. Cl.: A61P 35/00

(54) **ANTI-CHEMOKIN LIKE RECEPTOR 1 ANTIBODIES AND THEIR THERAPEUTIC APPLICATIONS**

(30) Priority: 03.04.2018 EP 18305395
(62) Divisional of application: 19715092.3
(71) Applicant: OSE IMMUNOTHERAPEUTICS, 44200 Nantes (FR)
(72) Inventor: POIRIER, Nicolas, 44119 GRANDCHAMPS DES FONTAINES (FR); MARY, Caroline, 44680 SAINTE-PAZANNE (FR); VANHOVE, Bernard, 44400 REZE (FR); GAUTTIER, Vanessa, 44400 REZE (FR); TRILLEAUD, Charlène, 44400 REZE (FR); DUBOURDEAU, Marc, 31240 SAINT-JEAN (FR)
(74) Representative: Hoffmann Eitle

(57) **Abstract**

The present invention provides anti-CMKLR1 compounds having an agonist capability on the interaction between Resolvin E1 and CMKLR1, and their uses for treating or preventing a disease, in particular wherein the resolution of inflammation is delayed or disrupted.

## Description

### Field of the invention

The invention pertains to the field of immunotherapy. The present invention provides new anti-chemerin receptor antibodies which have an agonist activity on chemokine like receptor-1 (CMKLR1). The present invention also provides uses of such an antibody in therapy, in particular for treating autoimmune diseases and chronic inflammatory diseases, infectious diseases, cancers, and any condition wherein the resolution phase of inflammation is disrupted or delayed.

### Background of the invention

The critical role of inflammatory processes in health and diseases has long been recognized. The detailed molecular mechanisms and biological events that regulate the progression and the resolution of inflammation remain of critical interest. Recent investigations have provided strong evidence that the resolution of inflammation is not a passive process, as believed earlier. Resolution of the inflammation is instead a biosynthetically active process, regulated by biochemical mediators and receptors-signaling pathways. Resolution is therefore driven by specialized pro-resolving mediators. Inflammation is a spontaneous mechanism that occurs during an infection, an injury or a traumatism. Inflammation is inevitable and usually salutary, and its response is orchestrated by a delicate balance between positive and negative feedback loops. The inflammation is usually divided in 3 steps: initiation, amplification and resolution.

The initiation step is characterized by a vasodilatation of blood vessel. Resident cells (Dendritic Cells (DCs) and Macrophages) recognize the pathogen infecting the body or danger signals. This step induces the secretion of cytokines, chemokines and the production of pro-inflammatory lipid mediators named Prostaglandins (PG) and leukotrienes. Chemokines return in blood circulation and induce recruitment of cellular actors of innate immunity. The amplification step begins with the recruitment of cells of inflammation of the immune system. The first actors recruited to the site of infection are the Polymorphonuclears (PMN). During the cellular phase the PMN recognize the pathogen and are in charge of its elimination. Other cells travel to the inflamed site for helping the PMN such as DCs and the pro-inflammatory macrophages M1. DCs go in the blood circulation and reach the lymph node to activate the adaptive immunity. Lymphocytes T and B reach the site of inflammation and eradicate the infected cells. When the pathogen is completely eliminated, it's become primordial that inflammation is stopped to prevent chronic inflammation that would become pathogenic for organism. To do so, an active mechanism named Resolution of the inflammation takes place. Resolution is accordingly described as the last step of inflammation.

The resolution process which allows the ending of the inflammatory response is a complex process involving the sequential and chronological engagement of cellular (e.g. granulocytes or macrophages) and chemical (e.g. cytokines or specialized pro-solving mediators or factors) effectors.

A resolution defect can result in an increased penetration of granulocytes at the inflammatory site (measured for example by histology, cytometry or indirect biochemical techniques such as elastase quantification by enzyme immunoassay or molecular quantification by PCR of granulocyte receptor 1), a delay in the apoptosis of such cells (measured for example by cytology using specific antibodies against annexin 5). A defect in the resolution of the inflammation may also result in sustained or increased synthesis of pro-inflammatory cytokines such as TNF-alpha, IL8 or IL12 and a decrease of anti-inflammatory cytokines such as IL-10 (measured by enzyme immunoassay or by PCR), sustained or increased activation of transcription factors involved in the synthesis of inflammatory cytokines such as NF-kappaB (measured for example by nuclear translocation or by Western blot and quantification of the level of degradation of IkappaB). It can also be measured by quantification of specialized pro-resolving mediators (such as lipoxins, resolvins, protectins or maresins) or their precursors (like 17-HDOHE or 14-HDOHE) by mass spectrometry or enzyme immunoassay. A defect of resolution then results in a defect of the synthesis of one or more of these mediators. A resolution defect can also result from a decrease of the expression of the receptors of the resolution molecules (ALX / CMK1R1, GPR32, GPR18) or internalization and processing of those receptors into the cytoplasm or to an overexpression of some receptors of inflammatory cytokines or lipids. These conditions may be measured by histology, cytology or PCR. The resolution defect can also result in a decreased or inhibited switch of M1 to M2 macrophages, a damage in phagocytosis or efferocytosis of the same cells.

It is now clear that a family of chemicals actively promotes resolution and tissue repair of inflammation without compromising host defense in addition to checkpoints activator and/or inhibitors involved in such mechanism. Events at the onset of acute inflammation establish biosynthetic pathways for a series of chemical mediators that could serve as antagonists as well as agonists meaning that they do not merely inhibit inflammation pathways but they strike inflammation leading to the restoration of tissue homeostasis and function. Anti-inflammation and pro-resolution factors are therefore not equivalent (Buckley et al., 2014) (Serhan, 2014a). Failure in the resolution of acute inflammation participates in chronic inflammation development. Anti-inflammatory compounds accordingly refer to inhibitors or blockers of the resolution of inflammation, as do molecules stopping immune extravasation; while pro-resolving factors stimulate and/or activate specific processes such as apoptosis or efferocytosis which initiate or enhance the resolution of the inflammation.

Resolution is initiated shortly after the beginning of the inflammatory response by the PMN, it eliminates the pathogen and in parallel starts the synthesis of specialized pro-resolving mediators (SPM). These SPM are the main actors of the resolution phase. Neutrophils govern the initiation of the resolution phase of inflammation by enabling activation of pro-resolving circuits to ensure safe conclusion of the inflammatory response. In the early stage of resolution, neutrophils undergo a phenotype switch to produce different profiles of lipid mediators depending on the cells and substrates present in the immediate environment. PMN-LT switch into PMN-LO (lipoxygenase) pathway under the action of lipoxin and Resolvins. They are exposed to autacoid gradient initiating phenotypic changes. Lipoxins are generated via biosynthetic route engaged during cell-cell interaction (PMN-5-LO/ tissue resident cell - 5-LO). It has been observed that wound healing is delayed in neutrophil deletion model, and that neutrophils release proteases that deactivate inflammatory cytokines.

Various molecules are involved in the initiation or the inhibition of the resolution of inflammation. The following molecules are illustrative of such active molecules. COX-2 (Cyclooxygenase-2), a prostaglandin-endoperoxide synthase (PTGS), is an enzyme responsible for formation of prostanoids, including thromboxane and prostaglandins such as prostacyclin, and has a dual role as a contributor to the onset of inflammation, and later as a helper to resolve the process. COX-2 inhibitors may have a benefic effect in the early phase of inflammation. COX-2 inhibitors have also deleterious consequences, such as decreasing early PMN trafficking, disrupting the production of LXA, decreasing macrophage phagocytosis, and reducing PGE2 and LXA. An anti-inflammatory prostaglandin called PGD2/15dPGJ is also involved in the negative feedback, but other prostaglandins, like PGE2, are involved in the positive feedback during the resolution of the inflammation.

G protein-coupled receptors (GPCRs) constitute a vast protein family that encompasses a wide range of functions, including various autocrine, paracrine and endocrine processes. They show considerable diversity at the sequence level, on the basis of which they can be separated into distinct groups. Typically, GPCRs activation engages broad networks of signaling pathways which are mediated by either G proteins or B-arrestin or both.

Chemokine-like receptor 1 (CMKLR1), also known as ChemR23, and chemokine receptor-like 2 (CCRL2) are 7-transmembrane receptors identified by their homology to known G-protein-coupled receptors (AJ Kennedy and AP Davenport, 2018). The Chemokine-like Receptor 1 (CMKLR1; also named Dez in Murine animals), is an orphan G protein-coupled receptor related to GPR-1 (38% overall amino acid identity), C3a receptor (38%), C5a anaphylatoxin receptor (36%) and formyl Met-Leu-Phe receptors (35%). ChemR23 is more distantly related to the chemokine receptors subfamily (Samson et al., 1998). CMKLR1 is expressed on monocytes, on macrophages, on dendritic cells, and NK on cells, as well as on adipocytes and endothelial cells. CMKLR1 expression was also described in many cell populations other than leukocytes, including preadipocytes and adipocytes (Goralski et al., 2007; Roh et al., 2007), skeletal muscle cells (Sell et al., 2009), and endothelial cells (Kaur et al., 2010), and additional roles for the chemerin/CMKLR1 system were proposed in the control of lipid and glucose metabolism (Bozaoglu et al., 2007; Ernst and Sinal, 2010), blood pressure (Watts et al., 2013), and angiogenesis (Kaur et al., 2010).

Recent studies identified ligands for these receptors and their functions have begun to be unveiled. Accordingly, the plasma protein-derived chemoattractant chemerin is a ligand for CMKLR1 and activation of CMKLR1 with chemerin has been shown to induce the migration of macrophages and dendritic cells (DCs) *in vitro,* suggesting a pro-inflammatory role of Chemerin. *In vivo* studies using CMKLR-deficient mice suggest on the contrary that these receptors may have an anti-inflammatory role, possibly due to the recruitment of plasmacytoid DCs. The chemerin/CMKLR1 interaction also promotes adipogenesis and angiogenesis.

Chemerin acts as a chemokine during inflammation, to recruit cells at the site of infection. This ligand promotes resolution of inflammation in animal models of acute inflammation by enhancement of PMN apoptosis and M2-dependent efferocytosis (phagocytosis non-phlogistic; without releasing proinflammatory mediators), and by decreasing the DC's migration and the secretion of IL12 (proinflammatory cytokine) (Serhan, 2014b).

Chemerin is present in high amounts in inflammatory fluids, has antimicrobial activities, was shown to attract CMKLR1 expressing leukocytes, and promotes adhesion of macrophages to extracellular matrix proteins (Wittamer et al., 2003). In addition, chemerin was recently discovered to be an adipokine. Secreted by mature adipocytes, it stimulates preadipocytes to differentiation. Increased serum levels of chemerin have been associated with chronic inflammatory diseases, coronary artery disease, the metabolic syndrome, and obesity. There is indication that high chemerin production in obese adipose tissue might contribute to the increased infiltration of macrophages observed in obese adipose tissue leading to low-level inflammation. When Chemerin binds CMKLR1, two signaling pathways are activated: the G-protein signaling pathway and the β-arrestin signaling pathway.

The second ligand of CMKLR1 is the lipid mediator Resolvin E1 (RvE1) that belongs to the Resolvin family. The anti-inflammatory lipid mediator Resolvin E1 inhibits leukocyte infiltration and pro-inflammatory gene expression. These divergent results suggest that CMKLR1 is a multifunctional receptor. It is involved in increasing PMN apoptosis and M2 efferocytosis and is decreasing DC migration and secretion of pro-inflammatory cytokines like IL12. RvE1 is produced by neutrophils and endothelial cells. It is also induced *in vitro* with aspirin treatment because aspirin activate the COX-2 pathway responsible for the production of RvE1. Furthermore, neutrophil enable the conversion of 18R-HEPE to RvE1. In localized aggressive periodontitis (LAP) patients, it has been observed that macrophages reduce phagocytosis. RvE1 rescues impaired phagocytic activity of LAP macrophages. RvE1 additionally promotes resolution via reducing IL-23 and IL-6 in allergic airways of mice as well as increasing IFN-γ. RvE1 regulates natural killer (NK) cell migration and cytotoxicity. When RvE1 binds CMKLR1, only the G-protein signaling pathway is activated and the B-arrestin pathway is inhibited; and in particular conditions, the B-arresting pathway is inhibited.

Initially, the interest in the chemerin system was focused on its role in inflammation and chemotaxis of immune cells following its discovery in psoriasis disease. Most recently, in connection with its role in inflammation, in obesity, metabolic syndrome, it's potential role in association with cardiovascular functions has been considered, as well as role in reproductive biology. Therefore, the chemerin system is of major interest for its role in the inflammation process, in particular for its role in the resolution of the inflammation. A number of diseases are related to delay or disruption of the resolution process. Most of the specialized pro-resolving factor mediators currently known are derived from polyunsaturated fatty acids, including lipoxins, the resolving family, including E-series resolvins and D-series resolvins, protectins, and maresins. Nonetheless, pro-resolving molecules are difficult to synthesize because of their lipidic nature. Production of pro-resolving molecules in sufficient quantities, for a clinical trial for example, is a burden, and very few SPM have gone through efficient production. Besides that, antibodies specifically targeting G-protein-coupled receptors are difficult to produce. There is therefore a need for molecules having the capability to initiate or enhance the resolution stage of the inflammatory response like pro-resolving factors.

### Summary of the invention

In a first aspect, the invention relates to an anti-CMKLR1 antibody or antigen-binding fragment thereof or antigen-binding antibody mimetic or modified antibody. In the following disclosure, an anti-CMKLR1 compound is considered as being either an anti-CMKLR1 antibody or antigen-binding fragment thereof or antigen-binding antibody mimetic or modified antibody. In a particular embodiment of the invention, said compound is defined by the sequences of its CDRS. In a more particular embodiment of the invention, the anti-CMKLR1 compound is an antibody defined by the sequences of its CDRs and its framework regions (FR). An anti-CMKLR1 compound is a compound which binds specifically to the Chemokin-like receptor 1 (CMKLR1). In the following disclosure, the terms Chemokin-like receptor 1, CMKLR1 and ChemR23 are used interchangeably, and all designate the receptor encoded by gene *CMKLR1* in human or *cmklr1* in non-human animals. In a particular embodiment of the invention, the anti-CMKLR1 compound binds specifically to human CMKLR1 or in other words the invention relates to an anti-human CMKLR1 compound. As used herein, the term "CMKLR1" refers to a Chemokin-Like Receptor 1 protein (also designated as chemR23), a member of the G-protein coupled receptor family from a mammal species, preferably a human CMKLR1. A reference sequence of the human CMKLR1 protein, used in the examples of the present application, corresponds to the sequence associated to the Uniprot Accession number Q99788 (SEQ ID No: 1).

In a second aspect, the invention relates to an anti-CMKLR1 antibody or antigen-binding fragment thereof or antigen-binding antibody mimetic or modified antibody defined by at least one functional feature. In a preferred embodiment, said anti-CMKLR1 compound is defined by its capability to inhibit secretion of proinflammatory cytokines, in particular IL12, and/or its capability to enhance secretion of anti-inflammatory cytokines, in particular IL10 and/or CCL17. In a more particular embodiment, the anti-CMKLR1 compound inhibits or enhances cytokine secretion by macrophages, in particular M1 and/or M2 macrophages. In a particular embodiment, the anti-CMKLR1 compound of the invention enhances the polarization of macrophages into anti-inflammatory macrophages, in particular M2 macrophages.

In a third aspect, the invention relates to an anti-CMKLR1 antibody or antigen-binding fragment thereof or antigen-binding antibody mimetic that has agonist properties towards Resolvin E1 (RvE1) thereby mimicking the binding of RvE1 to CMKLR1 on CMKLR1 positive cells. *"Agonist properties towards RvE1-CMKLR1 interaction"* means that the antibody or antigen-binding fragment thereof or antigen-binding antibody mimetic or modified antibody of the invention, which targets the CMKLR1, has the effect of mimicking the binding of RvE1 to CMKLR1, thereby activating the receptor signaling pathway normally activated by RvE1, especially the binding of human RvE1 to human CMKLR1, in particular on dendritic cells, monocytes and macrophages. As a result of binding and activation of the receptor to produce a biological response, the compounds of the invention may lead to the activation of the G protein signaling pathway, in particular Gαᵢ signaling pathway and/or Gαₒ, without activating the β-arrestin pathway, in particular the compound of the invention may lead to the inhibition of the β-arrestin pathway. In particular, the binding of a compound according to the invention induces the activation of Akt and/or Erk protein(s) *in vitro* and/or *in vivo.* In other words, a Resolvin-E1 like agonist antibody may be defined as an antibody able to bind CMKLR1, and thereby able to induce the phosphorylation of the Akt and/or Erk protein(s), as compared to a control antibody. A control antibody may be an antibody which does not specifically bind CMKLR1. Phosphorylation of a protein may be determined according to methods well known by the skilled artisan, for example by the method disclosed in the examples of the present description.

In a particular embodiment, a compound of the invention is a RvE1-like agonist, i.e. a compound of the invention is an agonist of CMKLR1 signaling pathway induced by RvE1. In other words, the anti-CMKLR1 compound of the invention is an agonist of the interaction between RvE1 and CMKLR1, in particular between human RvE1 and human CMKLR1. In a particular embodiment, the compound of the invention enhances activation of the G protein pathway induced by CMKLR1. In another embodiment, the compound of the invention does not induce the activation of the β-arrestin pathway induced by CMKLR1. In another embodiment, the compound of the invention inhibits the β-arrestin pathway induced by CMKLR1. In another embodiment, since a compound of the invention induces at least one agonist effect of the binding of RvE1 to CMKLR1, and since RvE1 is a pro-resolution factor or pro-resolution mediator, a compound of the invention is a pro-resolution factor or a pro-resolution mediator.

In a particular embodiment, a compound of the invention does not interfere with the binding of Chemerin to CMKLR1. Chemerin is one of the natural ligand of CMKLR1. In other words, a compound according to the invention is not an agonist of the interaction between Chemerin and CMKLR1. The absence of such an agonist capability may be assessed according to the examples of the invention, wherein a competition assay to measure Chemerin-dependent B-arrestin recruitment by CMKLR1 receptor in presence of anti-CMKLR1 antibody of the invention is disclosed. In a preferred embodiment, the anti-CMKLR1 compound of the invention does not compete with Chemerin for the binding to CMKLR1. The absence of competition between an anti-CMKLR1 compound of the invention and chemerin may be determined when, in presence of the CMKLR1 compound of the invention, the binding of Chemerin to CMKLR1 is at least 50%, more preferably at least 80%, still more preferably at least 90% and most preferably similar, to the binding of Chemerin to CMKLR1, under the same experimental conditions but without the presence of the anti-CMKLR1 of the invention. Alternatively, the absence of competition between an anti-CMKLR1 compound of the invention and Chemerin may be determined according to the method illustrated in example 11.

In a particular embodiment, the anti-CMKLR1 compound has the capability *in vitro* and/or *in vivo* to activate at least one of the Akt signaling pathway proteins (also known as PI3K-Akt Pathway) and/or Erk signaling pathway proteins, preferably Akt protein and/or Erk protein, preferably both the Akt and the Erk proteins. Activation of a pathway may be assessed according to methods known in the art, and in particular with methods disclosed in the examples of the present invention.

In a particular embodiment, the antibody, antigen-binding fragment thereof, antigen-binding antibody mimetic or modified antibody of the invention enhances RvE1-induced secretion of IL10 cytokine *in vitro* and/or *in vivo,* in particular secretion of IL10 cytokine by macrophages. In a particular embodiment, the antibody, antigen-binding fragment thereof, antigen-binding antibody mimetic or modified antibody of the invention enhances RvE1-induced secretion of IL10 and CCL17 cytokines *in vitro* and/or *in vivo,* in particular secretion of IL10 and CCL17 by macrophages. In a particular embodiment, the antibody, antigen-binding fragment thereof, antigen-binding antibody mimetic or modified antibody of the invention inhibits the secretion of IL12 cytokine *in vitro* and/or *in vivo,* in particular secretion of IL12 by macrophages. The inhibition may be partial, i.e. the level of secretion of IL12 in the presence of the anti-CMKLR1 compound is decreased over a baseline level (i.e. the level without RvE1 or the anti-CMKLR1 compound), or the inhibition may be complete (no secretion of IL12).

### Detailed description of the invention

As used herein, the term "antibody" comprises polyclonal antibodies, monoclonal antibodies or recombinant antibodies. As used herein, a "monoclonal antibody" is intended to refer to a preparation of antibody molecules to obtain antibodies which share a common heavy chain and common light chain amino acid sequence, in contrast with "polyclonal" antibody preparations which contain a mixture of antibodies of different amino acid sequence. Monoclonal antibodies can be generated by several known technologies like phage, bacteria, yeast or ribosomal display, as well as by classical methods exemplified by hybridoma-derived antibodies. They may also be synthetized using the disclosed amino acid sequences as reference. Thus, the term "monoclonal" is used to refer to all antibodies derived from one nucleic acid clone.

The antibodies of the present invention include recombinant antibodies. As used herein, the term "recombinant antibody" refers to antibodies which are produced, expressed, generated or isolated by recombinant means, such as antibodies which are expressed using a recombinant expression vector transfected into a host cell; antibodies isolated from a recombinant combinatorial antibody library; antibodies isolated from an animal (*e.g.* a mouse) which is transgenic due to human immunoglobulin genes; or antibodies which are produced, expressed, generated or isolated in any other way in which particular immunoglobulin gene sequences (such as human immunoglobulin gene sequences) are assembled with other DNA sequences. Recombinant antibodies include, for example, chimeric and humanized antibodies.

As used herein, a "chimeric antibody" refers to an antibody in which the sequence of the variable domain derived from the germline of a mammalian species, such as a mouse, have been grafted onto the sequence of the constant domain derived from the germline of another mammalian species, such as a human.

As used herein, a "humanized antibody" refers in a first embodiment to an antibody in which CDR sequences derived from the germline of another mammalian species, such as a mouse, have been grafted onto human or humanized framework sequences. In a further embodiment, a "humanized antibody" refers to an antibody wherein at least one CDR and all or part of framework sequences have been humanized.

As used herein, an "antigen-binding fragment of an antibody" means a part of an antibody, i.e. a molecule corresponding to a portion of the structure of the antibody of the invention, that exhibits antigen-binding capability for CMKLR1, possibly in its native form; such fragment especially exhibits the same or substantially the same antigen-binding specificity for said antigen compared to the antigen-binding specificity of the corresponding four-chain antibody. Advantageously, the antigen-binding fragments have a similar binding affinity as the corresponding 4-chain antibodies. However, antigen-binding fragment that have a reduced antigen-binding affinity with respect to corresponding 4-chain antibodies are also encompassed within the invention. The antigen-binding capability can be determined by measuring the affinity between the antibody and the target fragment. These antigen-binding fragments may also be designated as "functional fragments" of antibodies.

Antigen-binding fragments of antibodies are fragments which comprise their hypervariable domains designated CDRs (Complementary Determining Regions) or part(s) thereof encompassing the recognition site for the antigen, i.e. the extracellular domain of CMKLR1, in particular the third loop of the extracellular domain of CMKLR1 (designated EL3), thereby defining antigen recognition specificity. EL3 is localized between amino acid residue 283 and amino acid residue 300 of SEQ ID No: 1. EL3 corresponds to the amino acid residues of SEQ ID No: 2. EL3 is also comprised within the polypeptide of SEQ ID No: 18.

Each Light and Heavy chain variable domains (respectively VL and VH) of a four-chain immunoglobulin has three CDRs designated VLCDR1, VLCDR2 and VLCDR3 for the light chain variable domain; and VHCDR1, VHCDR2, VHCDR3 for the heavy chain variable domain. Each Light chain and Heavy chain variable domains of a four-chain immunoglobulin has four framework regions (FR), designated LFR1, LFR2, LFR3 and LFR4 for the light chain variable domain; and HFR1, HFR2, HFR3 and HFR4 for the heavy chain variable domain.

The skilled person is able to determine the location of the various regions/domains of antibodies by reference to the standard definitions in this respect set forth, including a reference numbering system, a reference to the numbering system of KABAT or by application of the IMGT "collier de perle" algorithm. In this respect, for the definition of the sequences of the invention, it is noted that the delimitation of the regions/domains may vary from one reference system to another. Accordingly, the regions/domains as defined in the present invention encompass sequences showing variations in length or localization of the concerned sequences within the full-length sequence of the variable domains of the antibodies, of approximately +/- 10%.

Based on the structure of four-chain immunoglobulins, antigen-binding fragments can thus be defined by comparison with sequences of antibodies in the available databases and prior art, and especially by comparison of the location of the functional domains in these sequences, noting that the positions of the framework and constant domains are well defined for various classes of antibodies, especially for IgGs, in particular for mammalian IgGs. Such comparison also involves data relating to 3-dimensional structures of antibodies.

For illustration purpose of specific embodiments of the invention, antigen binding fragments of an antibody that contain the variable domains comprising the CDRs of said antibody encompass Fv, dsFv, scFv, Fab, Fab', F(ab')2. Fv fragments consist of the VL and VH domains of an antibody associated together by hydrophobic interactions; in dsFv fragments, the VH:VL heterodimer is stabilised by a disulphide bond; in scFv fragments, the VL and VH domains are connected to one another via a flexible peptide linker thus forming a single-chain protein. Fab fragments are monomeric fragments obtainable by papain digestion of an antibody; they comprise the entire L chain, and a VH-CH1 fragment of the H chain, bound together through a disulfide bond. The F(ab')2 fragment can be produced by pepsin digestion of an antibody below the hinge disulfide; it comprises two Fab' fragments, and additionally a portion of the hinge region of the immunoglobulin molecule. The Fab' fragments are obtainable from F(ab')2 fragments by cutting a disulfide bond in the hinge region. F(ab')2 fragments are divalent, i.e. they comprise two antigen binding sites, like the native immunoglobulin molecule; on the other hand, Fv (a VHVL dimmer constituting the variable part of Fab), dsFv, scFv, Fab, and Fab' fragments are monovalent, i.e. they comprise a single antigen-binding site. These basic antigen-binding fragments of the invention can be combined together to obtain multivalent antigen-binding fragments, such as diabodies, tribodies or tetrabodies. These multivalent antigen-binding fragments are also part of the present invention.

As used herein, the term modified antibody includes "bispecific" antibodies and refers to antibodies that recognize two different antigens by virtue of possessing at least one region (e.g. derived from a variable region of a first antibody) that is specific for a first antigen, and at least a second region (e.g. derived from a variable region of a second antibody) that is specific for a second antigen. A bispecific antibody specifically binds to two target antigens and is thus one type of multispecific antibody. Multispecific antibodies, which recognize two or more different antigens, can be produced by recombinant DNA methods or include, but are not limited to, antibodies produced chemically by any convenient method. Bispecific antibodies include all antibodies or conjugates of antibodies, or polymeric forms of antibodies which are capable of recognizing two different antigens. Bispecific antibodies include antibodies that have been reduced and reformed so as to retain their bivalent characteristics and to antibodies that have been chemically coupled so that they can have several antigen recognition sites for each antigen such as BiME (Bispecific Macrophage Enhancing antibodies), BiTE (bispecific T cell engager), DART (Dual affinity retargeting); DNL (dock-and-lock), DVD-Ig (dual variable domain immunoglobulins), HAS (human serum albumin), kih (knobs into holes).

Antigen-binding antibody mimetics are organic compounds that specifically bind antigens, but that are not structurally related to antibodies. They are usually artificial peptides or small proteins with a molar mass of about 3 to 20 kDa. Nucleic acids and small molecules are sometimes considered antibody mimetics as well, but not artificial antibodies, antibody fragments and fusion proteins composed from these. Common advantages over antibodies are better solubility, tissue penetration, stability towards heat and enzymes, and comparatively low production costs. Antibody mimetics are being developed as therapeutic and diagnostic agents. Antigen-binding antibody mimetics may also be selected among the group comprising affibodies, affilins, affimers, affitins, DARPins, and Monobodies.

An antigen-binding antibody mimetic is more preferentially selected from the groups comprising affitins and anticalins. Affitins are artificial proteins with the ability to selectively bind antigens. They are structurally derived from the DNA binding protein Sac7d, found in *Sulfolobus acidocaldarius,* a microorganism belonging to the archaeal domain. By randomizing the amino acids on the binding surface of Sac7d, e.g. by generating variants corresponding to random substitutions of 11 residues of the binding interface of Sac7d, an affitin library may be generated and subjecting the resulting protein library to rounds of ribosome display, the affinity can be directed towards various targets, such as peptides, proteins, viruses and bacteria. Affitins are antibody mimetics and are being developed as tools in biotechnology. They have also been used as specific inhibitors for various enzymes (Krehenbrink et al., J. mol. Biol., 383:5, 2008). The skilled person may readily develop affitins with the required binding properties using methods know in the art, in particular as disclosed in patent application WO2008068637 and the above-cited publication, in particular the generation of phage display and/or ribosome display libraries and their screening using an antigen as disclosed herein. Anticalins are artificial proteins that are able to bind to antigens, either to proteins or to small molecules. They are antibody mimetics derived from human lipocalins which are a family of naturally binding proteins. Anticalins are about eight times smaller with a size of about 180 amino acids and a mass of about 20 kDa (Skerra, Febs J., 275:11, 2008). Anticalin phage display libraries have been generated which allow for the screening and selection, in particular of anticalins with specific binding properties. The skilled person may readily develop anticalins with the required binding properties using methods know in the art, in particular as disclosed in EP patent EP1270725 B1, US patent US8536307 B2, Schlehuber and Skerra, Biophys. Chem., 96:2-3, 2002 and the above-cited publication, in particular the generation of phage display and/or ribosome display libraries and their screening using an antigen as disclosed herein. Anticalins and affitins may both be produced in a number of expression system comprising bacterial expression systems. Thus, the invention includes the use of affitins, anticalins and other similar antibody mimetics with the features of the antibodies described herein, in particular with regard to their binding capability to CMKLR1, to their agonist capability towards the binding between RvE1 and CMKLR1, their capability to induce or inhibit secretion of particular cytokines as described herein, to their use in the treatment or the prevention of a disease as described herein, all of which are contemplated as mimetics according to the invention.

As used herein, a "modified antibody" refers to antibodies the amino sequence of which has been modified by mutation of at least one amino acid residue. Accordingly, "modified antibody" encompasses chimeric antibodies or humanized antibodies as defined herein, "modified antibody" may also correspond to a molecule comprising an antibody or an antigen-binding fragment thereof, wherein said monoclonal antibody or functional fragment thereof is associated with a functionally different molecule. A modified antibody of the invention may be either a fusion chimeric protein or a conjugate resulting from any suitable form of attachment including covalent attachment, grafting, chemical bonding with a chemical or biological group or with a molecule, such as a PEG polymer or another protective group or molecule suitable for protection against proteases cleavage in vivo, for improvement of stability and/or half-life of the antibody or functional fragment. With similar techniques, especially by chemical coupling or grafting, a modified antibody can be prepared with a biologically active molecule, said active molecule being for example chosen among toxins, in particular Pseudomonas exotoxin A, the A-chain of plant toxin ricin or saporin toxin, especially a therapeutic active ingredient, a vector (including especially a protein vector) suitable for targeting the antibody or functional fragment to specific cells or tissues of the human body, or it may be associated with a label or with a linker, especially when fragments of the antibody are used. PEGylation of the antibody or functional fragments thereof is a particular interesting embodiment as it improves the delivery conditions of the active substance to the host, especially for a therapeutic application. PEGylation can be site specific to prevent interference with the recognition sites of the antibodies or functional fragments, and can be performed with high molecular weight PEG. PEGylation can be achieved through free cysteine residues present in the sequence of the antibody or functional fragment or through added free Cysteine residues in the amino sequence of the antibody or functional fragment. According to the present invention, when the term "antibody" is used, it means either an antibody, an antigen-binding fragment thereof, an antigen-binding antibody mimetic or a modified antibody.

"Humanized" forms of non-human (e.g., murine) antibodies are chimeric immunoglobulins, immunoglobulin chains or fragments thereof (such as Fv, Fab, Fab', F(ab')2 or other target-binding subsequences of antibodies) which contain minimal sequence derived from non-human immunoglobulin. In general, the humanized antibody will comprise substantially all of at least one, and typically two, variable domains, in which all the CDR regions correspond to those of a non-human immunoglobulin and/or humanized version of those; and all or substantially all of the FR regions are those of a human immunoglobulin template sequence. The humanized antibody may also comprise at least a portion of an immunoglobulin constant region (Fc), typically that of a human immunoglobulin template chosen. In a particular embodiment, the invention relates to an antibody comprising a heavy chain variable region as disclosed herein and a light chain variable region as disclosed herein, the heavy chain variable region and/or the light chain variable region further comprising a constant region, in particular a Fc region.

The terms "Specifically binding" and "specifically bind to" refer to the ability of an antibody, antigen-binding fragment thereof, antigen-binding antibody mimetic or a modified antibody according to the invention to bind to CMKLR1 with an affinity of at least 1 X 10⁻⁶ M, 1 X 10⁻⁷ M, 1 X 10⁻⁸ M, 1 X 10⁻⁹ M, 1 X 10⁻¹⁰ M, 1 X 10⁻¹¹ M, 1 X 10⁻¹² M, or more, and/or to bind to CMKLR1 with an affinity which is at least two-fold greater to its affinity for a non-specific target (*e.g.* another protein than CMKLR1). The affinity may be assessed according to various methods well known from those skilled in the art. These methods include but are not limited to biosensors such as Biacore analysis, Blitz analysis and Scatchard plot.

The term "therapeutically effective amount" is used to refer to an amount of any given compound as defined herein sufficient for at least the improvement of the clinical or physiological condition of a treated patient. The therapeutically effective amount of the antibody, antigen-binding fragment thereof, antigen-binding antibody mimetic or modified antibody according to the invention to be administered is governed by considerations such as the disorder being treated, the particular mammal being treated, the clinical condition of the individual patient, the cause of the disorder, the site of delivery of the agent, the method of administration, the scheduling of administration, and other factors known to medical practitioners.

All the embodiments disclosed herein for antibodies or antigen-binding fragments thereof are transposed *mutatis mutandis* to the macromolecules of the invention, in particular to antigen-binding antibody mimetics and to modified antibodies.

In a first aspect, the invention relates to an antibody, an antigen-binding fragment thereof, an antigen-binding antibody mimetic or a modified antibody which specifically binds to CMKLR1, in particular to the EL3 loop of CMKLR1, in particular to a polypeptide comprising the amino acid residues of SEQ ID No: 2, and more particularly to an epitope localized within SEQ ID No: 2 or within the EL3 loop of CMKLR1 and consisting of the sequence of amino acid residues " AMPGS " (SEQ ID No: 152) and comprising:
- A heavy chain variable domain comprising HCDR1, HCDR2 and HCDR3;
- A light chain variable domain comprising LCDR1, LCDR2 and LCDR3;

In another aspect, the invention relates to an anti-CMKLR1 compound selected from the group of an antibody or an antigen-binding fragment thereof or an antigen-binding antibody mimetic or a chimeric or humanized antibody, which specifically binds to CMKLR1, said compound comprising:
- an antibody heavy chain variable domain comprising the three CDRs VHCDR1, VHCDR2 and VHCDR3, wherein:
   - VHCDR1 comprises or consists of the amino acid sequences set forth in SEQ ID No: 4 or a mutated sequence thereof wherein amino acid residue(s) is(are) substituted; and
   - VHCDR2 comprises or consists of the amino acid sequences set forth in SEQ ID No: 6 or a mutated sequence thereof wherein amino acid residue(s) is(are) substituted; and
   - VHCDR3 comprises or consists of the amino acid sequences set forth in SEQ ID No: 8 or a mutated sequence thereof wherein amino acid residue(s) is(are) substituted providing amino acid residues at position 1 and 2 of SEQ ID No: 8 are respectively L and I or L; and
- an antibody light chain variable domain comprising the three CDRs VLCDR1, VLCDR2 and VLCDR3, wherein:
   - VLCDR1 comprises or consists of the amino acid sequences set forth in SEQ ID No: 12 or a mutated sequence thereof wherein amino acid residue(s) is(are) substituted; and
   - VLCDR2 comprises or consists of the amino acid sequences set forth in SEQ ID No: 14 or a mutated sequence thereof wherein amino acid residue(s) is(are) substituted; and
   - VLCDR3 comprises or consists of the amino acid sequences set forth in SEQ ID No: 15 or a mutated sequence thereof wherein the amino acid residue(s) is(are) substituted;

   wherein the anti-CMKLR1 compound is a RvE1-like agonist of CMKLR1, in particular wherein the anti-CMKLR1 compound is a pro-resolution factor, in particular on myeloid cell lineages;
   in particular wherein the anti-CMKLR1 compound is an anti-human CMKLR1 compound.

In another aspect, the invention relates to an anti-CMKLR1 compound selected from the group of an antibody or an antigen-binding fragment thereof or an antigen-binding antibody mimetic or a chimeric or humanized antibody, which specifically binds to CMKLR1, said compound comprising an antibody heavy chain variable domain comprising a VHCDR3 comprising or consisting of the amino acid sequences set forth in SEQ ID No: 8 or a mutated sequence thereof wherein amino acid residue(s) is(are) substituted providing amino acid residues at position 1 and 2 of SEQ ID No: 8 are respectively L and I or L; and
wherein the anti-CMKLR1 compound specifically binds to an epitope localized within the third extra-cellular loop (EL3) of CMKLR1, in particular wherein the compound binds specifically to a polypeptide comprising or consisting of amino acid residues of sequence SEQ ID No: 2 or SEQ ID No: 152; and
wherein the anti-CMKLR1 compound is a Resolvin E1-like agonist of CMKLR1, in particular wherein the anti-CMKLR1 compound is a pro-resolution factor, in particular on myeloid cell lineages; and
wherein said compound competes with an antibody comprising the heavy variable domain corresponding to SEQ ID No: 9 and the light chain variable domain corresponding to SEQ ID No: 16, more particularly with antibody 2G1, for the binding to a polypeptide comprising or consisting of amino acid residues of sequence SEQ ID No: 2 or SEQ ID No: 152 or to a polypeptide comprising or consisting of the third loop (EL3) of the extracellular domain of CMKLR1.

In another aspect, the invention relates to an anti-CMKLR1 compound selected from the group of an antibody or an antigen-binding fragment thereof or an antigen-binding antibody mimetic or a chimeric or humanized antibody, which specifically binds to CMKLR1, said compound comprising:
- an antibody heavy chain variable domain comprising the three CDRs VHCDR1, VHCDR2 and VHCDR3, wherein:
   - VHCDR1 comprises or consists of the amino acid sequences set forth in SEQ ID No: 4 or a mutated sequence thereof wherein amino acid residue(s) is(are) substituted; and
   - VHCDR2 comprises or consists of the amino acid sequences set forth in SEQ ID No: 6 or a mutated sequence thereof wherein amino acid residue(s) is(are) substituted; and
   - VHCDR3 comprises or consists of the amino acid sequences set forth in SEQ ID No: 8 or a mutated sequence thereof wherein amino acid residue(s) is(are) substituted providing amino acid residues at position 1 and 2 of SEQ ID No: 8 are respectively L and I or L; and
- an antibody light chain variable domain comprising the three CDRs VLCDR1, VLCDR2 and VLCDR3, wherein:
   - VLCDR1 comprises or consists of the amino acid sequences set forth in SEQ ID No: 12 or a mutated sequence thereof wherein amino acid residue(s) is(are) substituted; and
   - VLCDR2 comprises or consists of the amino acid sequences set forth in SEQ ID No: 14 or a mutated sequence thereof wherein amino acid residue(s) is(are) substituted; and
   - VLCDR3 comprises or consists of the amino acid sequences set forth in SEQ ID No: 15 or a mutated sequence thereof wherein the amino acid residue(s) is(are) substituted;
   wherein the anti-CMKLR1 compound is a RvE1-like agonist of CMKLR1, in particular wherein the anti-CMKLR1 compound is a pro-resolution factor, in particular on myeloid cell lineages;
said compound competes with an antibody comprising the heavy variable domain corresponding to SEQ ID No: 9 and the light chain variable domain corresponding to SEQ ID No: 16, more particularly with antibody 2G1, for the binding to a polypeptide (or antigen) comprising or consisting of amino acid residues of sequence SEQ ID No: 2 and/or SEQ ID No: 152 and/or for the binding to the third extra-loop of CMKLR1 (*i.e.,* cross-compete for the binding to the amino acid residues of SEQ ID No: 2 and/or SEQ Id No: 152, or for the binding to the third extra-loop of CMKLR1). The binding to the polypeptide comprising or consisting of amino acid residues of sequence SEQ ID No: 2 and/or SEQ Id No: 152 and/or for the binding to the third extra-loop of CMKLR1 may be assessed by the examples disclosed in the examples of the invention, in particular in example 9 by binding affinity analysis by ELISA assays. To determine if a test antibody can compete for binding to the same antigen or to the third loop as the epitope bound by the 2G1 antibody (or an antigen-binding fragment comprising the heavy variable domain corresponding to SEQ ID No: 9 and the light chain domain corresponding to SEQ ID No: 16), a cross-blocking assay (*e.g.* a competitive ELISA assay) can be performed. In an exemplary competitive ELISA assay, a polypeptide comprising or consisting of the epitope or the third loop may be coated on the wells of a microtiter plate and pre-incubated with or without candidate competing antibody and then a biotin-labeled 2G1 antibody of the invention is added. The amount of labeled anti-2G1 antibody bound to the polypeptide comprising or consisting of the polypeptide of SEQ ID No: 2 or SEQ ID No: 152 or the third loop of CMKLR1 in the wells is measured using avidin-peroxidase conjugate and appropriate substrate. The antibody can be labeled with a radioactive or fluorescent label or some other detectable and measurable label. The amount of labeled anti-2G1 antibody that bound to the polypeptide of SEQ ID No: 2 or SEQ ID No: 152 or to the third loop will have an indirect correlation to the ability of the candidate competing antibody (test antibody) to compete for binding to the same epitope or to the same loop, *i.e.,* the greater the affinity of the test antibody for the same epitope, the less labeled 2G1 antibody will be bound to the antigen-coated wells. A candidate competing antibody is considered an antibody that competes for binding to the same polypeptide or third loop as 2G1 antibody of the invention if the candidate antibody can block binding of the 2G1 antibody by at least 20%, preferably by at least 20-50%, even more preferably, by at least 50% as compared to a control performed in parallel in the absence of the candidate competing antibody (but may be in the presence of a known non-competing antibody). It will be understood that variations of this assay can be performed to arrive at the same quantitative value.

In another aspect, the invention relates to an antibody, antigen-binding fragment thereof, antigen-binding antibody mimetic or chimeric or humanized antibody which specifically binds to CMKLR1, in particular to human CMKLR1, comprising:
- an antibody heavy chain variable domain comprising the three CDRs VHCDR1, VHCDR2 and VHCDR3, wherein:
   - VHCDR1 comprises or consists of the amino acid sequences set forth in SEQ ID No: 3; SEQ ID No: 4; SEQ ID No: 62; SEQ ID No: 63; SEQ ID No: 64 or SEQ ID No: 65; and
   - VHCDR2 comprises or consists of the amino acid sequences set forth in SEQ ID No:5; SEQ ID No: 6; SEQ ID No: 66; SEQ ID No: 67; SEQ ID No: 68; SEQ ID No: 69; SEQ ID No: 70; SEQ ID No: 71 or SEQ ID No: 72; and
   - VHCDR3 comprises or consists of the amino acid sequences set forth in SEQ ID No: 7; SEQ ID No: 8; SEQ ID No: 73; SEQ ID No: 74, SEQ ID No: 75; SEQ ID No: 144; SEQ ID No: 145, SEQ ID No: 146; SEQ ID No: 147; SEQ ID No: 148, SEQ ID No: 149; SEQ ID No: 150 or SEQ ID No: 151 and/or
   - An antibody light chain variable domain comprising the three CDRs VLCDR1, VLCDR2 and VLCDR3, wherein:
      - VLCDR1 comprises or consists of the amino acid sequences set forth in SEQ ID No: 11; SEQ ID No: 12; SEQ ID No: 76; SEQ ID No: 77; SEQ ID No: 78; SEQ ID No: 79 or SEQ ID No: 80; and
      - VLCDR2 comprises or consists of the amino acid sequences set forth in SEQ ID No: 13; SEQ ID No: 14; SEQ ID No: 81; SEQ ID No: 82; SEQ ID No: 83; SEQ ID No: 84; SEQ ID No: 85; SEQ ID No: 86; SEQ ID No: 87 or SEQ ID No: 88; and
      - VLCDR3 comprises or consists of the amino acid sequences set forth in SEQ ID No: 15 or SEQ ID No: 89.

In a particular embodiment, the invention relates to an antibody, antigen-binding fragment thereof, antigen-binding antibody mimetic or modified antibody which specifically binds to CMKLR1, in particular to human CMKLR1, comprising:
- an antibody heavy chain variable domain comprising the three CDRs VHCDR1, VHCDR2 and VHCDR3, wherein:
   - VHCDR1 comprises or consists of the amino acid sequences set forth in SEQ ID No: 4; SEQ ID No: 62; or SEQ ID No: 63; and
   - VHCDR2 comprises or consists of the amino acid sequences set forth in SEQ ID No: 67; SEQ ID No: 70 or SEQ ID No: 72; and
   - VHCDR3 comprises or consists of the amino acid sequence set forth in SEQ ID No: 8; SEQ ID No.: 144 or SEQ ID No. 148; and/or
- an antibody light chain variable domain comprising the three CDRs VLCDR1, VLCDR2 and VLCDR3, wherein:
   - VLCDR1 comprises or consists of the amino acid sequence set forth in SEQ ID No: 77; and
   - VLCDR2 comprises or consists of the amino acid sequences set forth in SEQ ID No: 14; SEQ ID No: 81 or SEQ ID No: 84; and
   - VLCDR3 comprises or consists of the amino acid sequences set forth in SEQ ID No: 15 or SEQ ID No: 89.

In a particular embodiment of the invention, the antibody heavy chain variable domain comprises VHCDR1 of the amino acid sequence set forth in SEQ ID No: 4; VHCDR2 of the amino acid sequence set forth in SEQ ID No: 67 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 4; VHCDR2 of the amino acid sequence set forth in SEQ ID No: 70 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 4; VHCDR2 of the amino acid sequence set forth in SEQ ID No: 72 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 62; VHCDR2 of the amino acid sequence set forth in SEQ ID No: 67 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 62; VHCDR2 of the amino acid sequence set forth in SEQ ID No: 70 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 62; VHCDR2 of the amino acid sequence set forth in SEQ ID No: 72 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 63; VHCDR2 of the amino acid sequence set forth in SEQ ID No: 67 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 63; VHCDR2 of the amino acid sequence set forth in SEQ ID No: 70 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody heavy chain variable domain comprises VHCDR1 of the amino acid sequence set forth in SEQ ID No: 63; VHCDR2 of the amino acid sequence set forth in SEQ ID No: 72 and VHCDR3 of the amino acid sequence set forth in SEQ ID No: 144 or SEQ ID No: 148, more particularly of the amino acid sequence set forth in SEQ ID No: 144.

In a particular embodiment of the invention, the antibody light chain variable domain comprises VLCDR1 of the amino acid sequence set forth in SEQ ID No: 77; VLCDR2 of the amino acid sequence set forth in SEQ ID No: 14 and VLCDR3 of the amino acid sequence set forth in SEQ ID No: 15.

In a particular embodiment of the invention, the antibody light chain variable domain comprises VLCDR1 of the amino acid sequence set forth in SEQ ID No: 77; VLCDR2 of the amino acid sequence set forth in SEQ ID No: 14 and VLCDR3 of the amino acid sequence set forth in SEQ ID No: 89.

In a particular embodiment of the invention, the antibody light chain variable domain comprises VLCDR1 of the amino acid sequence set forth in SEQ ID No: 77; VLCDR2 of the amino acid sequence set forth in SEQ ID No: 81 and VLCDR3 of the amino acid sequence set forth in SEQ ID No: 15.

In a particular embodiment of the invention, the antibody light chain variable domain comprises VLCDR1 of the amino acid sequence set forth in SEQ ID No: 77; VLCDR2 of the amino acid sequence set forth in SEQ ID No: 81 and VLCDR3 of the amino acid sequence set forth in SEQ ID No: 89.

In a particular embodiment of the invention, the antibody light chain variable domain comprises VLCDR1 of the amino acid sequence set forth in SEQ ID No: 77; VLCDR2 of the amino acid sequence set forth in SEQ ID No: 84 and VLCDR3 of the amino acid sequence set forth in SEQ ID No: 15.

In a particular embodiment of the invention, the antibody light chain variable domain comprises VLCDR1 of the amino acid sequence set forth in SEQ ID No: 77; VLCDR2 of the amino acid sequence set forth in SEQ ID No: 84 and VLCDR3 of the amino acid sequence set forth in SEQ ID No: 89.

In a particular embodiment of the invention, the antibody, or antigen-binding fragment thereof, or antigen-binding antibody mimetic or modified antibody is a humanized antibody of a murine antibody, in particular wherein at least one framework region (FR1 and/or FR2 and/or FR3 and/or FR4) of the antibody light chain and/or heavy chain variable domain is derived from human chain framework regions, said light chain and/or heavy chain further comprising any combination of CDRs described herein, or wild type CDRs.

The wild type antibodies, referenced in the present disclosure as 2G1 and 2G4 (having the same variable region but with a different heavy chain constant region) have been defined and synthetized starting from multiple options in the amino acid sequence of the third CDR of the heavy chain. Several putative antibody sequences have accordingly been generated, synthetized and tested for their binding and for their biological properties. Within the plurality of newly synthetized antibodies, the presence of the sequence of amino acid residues "5' RLIY 3' " or "5' RLLY 3' " within the third CDR (according to IMGT) of the heavy chain variable domain has been determined as being highly implicated in the agonist property of the antibody towards the RvE1-CMKLR1 interaction. Antibodies having a different amino acid residue sequence (like "5' RIIY 3' "; "5' RILY 3' ") showed less appreciable capability to be agonists of the RvE1 binding to CMKLR1.

The wild type anti-CMKLR1 antibodies synthetized (referenced 2G1 and 4G1 in the present disclosure) are therefore a chimeric antibody with murine variable regions and human constant regions (human IgG1 for 2G1 or human IgG4 for 2G4). 2G1 has the following amino acid sequences in its variable sequences:
Heavy chain: 
metdtlllwvlllwvpgstgdeVQLVASGGGLVQPGGSLKLSCAASGFTFSSYGMSWVRQ
TPDRRLELVATINRYGGSTYYPDSVKGRFTISRDNAKNTLYLQMSSLKSEDTAM
YYCPRLIYYGNEGDSWGQGTTLTVSS
 wherein the signal peptide is represented in lower case, the CDRs are in bold characters (according to Kabat) or underlined (according to IMGT). Full sequence of the heavy chain corresponds to SEQ ID No: 10, with the signal peptide, while SEQ ID No: 9 corresponds to the heavy chain without the signal peptide. CDR1, CDR2 and CDR3 according to Kabat correspond to SEQ ID No: 4, SEQ ID No: 6 and SEQ ID No: 144 respectively; while CDR1, CDR2 and CDR3 according to IMGT correspond to SEQ ID No: 3, SEQ ID No: 5 and SEQ ID No: 7 respectively. CDR3 may be modified by substituting the amino acid residue ILE in second position by the amino acid residue LEU (second position according to Kabat numbering) leading to a heavy chain of sequence: 
metdtlllwvlllwvpgstgdeVQLVASGGGLVQPGGSLKLSCAASGFTFSSYGMSWVRQ
TPDRRLELVATINRYGGSTYYPDSVKGRFTISRDNAKNTLYLQMSSLKSEDTAM
YYCPRLLYYGNEGDSWGQGTTLTVSS
Light chain: 
metdtlllwvlllwvpgstgdQIVLTQSPAIMSASPGEKVTMTCSASSSVSFMHWYQQKS
GTSPKRWIYDTTKLTSGVPARFSGSGSGTFYSLTISSMEAEDAATYYCQQWNS
KPPLTFGPGTKLELKR
wherein the signal peptide is represented in lower case, the CDRs are in bold characters (according to Kabat) or underlined (according to IMGT). Full sequence of the light chain corresponds to SEQ ID No: 17, with the signal peptide, while SEQ ID No: 16 corresponds to the heavy chain without the signal peptide. CDR1, CDR2 and CDR3 according to Kabat correspond to SEQ ID No: 12, SEQ ID No: 14 and SEQ ID No: 15 respectively; while CDR1, CDR2 and CDR3 according to IMGT correspond to SEQ ID No: 11, SEQ ID No: 13 and SEQ ID No: 15 respectively.

As known in the art, and as exemplified with the present chimeric antibody, a heavy chain variable domain and a light chain variable domain both comprise 3 CDRs (CDR1, CDR2 and CDR3 from 5' end to 3' end respectively) and 4 framework regions (FR1, FR2, FR3 and FR4 from 5' end to 3' end respectively). Humanization of the murine antibody may consist of humanizing at least one framework region within the light chain variable region or within the heavy chain variable region or both. In a particular embodiment, several framework regions may be humanized, in particular within the heavy chain variable region and within the light chain variable region. The wild type CDRs may be conserved, but the CDRs may also be replaced by the CDRs already described herein. Hence, the anti-CKLMR1 compound according to the invention may comprise at least 1, or at least 2, or at least 3 or at least 4, or at least 5, or 6 wild type CDRs when the framework regions are humanized. In other words, the anti-CMKLR1 compound is a humanized version of the parental chimeric antibody 2G1, wherein at least 1 framework region is humanized, in particular wherein at least 1 framework region and at least 1 CDR are humanized. In a particular embodiment of the invention, the variable regions of the antibody may be associated with antibody constant regions, for example the constant regions set forth in SEQ ID No: 134 (encoded by the nucleotide sequence of SEQ ID No: 133); SEQ ID No: 136 (encoded by the nucleotide sequence of SEQ ID No: 135); SEQ ID No: 138 (encoded by the nucleotide sequence of SEQ ID No: 137); SEQ ID No: 139; SEQ ID No: 140 and SEQ ID No: 141. Such a combination is illustrated with the light chain of SEQ ID No: 142 and of the heavy chain of SEQ ID No: 143.

In particular embodiment of the invention, the antibody or antigen-binding fragment thereof, or antigen-binding antibody mimetic or modified antibody comprises at least one humanized framework region within its antibody heavy chain variable domain, wherein:
- the antibody heavy chain variable domain comprises four framework regions HFR1, HFR2, HFR3 and HFR4, wherein:
   - HFR1 comprises or consists of the amino acid sequences set forth in SEQ ID No: 90; SEQ ID No: 91; SEQ ID No: 92 or SEQ ID No: 93; and/or
   - HFR2 comprises or consists of the amino acid sequences set forth in SEQ ID No: 94; SEQ ID No: 95; SEQ ID No: 96; SEQ ID No: 97; SEQ ID No: 98; SEQ ID No: 99 or SEQ ID No: 100; and/or
   - HFR3 comprises or consists of the amino acid sequences set forth in SEQ ID No: 101; SEQ ID No: 102; SEQ ID No: 103; SEQ ID No: 104; SEQ ID No: 105 or SEQ ID No: 106; and/or
   - HFR4 comprises or consists of the amino acid sequences set forth in SEQ ID No: 107 or SEQ ID No: 108.

In a particular embodiment of the invention, the antibody or antigen-binding fragment thereof, or antigen-binding antibody mimetic or modified antibody comprises at least one humanized framework region within its antibody light chain variable domain, wherein:
- the antibody light chain variable domain comprises four framework regions LFR1, LFR2, LFR3 and LFR4, wherein:
   - LFR1 comprises or consists of the amino acid sequences set forth in SEQ ID No: 109; SEQ ID No: 110; SEQ ID No: 111 or SEQ ID No: 112; and/or
   - LFR2 comprises or consists of the amino acid sequences set forth in SEQ ID No: 113; SEQ ID No: 114; SEQ ID No: 115; SEQ ID No: 116 or SEQ ID No: 117; and/or
   - LFR3 comprises or consists of the amino acid sequences set forth in SEQ ID No: 118; SEQ ID No: 119; SEQ ID No: 120; SEQ ID No: 121; SEQ ID No: 122 or SEQ ID No: 132; and/or
   - LFR4 comprises or consists of the amino acid sequences set forth in SEQ ID No: 123 or SEQ ID No: 124.

In a particular embodiment of the invention, the antibody or antigen-binding fragment thereof, or antigen-binding antibody mimetic or modified antibody comprises at least one humanized framework region within its antibody light chain variable region and within its antibody heavy chain variable region, wherein:
- the antibody heavy chain variable domain comprises four framework regions HFR1, HFR2, HFR3 and HFR4, wherein:
   - HFR1 comprises or consists of the amino acid sequences set forth in SEQ ID No: 90; SEQ ID No: 91; SEQ ID No: 92 or SEQ ID No: 93; and/or
   - HFR2 comprises or consists of the amino acid sequences set forth in SEQ ID No: 94; SEQ ID No: 95; SEQ ID No: 96; SEQ ID No: 97; SEQ ID No: 98; SEQ ID No: 99 or SEQ ID No: 100; and/or
   - HFR3 comprises or consists of the amino acid sequences set forth in SEQ ID No: 101; SEQ ID No: 102; SEQ ID No: 103; SEQ ID No: 104; SEQ ID No: 105 or SEQ ID No: 106; and/or
   - HFR4 comprises or consists of the amino acid sequences set forth in SEQ ID No: 107 or SEQ ID No: 108; and
- the antibody light chain variable domain comprises four framework regions LFR1, LFR2, LFR3 and LFR4, wherein:
   - LFR1 comprises or consists of the amino acid sequences set forth in SEQ ID No: 109; SEQ ID No: 110; SEQ ID No: 111 or SEQ ID No: 112; and/or
   - LFR2 comprises or consists of the amino acid sequences set forth in SEQ ID No: 113; SEQ ID No: 114; SEQ ID No: 115; SEQ ID No: 116 or SEQ ID No: 117; and/or
   - LFR3 comprises or consists of the amino acid sequences set forth in SEQ ID No: 118; SEQ ID No: 119; SEQ ID No: 120; SEQ ID No: 121; SEQ ID No: 122 or SEQ ID No: 132; and/or
   - LFR4 comprises or consists of the amino acid sequences set forth in SEQ ID No: 123; SEQ ID No: 124.

In a particular embodiment of the invention, the antibody or antigen-binding fragment thereof, or antigen-binding antibody mimetic or modified antibody comprises:
- A heavy chain variable region comprising HFR1 of SEQ ID No: 91; HFR2 of SEQ ID No: 95; HFR3 of SEQ ID No: 102 and HFR4 of SEQ ID No: 108.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment of the invention, the antibody or antigen-binding fragment thereof, or antigen-binding antibody mimetic or modified antibody comprises:
- A heavy chain variable region comprising HFR1 of SEQ ID No: 92; HFR2 of SEQ ID No: 96; HFR3 of SEQ ID No: 103 and HFR4 of SEQ ID No: 108.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment of the invention, the antibody or antigen-binding fragment thereof, or antigen-binding antibody mimetic or modified antibody comprises:
- A heavy chain variable region comprising HFR1 of SEQ ID No: 92; HFR2 of SEQ ID No: 98; HFR3 of SEQ ID No: 106 and HFR4 of SEQ ID No: 108.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment of the invention, the antibody or antigen-binding fragment thereof, or antigen-binding antibody mimetic or modified antibody comprises:
- A heavy chain variable region comprising HFR1 of SEQ ID No: 93; HFR2 of SEQ ID No: 96; HFR3 of SEQ ID No: 104 and HFR4 of SEQ ID No: 108.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment of the invention, the antibody or antigen-binding fragment thereof, or antigen-binding antibody mimetic or modified antibody comprises:
- A light chain variable region comprising LFR1 of SEQ ID No: 111; LFR2 of SEQ ID No: 115; LFR3 of SEQ ID No: 120 and LFR4 of SEQ ID No: 124.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment of the invention, the antibody or antigen-binding fragment thereof, or antigen-binding antibody mimetic or modified antibody comprises:
- A light chain variable region comprising LFR1 of SEQ ID No: 110; LFR2 of SEQ ID No: 114; LFR3 of SEQ ID No: 119 and LFR4 of SEQ ID No: 124.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment of the invention, the antibody or antigen-binding fragment thereof, or antigen-binding antibody mimetic or modified antibody comprises:
- A light chain variable region comprising LFR1 of SEQ ID No: 110; LFR2 of SEQ ID No: 114; LFR3 of SEQ ID No: 122 and LFR4 of SEQ ID No: 124.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment of the invention, the antibody or antigen-binding fragment thereof, or antigen-binding antibody mimetic or modified antibody comprises:
- A light chain variable region comprising LFR1 of SEQ ID No: 112; LFR2 of SEQ ID No: 116; LFR3 of SEQ ID No: 121 and LFR4 of SEQ ID No: 124.

Such an antibody may comprise the CDRs of the parental chimeric antibody (namely HCDR1 of SEQ ID No: 4; HCDR2 of SEQ ID No: 6; HCDR3 of SEQ ID No: 8 or SEQ ID No: 144 or SEQ ID No: 148; LCDR1 of SEQ ID No: 12; LCDR2 of SEQ ID No: 14 and LCDR3 of SEQ ID No: 15).

In a particular embodiment, the antibody or antigen-binding fragment thereof, or antigen-binding antibody mimetic or modified antibody comprises a humanized antibody light chain variable domain and a humanized antibody heavy chain variable domain; wherein:
- the antibody heavy chain variable domain comprises:
   - VHCDR1 of the amino acid sequence set forth in SEQ ID No: 4; VHCDR2 of the amino acid sequence set forth in SEQ ID No: 67 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 4; VHCDR2 of the amino acid sequence set forth in SEQ ID No: 70 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 4; VHCDR2 of the amino acid sequence set forth in SEQ ID No: 72 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 62; VHCDR2 of the amino acid sequence set forth in SEQ ID No: 67 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 62; VHCDR2 of the amino acid sequence set forth in SEQ ID No: 70 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 62; VHCDR2 of the amino acid sequence set forth in SEQ ID No: 72 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 63; VHCDR2 of the amino acid sequence set forth in SEQ ID No: 67 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 63; VHCDR2 of the amino acid sequence set forth in SEQ ID No: 70 and VHCDR3 of the amino acid sequence set forth in SEQ ID No: 8 or SEQ ID No: 144 or SEQ ID No: 148; or
   - VHCDR1 of the amino acid sequence set forth in SEQ ID No: 63; VHCDR2 of the amino acid sequence set forth in SEQ ID No: 72 and VHCDR3 of the amino acid sequence set forth in SEQ ID No: 8 or SEQ ID No: 144 or SEQ ID No: 148; and
- The antibody light chain variable domain comprises:
   - VLCDR1 of the amino acid sequence set forth in SEQ ID No: 77; VLCDR2 of the amino acid sequence set forth in SEQ ID No: 14 and VLCDR3 of the amino acid sequence set forth in SEQ ID No: 15; or
   - VLCDR1 of the amino acid sequence set forth in SEQ ID No: 77; VLCDR2 of the amino acid sequence set forth in SEQ ID No: 14 and VLCDR3 of the amino acid sequence set forth in SEQ ID No: 89; or
   - VLCDR1 of the amino acid sequence set forth in SEQ ID No: 77; VLCDR2 of the amino acid sequence set forth in SEQ ID No: 81 and VLCDR3 of the amino acid sequence set forth in SEQ ID No: 15; or
   - VLCDR1 of the amino acid sequence set forth in SEQ ID No: 77; VLCDR2 of the amino acid sequence set forth in SEQ ID No: 81 and VLCDR3 of the amino acid sequence set forth in SEQ ID No: 89;
   - VLCDR1 of the amino acid sequence set forth in SEQ ID No: 77; VLCDR2 of the amino acid sequence set forth in SEQ ID No: 84 and VLCDR3 of the amino acid sequence set forth in SEQ ID No: 15; or
   - VLCDR1 of the amino acid sequence set forth in SEQ ID No: 77; VLCDR2 of the amino acid sequence set forth in SEQ ID No: 84 and VLCDR3 of the amino acid sequence set forth in SEQ ID No: 89.

It should be noted that these combinations of CDRs (VHCDRs and VLCDRs) may be combined with the humanized framework regions described herein, in particular with the following framework regions:
- HFR1 of SEQ ID No: 91; HFR2 of SEQ ID No: 95; HFR3 of SEQ ID No: 102 and HFR4 of SEQ ID No: 108; or
- HFR1 of SEQ ID No: 92; HFR2 of SEQ ID No: 96; HFR3 of SEQ ID No: 103 and HFR4 of SEQ ID No: 108; or
- HFR1 of SEQ ID No: 92; HFR2 of SEQ ID No: 98; HFR3 of SEQ ID No: 106 and HFR4 of SEQ ID No: 108; or
- HFR1 of SEQ ID No: 93; HFR2 of SEQ ID No: 96; HFR3 of SEQ ID No: 104 and HFR4 of SEQ ID No: 108; and
- A light chain variable region comprising LFR1 of SEQ ID No: 111; LFR2 of SEQ ID No: 115; LFR3 of SEQ ID No: 120 and LFR4 of SEQ ID No: 124; or
- A light chain variable region comprising LFR1 of SEQ ID No: 110; LFR2 of SEQ ID No: 114; LFR3 of SEQ ID No: 119 and LFR4 of SEQ ID No: 124; or
- A light chain variable region comprising LFR1 of SEQ ID No: 110; LFR2 of SEQ ID No: 114; LFR3 of SEQ ID No: 122 and LFR4 of SEQ ID No: 124; or
- A light chain variable region comprising LFR1 of SEQ ID No: 112; LFR2 of SEQ ID No: 116; LFR3 of SEQ ID No: 121 and LFR4 of SEQ ID No: 124.

In a particular embodiment of the invention, the antibody or antigen-binding fragment thereof, or antigen-binding antibody mimetic or modified antibody comprises a heavy chain variable domain comprising or consisting of the amino acid sequences set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41 or SEQ ID No: 42; in particular the heavy chain variable domain comprises or consists of the amino acid sequences set forth in SEQ ID No: 20; SEQ ID No: 21; SEQ ID No: 22; SEQ ID No: 23; SEQ ID No: 28: SEQ ID No; 29; SEQ ID No: 30; SEQ ID No: 36; SEQ ID No: 37; SEQ ID No: 38; or SEQ ID No: 39.

In a particular embodiment of the invention, the antibody or antigen-binding fragment thereof, or antigen-binding antibody mimetic or modified antibody comprises a light chain variable domain comprising or consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61; in particular the heavy chain variable domain comprises or consists of the amino acid sequences set forth in SEQ ID No: 43; SEQ ID No: 44; SEQ ID No: 45; SEQ ID No: 46; SEQ ID No: 47; SEQ ID No: 48; SEQ ID No: 50; SEQ ID No: 51: SEQ ID No: 52; SEQ ID No: 53; SEQ ID No: 54; SEQ ID No: 56; SEQ ID No: 57; SEQ ID No: 58; SEQ ID No: 59 or SEQ ID No: 60.

In a particular embodiment of the invention, the antibody or antigen-binding fragment thereof, or antigen-binding antibody mimetic or modified antibody comprises:
- A heavy chain variable domain comprising HCDR1 of SEQ ID No: 4; HCDR2 of SEQ ID No: 6, HCDR3 of SEQ ID No: 8 or SEQ ID No: 144 or SEQ ID No: 148, HFR1 of SEQ ID No: 90; HFR2 of SEQ ID No: 94; HFR3 of SEQ ID No: 101 and HFR4 of SEQ ID No: 107; and
- A light chain variable domain comprising LCDR1 of SEQ ID No: 12; LCDR2 of SEQ ID No: 14, LCDR3 of SEQ ID No: 15, LFR1 of SEQ ID No: 109; LFR2 of SEQ ID No: 113; LFR3 of SEQ ID No: 118 and LFR4 of SEQ ID No: 123.

In a particular embodiment of the invention, the antibody or antigen-binding fragment thereof, or antigen-binding antibody mimetic or modified antibody comprises a heavy chain variable domain of SEQ ID No: 10 and a light chain variable domain of SEQ ID No: 17.

In a particular embodiment of the invention, the antibody or antigen-binding fragment thereof, or antigen-binding antibody mimetic or modified antibody comprises:
- a heavy chain variable domain comprising or consisting of the amino acid sequences set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41 or SEQ ID No: 42; in particular the heavy chain variable domain comprises or consists of the amino acid sequences set forth in SEQ ID No: 20; SEQ ID No: 21; SEQ ID No: 22; SEQ ID No: 23; SEQ ID No: 28: SEQ ID No; 29; SEQ ID No: 30; SEQ ID No: 36; SEQ ID No: 37; SEQ ID No: 38; or SEQ ID No: 39;
- a light chain variable domain comprising or consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61; in particular the heavy chain variable domain comprises or consists of the amino acid sequences set forth in SEQ ID No: 43; SEQ ID No: 44; SEQ ID No: 45; SEQ ID No: 46; SEQ ID No: 47; SEQ ID No: 48; SEQ ID No: 50; SEQ ID No: 51: SEQ ID No: 52; SEQ ID No: 53; SEQ ID No: 54; SEQ ID No: 56; SEQ ID No: 57; SEQ ID No: 58; SEQ ID No: 59 or SEQ ID No: 60.

In a particular embodiment of the invention, the antibody or antigen-binding fragment thereof, or antigen-binding antibody mimetic or modified antibody comprises a heavy chain variable domain wherein HFR1 comprises or consists of amino acid sequence set forth in SEQ ID No: 19 (E/Q VQLV A/E/Q SG G/A/S G L/E V/L Q/K P/K PG G/A S L/V K/R/V L/V SC A/K AS).

In a particular embodiment of the invention, the antibody or antigen-binding fragment thereof, or antigen-binding antibody mimetic or modified antibody comprises a heavy chain variable domain wherein HFR2 comprises or consists of amino acid sequence set forth in SEQ ID No: 125 (WVR Q/A TP D/G R/K R/G/Q LELVA).

In a particular embodiment of the invention, the antibody or antigen-binding fragment thereof, or antigen-binding antibody mimetic or modified antibody comprises a heavy chain variable domain wherein HFR3 comprises or consists of amino acid sequence set forth in SEQ ID No: 126 (R F/V T/V I S/T RDN A/S K/T/V N/S TLY L/M Q/E M/L/I SSL K/R S/A EDTA M/V YYCPR).

In a particular embodiment of the invention, the antibody or antigen-binding fragment thereof, or antigen-binding antibody mimetic or modified antibody comprises a heavy chain variable domain wherein HFR4 comprises or consists of amino acid sequence set forth in SEQ ID No: 127 (WGQGT T/L L/V TVSS).

In a particular embodiment of the invention, the antibody or antigen-binding fragment thereof, or antigen-binding antibody mimetic or modified antibody comprises a heavy chain variable domain wherein LFR1 comprises or consists of amino acid sequence set forth in SEQ ID No: 128 (Q/A/E I V/Q LTQSP A/S/D I/S/F/T M/L/Q S A/S/L S/V P/V/T G/P E/D/K K/R V/A T M/I/L TC).

In a particular embodiment of the invention, the antibody or antigen-binding fragment thereof, or antigen-binding antibody mimetic or modified antibody comprises a heavy chain variable domain wherein LFR2 comprises or consists of amino acid sequence set forth in SEQ ID No: 129 (WYQQK S/P G/D T/K S/A P K/R RWIY).

In a particular embodiment of the invention, the antibody or antigen-binding fragment thereof, or antigen-binding antibody mimetic or modified antibody comprises a heavy chain variable domain wherein LFR3 comprises or consists of amino acid sequence set forth in SEQ ID No: 130 (G V/I P A/S RFSGSGSGT F/D Y S/T LTI S/N S M/L E/Q A/P ED A/F A T/V YYC).

In a particular embodiment of the invention, the antibody or antigen-binding fragment thereof, or antigen-binding antibody mimetic or modified antibody comprises a heavy chain variable domain wherein LFR4 comprises or consists of amino acid sequence set forth in SEQ ID No: 131 (FG P/G GTK L/V E L/I KR).

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 20; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 21; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 22; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 23; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 24; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 25; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 26; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 27; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 28; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 29; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 30; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 31; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 32; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 33; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 34; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 35; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 36; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 37; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 38; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 39; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 40; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 41; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

In a particular embodiment of the invention, the antibody or antigen-binding fragment thereof, or antigen-binding antibody mimetic or modified antibody comprises:
- a heavy chain variable domain comprising or consisting of the amino acid sequence set forth in SEQ ID No: 42; and
- a light variable domain comprising of consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

The invention relates to any of the above defined compound of the invention, for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders, dry eye syndrome; cancer diseases, in particular solid and liquid cancers, metastatic cancers, in particular carcinoma, in particular mammary carcinoma or colon carcinoma, or lung cancer or myeloid cancer, in particular leukemia, in particular a cancer wherein cancer cells express CMKLR1 or where the microenvironment of the tumor is invaded by cells expressing or overexpressing CMKLR1. In a particular embodiment, the invention relates to any of the above defined compound of the invention, for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders and dry eye syndrome. In another particular embodiment of the invention, the invention relates to any of the above defined compound of the invention, for use in the prevention and/or the treatment of a cancer, in particular solid and liquid cancers, metastatic cancers, in particular carcinoma, in particular mammary carcinoma or colon carcinoma, or lung cancer or myeloid cancer, in particular leukemia, in particular a cancer wherein cancer cells express CMKLR1 or where the microenvironment of the tumor is invaded by cells expressing or overexpressing CMKLR1.

In a particular embodiment, the invention relates to an anti-CMKLR1 compound selected from the group of an antibody, or antigen-binding fragment thereof, or antigen-binding antibody mimetic or modified antibody, which specifically binds to CMKLR1, said compound comprising:
- an antibody heavy chain variable domain comprising the three CDRs VHCDR1, VHCDR2 and VHCDR3, wherein:
   - VHCDR1 comprises or consists of the amino acid sequences set forth in SEQ ID No: 3; SEQ ID No: 4; SEQ ID No: 62; SEQ ID No: 63; SEQ ID No: 64 or SEQ ID No: 65; and
   - VHCDR2 comprises or consists of the amino acid sequences set forth in SEQ ID No: 5; SEQ ID No: 6; SEQ ID No: 66; SEQ ID No: 67; SEQ ID No: 68; SEQ ID No: 69; SEQ ID No: 70; SEQ ID No: 71 or SEQ ID No: 72; and
   - VHCDR3 comprises or consists of the amino acid sequences set forth in SEQ ID No: 7; SEQ ID No: 8; SEQ ID No: 73; SEQ ID No: 74, SEQ ID No: 75; SEQ ID No: 144; SEQ ID No: 145; SEQ ID No: 146; SEQ ID No: 147; SEQ ID No: 148; SEQ ID No: 149; SEQ ID No: 150 or SEQ ID No: 151; and/or
- an antibody light chain variable domain comprising the three CDRs VLCDR1, VLCDR2 and VLCDR3, wherein:
   - VLCDR1 comprises or consists of the amino acid sequences set forth in SEQ ID No: 11; SEQ ID No: 12; SEQ ID No: 76; SEQ ID No: 77; SEQ ID No: 78; SEQ ID No: 79 or SEQ ID No: 80; and
   - VLCDR2 comprises or consists of the amino acid sequences set forth in SEQ ID No: 13; SEQ ID No: 14; SEQ ID No: 81; SEQ ID No: 82; SEQ ID No: 83; SEQ ID No: 84; SEQ ID No: 85; SEQ ID No: 86; SEQ ID No: 87 or SEQ ID No: 88; and
   - VLCDR3 comprises or consists of the amino acid sequences set forth in SEQ ID No: 15 or SEQ ID No: 89;
for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders, dry eye syndrome; cancer diseases, in particular solid and liquid cancers, metastatic cancers, in particular carcinoma, in particular mammary carcinoma or colon carcinoma, or lung cancer or myeloid cancer, in particular leukemia, in particular a cancer wherein cancer cells express CMKLR1 or where the microenvironment of the tumor is invaded by cells expressing or overexpressing CMKLR1. In a particular embodiment, the invention relates to any of the above defined compound of the invention, for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders and dry eye syndrome. In another particular embodiment of the invention, the invention relates to any of the above defined compound of the invention, for use in the prevention and/or the treatment of a cancer, in particular solid and liquid cancers, in particular carcinoma, in particular mammary carcinoma or colon carcinoma, or myeloid cancer, in particular leukemia, in particular a cancer wherein cancer cells express CMKLR1 or where the microenvironment of the tumor is invaded by cells expressing or overexpressing CMKLR1.

In a particular embodiment of the invention, the antibody or antigen-binding fragment thereof, or antigen-binding antibody mimetic or modified antibody comprises:
- A heavy chain variable domain comprising HCDR1 of SEQ ID No: 4; HCDR2 of SEQ ID No: 6, HCDR3 of SEQ ID No: 8 or SEQ ID No: 144 or SEQ ID No: 148, HFR1 of SEQ ID No: 90; HFR2 of SEQ ID No: 94; HFR3 of SEQ ID No: 101 and HFR4 of SEQ ID No: 107; and
- A light chain variable domain comprising LCDR1 of SEQ ID No: 12; LCDR2 of SEQ ID No: 14, LCDR3 of SEQ ID No: 15, LFR1 of SEQ ID No: 109; LFR2 of SEQ ID No: 113; LFR3 of SEQ ID No: 118 and LFR4 of SEQ ID No: 123;
for use in the therapeutic treatment and/or the prevention of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders, dry eye syndrome; cancer diseases, in particular solid and liquid cancers, metastatic cancers, in particular carcinoma, in particular mammary carcinoma or colon carcinoma, or lung cancer or myeloid cancer, in particular leukemia, in particular a cancer wherein cancer cells express CMKLR1 or where the microenvironment of the tumor is invaded by cells expressing or overexpressing CMKLR1. In a particular embodiment, the invention relates to any of the above defined compound of the invention, for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders and dry eye syndrome. In another particular embodiment of the invention, the invention relates to any of the above defined compound of the invention, for use in the prevention and/or the treatment of a cancer, in particular solid and liquid cancers, in particular carcinoma, in particular mammary carcinoma or colon carcinoma, or myeloid cancer, in particular leukemia, in particular a cancer wherein cancer cells express CMKLR1 or where the microenvironment of the tumor is invaded by cells expressing or overexpressing CMKLR1.

In a particular embodiment of the invention, the antibody or antigen-binding fragment thereof, or antigen-binding antibody mimetic or modified antibody comprises a heavy chain variable domain of SEQ ID No: 10 and a light chain variable domain of SEQ ID No: 17;
for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders, dry eye syndrome; cancer diseases, in particular solid and liquid cancers, metastatic cancers, in particular carcinoma, in particular mammary carcinoma or colon carcinoma, or lung cancer or myeloid cancer, in particular leukemia, in particular a cancer wherein cancer cells express CMKLR1 or where the microenvironment of the tumor is invaded by cells expressing or overexpressing CMKLR1. In a particular embodiment, the invention relates to any of the above defined compound of the invention, for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders and dry eye syndrome. In another particular embodiment of the invention, the invention relates to any of the above defined compound of the invention, for use in the prevention and/or the treatment of a cancer, in particular solid and liquid cancers, in particular carcinoma, in particular mammary carcinoma or colon carcinoma, or myeloid cancer, in particular leukemia, in particular a cancer wherein cancer cells express CMKLR1 or where the microenvironment of the tumor is invaded by cells expressing or overexpressing CMKLR1.

In a particular embodiment of the invention, the antibody or antigen-binding fragment thereof, or antigen-binding antibody mimetic or modified antibody comprises:
- A heavy chain variable domain comprising HCDR1 of SEQ ID No: 4; HCDR2 of SEQ ID No: 6, HCDR3 of SEQ ID No: 8 or SEQ ID No: 144 or SEQ ID No: 148, and
   - HFR1 comprising or consisting of the amino acid sequences set forth in SEQ ID No: 91; SEQ ID No: 92 or SEQ ID No: 93; and
   - HFR2 comprising or consisting of the amino acid sequences set forth in SEQ ID No: 95; SEQ ID No: 96; SEQ ID No: 97; SEQ ID No: 98; SEQ ID No: 99 or SEQ ID No: 100; and
   - HFR3 comprising or consisting of the amino acid sequences set forth in SEQ ID No: 102; SEQ ID No: 103; SEQ ID No: 104; SEQ ID No: 105 or SEQ ID No: 106; and
   - HFR4 comprising or consisting of the amino acid sequence set forth in SEQ ID No: 108; and
- A light chain variable domain comprising LCDR1 of SEQ ID No: 12; LCDR2 of SEQ ID No: 14, LCDR3 of SEQ ID No: 15, and wherein
   - LFR1 comprising or consisting of the amino acid sequences set forth in SEQ ID No: 110; SEQ ID No: 111 or SEQ ID No: 112; and
   - LFR2 comprising or consisting s of the amino acid sequences set forth in SEQ ID No: 114; SEQ ID No: 115; SEQ ID No: 116 or SEQ ID No: 117; and
   - LFR3 comprising or consisting of the amino acid sequences set forth in SEQ ID No: 119; SEQ ID No: 120; SEQ ID No: 121; SEQ ID No: 122 or SEQ ID No: 132; and
   - LFR4 comprising or consisting of the amino acid sequence set forth in SEQ ID No: 124.

In a particular embodiment of the invention, the antibody or antigen-binding fragment thereof, or antigen-binding antibody mimetic or modified antibody comprises:
- a heavy chain variable domain comprising or consisting of the amino acid sequences set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41 or SEQ ID No: 42; in particular the heavy chain variable domain comprises or consists of the amino acid sequences set forth in SEQ ID No: 20; SEQ ID No: 21; SEQ ID No: 22; SEQ ID No: 23; SEQ ID No: 28: SEQ ID No; 29; SEQ ID No: 30; SEQ ID No: 36; SEQ ID No: 37; SEQ ID No: 38; or SEQ ID No: 39;
- a light chain variable domain comprising or consisting of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61; in particular the heavy chain variable domain comprises or consists of the amino acid sequences set forth in SEQ ID No: 43; SEQ ID No: 44; SEQ ID No: 45; SEQ ID No: 46; SEQ ID No: 47; SEQ ID No: 48; SEQ ID No: 50; SEQ ID No: 51: SEQ ID No: 52; SEQ ID No: 53; SEQ ID No: 54; SEQ ID No: 56; SEQ ID No: 57; SEQ ID No: 58; SEQ ID No: 59 or SEQ ID No: 60.
for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders, dry eye syndrome; cancer diseases, in particular solid and liquid cancers, metastatic cancer, in particular carcinoma, in particular mammary carcinoma, lung cancers or colon carcinoma, or myeloid cancer, in particular leukemia, in particular a cancer wherein cancer cells express CMKLR1 or where the microenvironment of the tumor is invaded by cells expressing or overexpressing CMKLR1. In a particular embodiment, the invention relates to any of the above defined compound of the invention, for use in the prevention and/or the treatment of a disease wherein the resolution of inflammation is delayed or disrupted, and/or a disease selected from the group of inflammatory diseases, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis; autoimmune diseases such as diabetes, in particular type I diabetes, psoriasis, lupus, rheumatoid arthritis, multiple sclerosis, Sjögren's syndrome, celiac disease, vasculitis, myasthenia gravis; infection diseases such as sepsis, peritonitis; degenerative diseases; wound healing disorders and dry eye syndrome. In another particular embodiment of the invention, the invention relates to any of the above defined compound of the invention, for use in the prevention and/or the treatment of a cancer, in particular solid and liquid cancers, in particular carcinoma, in particular mammary carcinoma or colon carcinoma, or myeloid cancer, in particular leukemia, in particular a cancer wherein cancer cells express CMKLR1 or where the microenvironment of the tumor is invaded by cells expressing or overexpressing CMKLR1.

In a particular embodiment of the invention, the antibody or antigen-binding fragment thereof or antigen-binding antibody mimetic or modified antibody has:
- a heavy chain variable domain comprising or consisting of the amino acid sequence set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41 or SEQ ID No: 42; in particular the heavy chain variable domain comprises or consists of the amino acid sequences set forth in SEQ ID No: 20; SEQ ID No: 21; SEQ ID No: 22; SEQ ID No: 23; SEQ ID No: 28: SEQ ID No; 29; SEQ ID No: 30; SEQ ID No: 36; SEQ ID No: 37; SEQ ID No: 38; or SEQ ID No: 39; and
- a light chain variable domain comprising or consisting of the amino acid sequence set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61; in particular the heavy chain variable domain comprises or consists of the amino acid sequences set forth in SEQ ID No: 43; SEQ ID No: 44; SEQ ID No: 45; SEQ ID No: 46; SEQ ID No: 47; SEQ ID No: 48; SEQ ID No: 50; SEQ ID No: 51: SEQ ID No: 52; SEQ ID No: 53; SEQ ID No: 54; SEQ ID No: 56; SEQ ID No: 57; SEQ ID No: 58; SEQ ID No: 59 or SEQ ID No: 60;
for use in the treatment of colitis or Crohn's disease in a subject that is refractory to corticosteroids and/or immunosuppressive treatment.

In another aspect, the invention relates to an anti-CMKLR1 compound as defined herein, which enhances the secretion of anti-inflammatory cytokines, in particular the secretion of I10, in particular by macrophages. In another aspect, the invention relates to an anti-CMKLR1 compound as defined herein, which enhances the secretion of anti-inflammatory cytokines, in particular the secretion of CCL17, in particular by macrophages. In another aspect, the invention relates to an anti-CMKLR1 compound as defined herein, which enhances the secretion of anti-inflammatory cytokines, in particular the secretion of I10 and/or CCL17, in particular by macrophages. The invention also relates to an anti-CMKLR1 compound as defined herein, which inhibits the secretion of pro-inflammatory cytokines, in particular IL12, in particular by macrophages. The cytokine secretion may be assessed by method known in the art, or by methods disclosed in the examples of the present invention.

In another aspect, the invention relates to an anti-CMKLR1 compound as defined herein, which inhibits the proliferation and/or the activation of dendritic cells, in particular human dendritic cells. Proliferation and/or activation of dendritic cells may be assessed by the method disclosed in the examples of the present invention.

In another aspect, the invention relates to a composition comprising an anti-CMKLR1 compound as described herein, in particular a pharmaceutical composition comprising an anti-CMKLR1 compound according to the invention and a further therapeutic agent, or pharmaceutical acceptable carrier. In a particular embodiment, the invention relates to a composition comprising an anti-CMKLR1 compound according to the invention and a therapeutic agent selected from the group consisting of immunomodulatory agent, immune checkpoint blocker, immune checkpoint activator, antibody, in particular anti-CD137 antibody (which targets 4-1BB, a TNF receptor superfamily member also known as CD137) or anti-SIRPa antibody (P84 - anti-mouse SIRPa from Merck Millipore).

In another aspect, the invention relates to a combination of compounds comprising an anti-CMKLR1 compound as described herein, in particular a pharmaceutical composition comprising an anti-CMKLR1 compound according to the invention and an anti-PD1 or an anti-PDL1 compound, in particular an anti-PD1 compound; such a compound is in particular selected from the group consisting of an antibody, an antigen-binding antibody fragment, an antigen-binding antibody mimetic, a small molecule like an aptamer or a peptide, a modified antibody, like but not limited to a humanized or a chimeric antibody, able to bind to PD1 or PDL1.

In another aspect, the invention relates to a combination of compounds comprising an anti-CMKLR1 compound as described herein, in particular a pharmaceutical composition comprising an anti-CMKLR1 compound according to the invention and an anti-SIRPa compound; such a compound is in particular selected from the group consisting of an antibody, an antigen-binding antibody fragment, an antigen-binding antibody mimetic, a small molecule like an aptamer or a peptide, a modified antibody, like but not limited to a humanized or a chimeric antibody, able to bind to SIRPa, in particular human SIRPa.

In another aspect, the invention concerns the therapeutic use of the anti-CMKLR1 compound of the invention, especially for inducing and/or enhancing the resolution of inflammation, in particular for inducing and/or enhancing the resolution of inflammation when said resolution is delayed or disrupted, in view of treating diseases wherein the extension of the inflammation is pathologic, or wherein the duration of the resolution of inflammation is pathologic.

In a particular embodiment of the invention, the anti-CKLMR1 compound binds CMKLR1 with an affinity (KD value) of at least 10E-8 M, more preferably with an affinity of at least 10E-9 M. The specific binding between the antibody, or antigen-binding fragment thereof, or antigen-binding antibody mimetic or modified antibody of the invention and CMKLR1 (or a region of CMKLR1 comprising the third extra-cellular loop, including amino acid sequences set forth in SEQ ID No: 2 and 18) implies that the antibody exhibits appreciable affinity for CMKLR1. "Appreciable affinity" includes binding with an affinity of about 10⁻⁸ M (KD) or stronger. Preferably, binding is considered specific when the binding affinity is between 10⁻⁸ M and 10⁻¹² M, optionally between 10⁻⁹ M and 10⁻¹⁰ M, in particular at least 10⁻⁹ M. Whether a binding domain specifically reacts with or binds to a target can be tested readily by, inter alia, comparing the reaction of said binding domain with a target protein or antigen with the reaction of said binding domain with proteins or antigens other than the target protein. Such an antibody of the invention specifically binds CMKLR1 and has an agonist effect towards the interaction between RvE1 and CMKLR1. Methods for determining antibody specificity and affinity by competitive inhibition are known in the art (see, e.g., Harlow et al., Antibodies: A Laboratory Manual, Cold Spring Harbor Laboratory Press, Cold Spring Harbor, NY (1998); Colligan et al., Current Protocols in Immunology, Green Publishing Assoc., NY (1992; 1993); Muller, Meth. Enzym., 92:589-601 (1983)). These methods include, but are not limited to, Biacore Analysis, Blitz analysis, flow cytometry and ELISA assay.

In a particular embodiment of the invention, the anti-CMKLR1 compound binds specifically to an epitope localized within the third extra-loop of CMKLR1, in particular to an epitope localized within amino acid residue sequences set forth in SEQ ID No: 2 or SEQ ID No: 18, in particular SEQ ID No: 2. An anti-CMKLR1 compound binding within this particular region of CMKLR1 may have an agonist property on CMKLR1, thereby mimicking the binding of RvE1 to CMKLR1.

In another aspect, the invention relates to an anti-CMKLR1 antibody or antigen-binding fragment thereof or antigen-binding antibody mimetic or modified antibody as defined here above, which has an agonist capability towards the interaction between RvE1 and CMKLR1, for use as a medicament.

In another aspect, the invention relates to an anti-CMKLR1 antibody or antigen-binding fragment thereof or antigen-binding antibody mimetic or modified antibody as defined here above, which has the capability to induce the activation of Akt and/or Erk protein(s) *in vitro* and/or *in vivo.* The activation of these proteins may be assessed by the methods described in the examples of the present invention. In particular, the anti-CMKLR1 antibody or antigen-binding fragment thereof or antigen-binding antibody mimetic or modified antibody has the capability to activate either Akt and/or Erk protein, or both, in macrophages, in particular in human macrophages.

The present invention also relates to a method of treatment in a subject in need thereof comprising administering to said subject an effective amount of an anti-CMKLR1 antibody or antigen-binding fragment thereof or antigen-binding antibody mimetic as defined above, which has an agonist capability towards the interaction between RvE1 and CMKLR1, or in other words which is an RvE1-agonist like factor or modulator.

Modifying the polarization of macrophages to favor anti-inflammatory cells can be useful in a number of pathologies or situations. As described above, this modification is particularly useful in the context of a disease selected from the group of inflammatory diseases, including but not limited to acute inflammatory diseases and chronic inflammatory diseases, inflammatory bowel disease, Crohn's disease, asthma, keratoconjunctivitis, periodontal disease, eczema, colitis, in particular ulcerative colitis or spontaneous colitis, diabetes, in particular type I diabetes, peritonitis, psoriasis, carcinoma, in particular mammary carcinoma or colon carcinoma, cancers, metastatic cancers, lung cancer, degenerative disease, infection disease, in particular sepsis, autoimmune diseases.

The present invention also relates to the use of an anti-CMKLR1 antibody or antigen-binding fragment thereof or antigen-binding antibody mimetic as defined above, which has an agonist capability towards the interaction between RvE1 and CMKLR1 in the manufacture of a medicament.

In another aspect, the invention relates to an anti-CMKLR1 antibody or antigen-binding fragment thereof or antigen-binding antibody mimetic or modified antibody, like but not limited to humanized or chimeric antibody, as defined here above, for its use in the treatment of a chronic inflammatory disease, in particular for treating chronic colitis.

In another aspect, the invention relates to an anti-CMKLR1 antibody or antigen-binding fragment thereof or antigen-binding antibody mimetic as defined above, which has an agonist activity towards the interaction between RvE1 and CMKLR1 for use in the treatment of a delayed or disrupted resolution of an inflammatory condition, in particular an inflammatory disease wherein its resolution is delayed or disrupted, and/or in the treatment or the prevention of a disease selected from the group of inflammatory diseases, including but not limited to acute inflammatory diseases and chronic inflammatory diseases, inflammatory bowel disease, Crohn's disease, asthma, keratoconjunctivitis, periodontal disease, eczema, colitis, in particular ulcerative colitis or spontaneous colitis, diabetes, in particular type I diabetes, peritonitis, psoriasis, carcinoma, in particular mammary carcinoma or colon carcinoma, cancers, degenerative disease, infection disease, in particular sepsis, autoimmune diseases.

As defined herein, "a delay or a disruption in the resolution of an inflammatory condition" occurs when the resolution of inflammation is delayed or disrupted as compared to a normal resolution (i.e. the resolution occurring in a patient who experiences normal resolution after an inflammatory event). A resolution delay or defect can result in an increased penetration of granulocytes at the inflammatory site. Hence, a resolution delay or defect may be assessed by quantification of granulocytes at the inflammatory site. Granulocyte population may be measured for example by histology, cytometry or indirect biochemical techniques such as elastase quantification by enzyme immunoassay or molecular quantification by PCR of granulocyte receptor 1). A resolution delay or defect may also be assessed by the determination of a delay in the apoptosis of granulocytes, measured for example by cytology using specific antibodies against annexin 5. A defect or a delay in the resolution of the inflammation may also be determined by assessing by quantifying the synthesis of pro-inflammatory cytokines such as TNF-alpha, IL8 or IL12 and anti-inflammatory cytokines such as IL-10. Cytokine secretion may be assessed by enzyme immunoassay or by PCR. A defect or a delay in the resolution of the inflammation may also be determined by assessing the activation of transcription factors involved in the synthesis of inflammatory cytokines, such as NF-kappaB which can be measured for example by nuclear translocation or by Western blot and/or by quantification of the level of degradation of IkappaB). A defect or a delay in the resolution of the inflammation may also be determined by quantifying specialized pro-resolving mediators (such as lipoxins, resolvins, protectins or maresins) or their precursors (like 17-HDOHE or 14-HDOHE) by mass spectrometry or enzyme immunoassay. A defect or a delay of the resolution then results in a defect of the synthesis of one or more of these mediators. A resolution defect or delay can also be determined when expression of the receptors of the resolution molecules is decreased. These receptors may be selected from the group comprising ALX, CMK1R1, GPR32 or GPR18. Alternatively or complementarily, the internalization and processing of those receptors into the cytoplasm may also be assessed. Alternatively or complementarily, expression of some receptors of inflammatory cytokines or lipids may also be assessed, an overexpression compared to a normal condition being significant of a delay or a defect in the resolution of the inflammation. These conditions may be measured by histology, cytology or PCR. The resolution defect can also result in a decreased or inhibited switch of M1 to M2 macrophages, a damage in phagocytosis or efferocytosis of the same cells. Hence, a delay or a defect of the resolution may be assessed by analyzing the switch of M1 to M2 macrophages in a particular condition as compared to a normal condition, as exemplified in the examples of the present invention.

According to a particular embodiment, anti-CMKLR1 compound can be used to treat an individual who has a cancer selected from the group consisting of mammary cancer, in particular mammary carcinoma cancer, melanoma, colon cancer, in particular colon carcinoma cancer, leukemia, in particular acuter myeloid leukemia, in particular when cancer cells over-express CMKLR1.

In an embodiment, the invention relates to an anti-human CMKLR1 antibody or antigen-binding fragment thereof or antigen-binding antibody mimetic or modified antibody as defined above, for its uses as defined above, wherein said anti-human CMKLR1 antibody or antigen-binding fragment thereof or antigen-binding antibody mimetic or modified antibody of the invention is administered to a patient presenting a CMKLR1-positive tumor.

The antibody or antigen-binding fragment thereof of the invention can be administered in a variety of suitable routes, e.g., intravenously (IV), subcutaneously (SC), or, intramuscularly (IM) to the subject. The anti-CMKLR1 compound can be administered alone or in combination with another therapeutic agent, e.g., a second human monoclonal antibody or antigen binding fragment thereof. In another example, the antibody is administered together with another agent, for example, an immunosuppressive agent, an erythropoiesis-stimulating agent (ESA), in combination with therapeutic cell compositions, and the like. In an embodiment, the invention relates to an anti-CMKLR1 compound or antigen-binding fragment thereof or antigen-binding antibody mimetic for its use as defined above, wherein the anti-CMKLR1 antibody or antigen-binding fragment is combined with a second therapeutic agent.

The administration of the second therapeutic agent can be simultaneous or not with the administration of the anti-CMKLR1 compound. Depending on the nature of the second agent, a co-administration can be prepared in the form of a combination drug (product), also known as a "combo". A combo is a fixed-dose combination that includes two or more active pharmaceutical ingredients combined in a single dosage form, which is manufactured and distributed in fixed doses. But the dose regimen and/or the administration route can also differ.

In a preferred embodiment, this second therapeutic agent is selected from the group consisting of chemotherapeutic agents, radiotherapy agents, immunotherapeutic agents, cell therapy agents (such as CAR-T cells), antibiotics and probiotics.

In particular, immunotherapeutic agents useful in the context of the invention are selected from the group consisting of therapeutic vaccines (DNA, RNA or peptide vaccines), immune checkpoint blockers or activators, in particular of adaptive immune cells (T or B lymphocytes) or immunoconjugates such as antibody-drug conjugates.

As used herein, the term "immunotherapeutic agents" refers in particular to agents that could take cancer vaccines from interesting biological phenomena to effective therapeutic agents including: T-cell growth factors to increase number and repertoire of naive T cells, growth factors to increase the number of dendritic cells (DCs), agonists to activate DCs and other antigen-pre senting cells (APCs), adjuvants to allow and augment cancer vaccines, agonists to activate and stimulate T cells, inhibitors of T-cell checkpoint blockade, T-cell growth factors to increase the growth and survival of immune T cells, agents to inhibit, block, or neutralize cancer cell and immune cell-derived immunosuppressive cytokine.

Numerous immune checkpoint blocker or activator are known in the art. In the context of the invention, examples of immune checkpoint blockers or activators of adaptive immune cells (B or T lymphocytes) that could be useful are anti-PDL1, anti-PD1, anti-CTLA4, anti-SIRPa; anti-CD137, anti-CD2, anti-CD28, anti-CD40, anti-HVEM, anti-BTLA, anti-CD160, anti-TIGIT, anti-TIM-1/3, anti-LAG-3, anti-2B4, and anti-OX40, anti-CD40 agonist, CD40-L, TLR agonists, anti-ICOS, ICOS-L and B-cell receptor agonists, in particular anti-CD137 and anti-SIRPa. In a particular embodiment of the invention, the second therapeutic agent is an anti-PDL1 or an anti-PD1 compound, in particular an anti-PD1 compound, and more particularly an anti-PD1 antibody. In a particular embodiment of the invention, the second therapeutic agent is an anti-SIRPa compound, in particular an anti-SIRPa antibody

Said immunotherapeutic agent can also be an antibody targeting tumoral antigen, particularly selected from the group consisting of anti-Her2, anti-EGFR, anti-CD20, anti-CD19, anti-CD52.

The antibody may be provided at an effective dose from about 1 ng/kg body weight to about 30 mg/kg body weight, or more. In specific embodiments, the dosage may range from 1 µg/kg to about 20 mg/kg, optionally from 10 µg/kg up to 10 mg/kg or from 100 µg/kg up to 5 mg/kg.

The term "effective dose" or "effective dosage" or "effective amount" is defined as an amount sufficient to achieve or at least partially achieve the desired effect. The term "effective dose" is meant to encompass an amount sufficient to cure or at least partially arrest the disease and its complications or alleviate the symptoms of the disease in a patient already suffering from the disease. Amounts or doses effective for this use will depend on the condition to be treated, the delivered antibody construct, the therapeutic context and objectives, the severity of the disease, prior therapy, the patient's clinical history and response to the therapeutic agent, the route of administration, the size (body weight, body surface or organ size) and/or condition (the age and general health) of the patient, and the general state of the patient's own immune system. The proper dose can be adjusted such that it can be administered to the patient once or over a series of administrations, and in order to obtain the optimal therapeutic effect.

Dosing for such purposes may be repeated as required, e.g. daily, semi-weekly, weekly, semi-monthly, monthly, or as required during relapses.

In another aspect, the invention relates to a pharmaceutical composition comprising an antibody or antigen-binding fragment thereof as defined above and a pharmaceutically acceptable carrier.

As used herein, a "pharmaceutical composition" is meant to encompass a composition suitable for administration to a subject or patient, such as a mammal, especially a human. In general, a "pharmaceutical composition" is sterile and is usually free of contaminants that are capable of eliciting an undesirable response within the subject (e.g., the compound(s) in the pharmaceutical composition is pharmaceutical grade). Pharmaceutical compositions can be designed for administration to subjects or patients in need thereof via a number of different routes of administration including oral, buccal, rectal, parenteral, intraperitoneal, intradermal, intracheal and the like.

As used herein, a "pharmaceutically acceptable carrier" is meant to encompass an excipient, diluent, carrier, and adjuvant that are useful in preparing a pharmaceutical composition that are generally safe, non-toxic and neither biologically nor otherwise undesirable, and include an excipient, diluent, carrier, and adjuvant that are acceptable for veterinary use as well as human pharmaceutical use. "A pharmaceutically acceptable carrier" as used herein includes both one and more than one such excipient, diluent, carrier, and adjuvant.

In particular, the invention relates to a pharmaceutical composition which comprises as an active ingredient an antibody or antigen-binding fragment thereof as defined above and a pharmaceutically acceptable carrier.

In another aspect, the invention relates to a therapeutic means, in particular a combination product means, which comprises as active ingredients: an anti-SIRPa antibody or antigen-binding fragment thereof or antigen-binding antibody mimetic as defined above and a second therapeutic agent, wherein said active ingredients are formulated for separate, sequential or combined therapy, in particular for combined or sequential use.

In particular, the invention relates to a combination product comprising an anti-CMKLR1 compound as defined above and a second therapeutic agent for simultaneous, separate or sequential use a medicament.

In an embodiment, the invention relates to a combination product as defined above, wherein the second therapeutic agent is selected from the group consisting of chemotherapeutic agents, radiotherapy agents, cell therapy agents, immunotherapeutic agents, antibiotics and probiotics.

In an embodiment, the invention relates to a combination product as defined above, wherein said immunotherapeutic agent is selected from the group consisting of therapeutic vaccines, immune checkpoint blockers or activators, in particular of adaptive immune cells (T and B lymphocytes) and antibody-drug conjugates.

In an embodiment, the invention relates to a combination product as defined above, wherein said immune checkpoint blocker or activator of adaptive immune cells (T and B lymphocytes) is selected from the group consisting of anti-PDL1, anti-PD1, anti-SIRPA, anti-CTLA4, anti-CD137, anti-CD2, anti-CD28, anti-CD40, anti-HVEM, anti-BTLA, anti-CD160, anti-TIGIT, anti-TIM-1/3, anti-LAG-3, anti-2B4, and anti-OX40, anti-CD40 agonist, CD40-L, TLR agonists, anti-ICOS, ICOS-L and B-cell receptor agonists, in particular selected from the group consisting of anti-PDL1, anti-PD1 and anti-CD137. In a particular embodiment of the invention, the second therapeutic agent is an anti-PDL1 or an anti-PD1 compound, in particular an anti-PD1 compound, and more particularly an anti-PD1 antibody. In a particular embodiment of the invention, the second therapeutic agent is an anti-SIRPa compound, in particular an anti-SIRPa antibody.

In one embodiment, said immunotherapeutic agent is an antibody targeting tumoral antigen, particularly selected from the group consisting of anti-Her2, anti-EGFR, anti-CD20, anti-CD19, anti-CD52.

In an aspect, the invention relates to a combination product as defined above, for simultaneous, separate or sequential use in the treatment of any condition susceptible of being improved or prevented by modifying macrophage polarization to anti-inflammatory macrophages.

In an embodiment, the invention relates to a method of treatment of any condition susceptible of being improved or prevented by modifying macrophage polarization to anti-inflammatory macrophages in a subject in need thereof comprising administering simultaneously, separately or sequentially to said subject an effective amount of a combination product as defined above.

In an embodiment, the invention relates to the use of a combination product as defined above in the manufacture of a medicament for the treatment any condition susceptible of being improved or prevented by modifying macrophage polarization to anti-inflammatory macrophages.

In an aspect, the invention relates to a combination product as defined above, for simultaneous, separate or sequential use in the treatment of a pathology selected from the group consisting of inflammatory diseases, including but not limited to acute inflammatory diseases and chronic inflammatory diseases, inflammatory bowel disease, Crohn's disease, asthma, keratoconjunctivitis, periodontal disease, eczema, colitis, in particular ulcerative colitis or spontaneous colitis, diabetes, in particular type I diabetes, peritonitis, psoriasis, carcinoma, in particular mammary carcinoma or colon carcinoma, cancers, metastatic cancers, lung cancer, degenerative disease, infection disease, in particular sepsis, autoimmune diseases or for use in vaccination.

In an embodiment, the invention relates to a method of treatment of a pathology selected from the group of inflammatory diseases, including but not limited to acute inflammatory diseases and chronic inflammatory diseases, inflammatory bowel disease, Crohn's disease, asthma, keratoconjunctivitis, periodontal disease, eczema, colitis, in particular ulcerative colitis or spontaneous colitis, diabetes, in particular type I diabetes, peritonitis, psoriasis, carcinoma, in particular mammary carcinoma or colon carcinoma, cancers, metastatic cancers, lung cancer, degenerative disease, infection disease, in particular sepsis, autoimmune diseases of a subject in need thereof comprising administering simultaneously, separately or sequentially to said subject an effective amount of a combination product as defined above.

In an embodiment, the invention relates to the use of a combination product as defined above, in the manufacture of a medicament for the treatment of a pathology selected from the group of inflammatory diseases, including but not limited to acute inflammatory diseases and chronic inflammatory diseases, inflammatory bowel disease, Crohn's disease, asthma, keratoconjunctivitis, periodontal disease, eczema, colitis, in particular ulcerative colitis or spontaneous colitis, diabetes, in particular type I diabetes, peritonitis, psoriasis, carcinoma, in particular mammary carcinoma or colon carcinoma, cancers, metastatic cancers, lung cancer, degenerative disease, infection disease, in particular sepsis, autoimmune diseases.

The invention is also related to a method for selecting an anti-CMKLR1 compound, said method comprising the following steps:
a) Providing a compound selected from the group of an antibody or an antigen-binding fragment thereof or a chimeric or humanized antibody said compound comprising:
   - an antibody heavy chain variable domain comprising the three CDRs VHCDR1, VHCDR2 and VHCDR3, wherein:
      - VHCDR1 comprises or consists of the amino acid sequences set forth in SEQ ID No: 4 or a mutated sequence thereof wherein amino acid residue(s) is(are) substituted; and
      - VHCDR2 comprises or consists of the amino acid sequences set forth in SEQ ID No: 6 or a mutated sequence thereof wherein amino acid residue(s) is(are) substituted; and
      - VHCDR3 comprises or consists of the amino acid sequences set forth in SEQ ID No: 8 or SEQ ID No: 144 or SEQ ID No: 148 or a mutated sequence thereof wherein amino acid residue(s) is(are) substituted providing amino acid residues at position 1 and 2 of SEQ ID No: 8 are respectively L and I; and
   - an antibody light chain variable domain comprising the three CDRs VLCDR1, VLCDR2 and VLCDR3, wherein:
      - VLCDR1 comprises or consists of the amino acid sequences set forth in SEQ ID No: 12 or a mutated sequence thereof wherein amino acid residue(s) is(are) substituted; and
      - VLCDR2 comprises or consists of the amino acid sequences set forth in SEQ ID No: 14 or a mutated sequence thereof wherein amino acid residue(s) is(are) substituted; and
      - VLCDR3 comprises or consists of the amino acid sequences set forth in SEQ ID No: 15 or a mutated sequence thereof wherein the amino acid residue(s) is(are) substituted;
b) Testing the capability of the compound to be a RvE1-like agonist of CMKLR1, in particular testing the capability of the compound to favor the resolution of inflammation; in particular on myeloid cells; and optionally
c) Testing the binding capability of the compound by BLITZ analysis or Biosensor to an epitope localized within the third loop E3 of CMKLR1, in particular the binding capability of the compound to a polypeptide comprising the amino acid residues of SEQ ID No: 2 or SEQ ID No: 152; and optionally
d) Testing the ability of the compound to not compete with the binding of chemerin to CMKLR1;
e) And when the binding capability of the compound tested in step c) is at least 10E-8 KD,
   i) Testing the capability of the compound to induce the phosphorylation of Akt and/or the phosphorylation of Erk after binding of the compound to CMKLR1, in particular the phosphorylation of Akt and the phosphorylation of Erk;
   ii) Testing the capability of the compound to enhance the secretion of anti-inflammatory cytokines, in particular IL10, more particularly IL10 and CCL17; and/or the capability of the compound to inhibit or reduce the secretion of pro-inflammatory cytokines, in particular IL12; in particular on myeloid cells expressing CMKLR1; and/or
   iii) Testing the capability of the compound to enhance the macrophage polarization to favor anti-inflammatory M2 type macrophages; in particular the capability of the compound to enhance the secretion of the phenotype marker CD200R; and/or
   iv) Testing the capability of the compound to inhibit the dendritic cells activation and/or proliferation.

The capabilities of the compound may be tested according to the examples disclosed in the examples of the present disclosure.

The invention also relates to a polynucleotide encoding an anti-CMKLR1 compound as defined herein. To this end, the invention also relates to a nucleic acid molecule, or a group of nucleic acid molecules, more particularly an isolated nucleic acid molecule(s) and/or a recombinant a nucleic acid molecule(s), which encode(s) any anti-CMKLR1 compound according to the present disclosure, more particularly which encode(s) a heavy chain variable domain comprising or consisting of the amino acid residues of sequences set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41 or SEQ ID No: 42 and/or a light chain variable domain comprising or consisting of the amino acid residues of sequences set forth in SEQ ID No: SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61. The nucleic acid molecule(s) may further comprise regulation sequences, like but not limited to enhancers, silencers, promoters, in particular expression promoters, signal peptide, for transcription and expression of the encoded heavy chain variable domain and/or the light chain variable domain.

The invention also relates to a vector comprising the polynucleotide as disclosed herein, or comprising the nucleic acid molecule(s) as disclosed herein. As used herein, a vector is a nucleic acid molecule used as a vehicle to transfer a genetic material into a cell, and in a preferred embodiment allows the expression of a polynucleotide inserted within the vector. The term vector encompasses plasmids, viruses, cosmids and artificial chromosomes. A vector generally comprises an origin of replication, a multicloning site, and a selectable marker. The vector itself is generally a nucleotide sequence, commonly a DNA sequence, that comprises an insert {transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences.

In another aspect, the invention relates to a cell, an isolated cell, a host cell, an isolated host cell, or a cell line comprising a vector as defined above. As used herein, these terms related to cells are intended to include any individual cell or cell culture that can be or has been recipient of vectors, exogenous nucleic acid molecules, and polynucleotides encoding the antibody construct of the present invention; and/or recipients of the antibody construct itself. The introduction of the respective material into the cell can be carried out by way of transformation, transfection and the like. These terms are also intended to include progeny or potential progeny of a single cell. Suitable host cells include prokaryotic or eukaryotic cells, and also include but are not limited to bacteria, yeast cells, fungi cells, plant cells, and animal cells such as insect cells and mammalian cells, *e.g.,* murine, rat, rabbit, macaque or human.

The following Figures and Examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

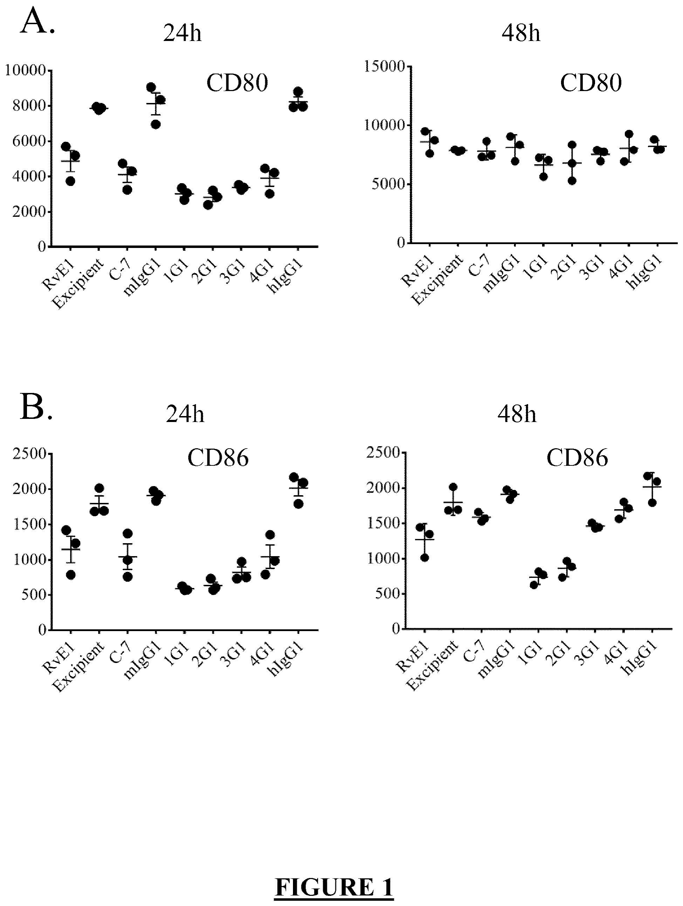

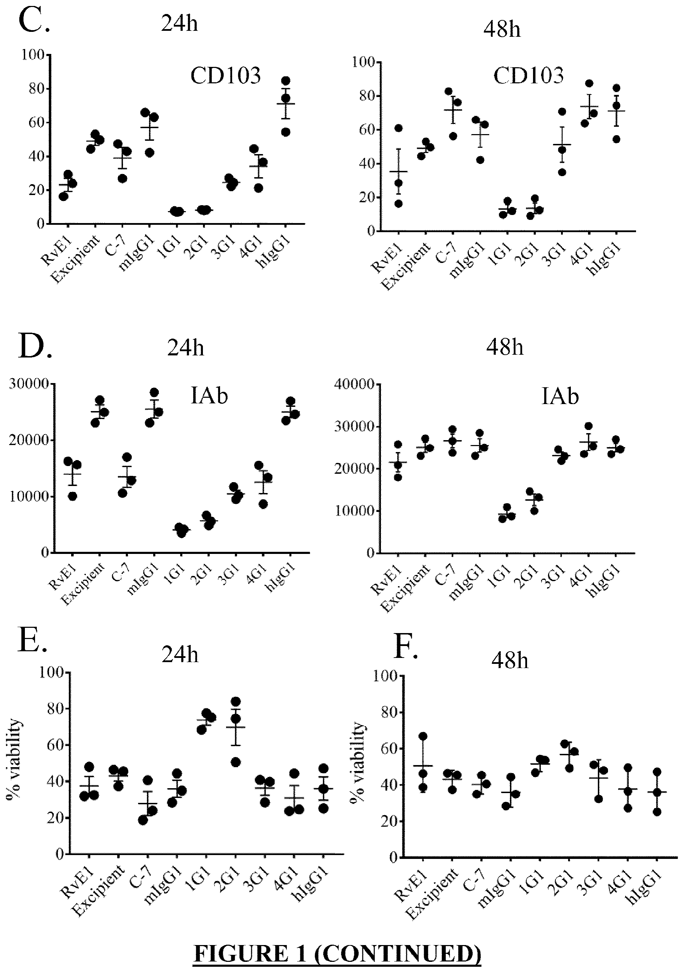

**Figure 1****. Effect of the anti-CMKLR1 variants 1G1, 2G1, 3G1 and 4G1 chimeric antibodies on the DCs maturation and differentiation.** Mice dendritic cells were incubated during maturation phase (24h or 48h) followed by a differentiation phase with excipient or RvE1 or anti-CMKLR1 antibodies variants (mutations on positions 1 and 2 of SEQ ID No. 8, corresponding to the VH-CDR3: 1G1 (LL), 2G1 (LI), 3G1 (IL) or 4G1 (II) or isotype controls (hIgG1 or mIgG)). Cells were then stained for FACS analysis with cell marker antibodies: **A.** CD80-PE; **B.** CD86-FITC; C. CD103-PerCPCy5.5; **D.** I/Ab-APC. Mean of Fluorescence was determined in each condition. **E** and **F** represent the viability of the cells measured by FACS using LIVE/DEAD^{®} kit from Life Technologies in each condition after 24 or 48 hours of maturation respectively.

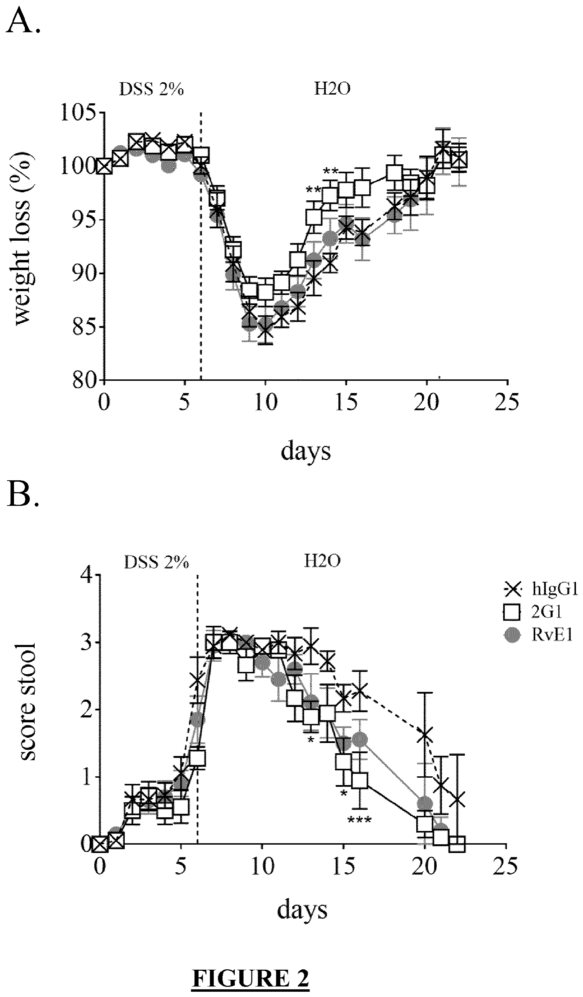

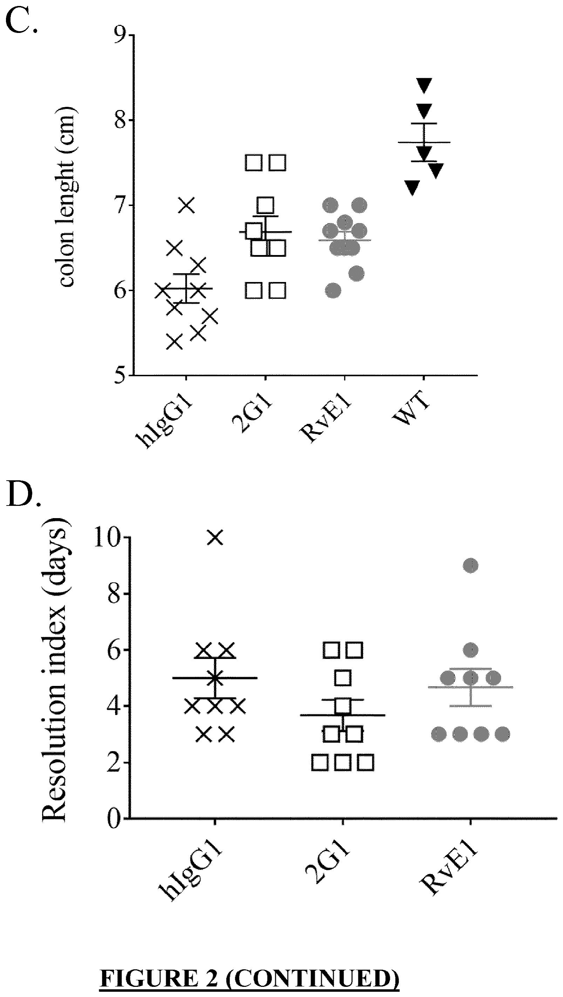

**Figure 2****. Effect of an anti-CMKLR1 antibody on a mice acute inflammatory colitis model induced by DSS.** Mice were injected after 6 days of DSS induction with isotype control hIgG1 (10µg per mouse) (x), RvE1 (1µg per mouse) daily (•), or 2G1 antibody (10µg per mouse) three times (□) for 5 days. Wild type mice, not treated, are represented by an ▼. **A.** Animal weight loss. **B.** Animal stool score **C.** Colon length. **D.** Resolution index.

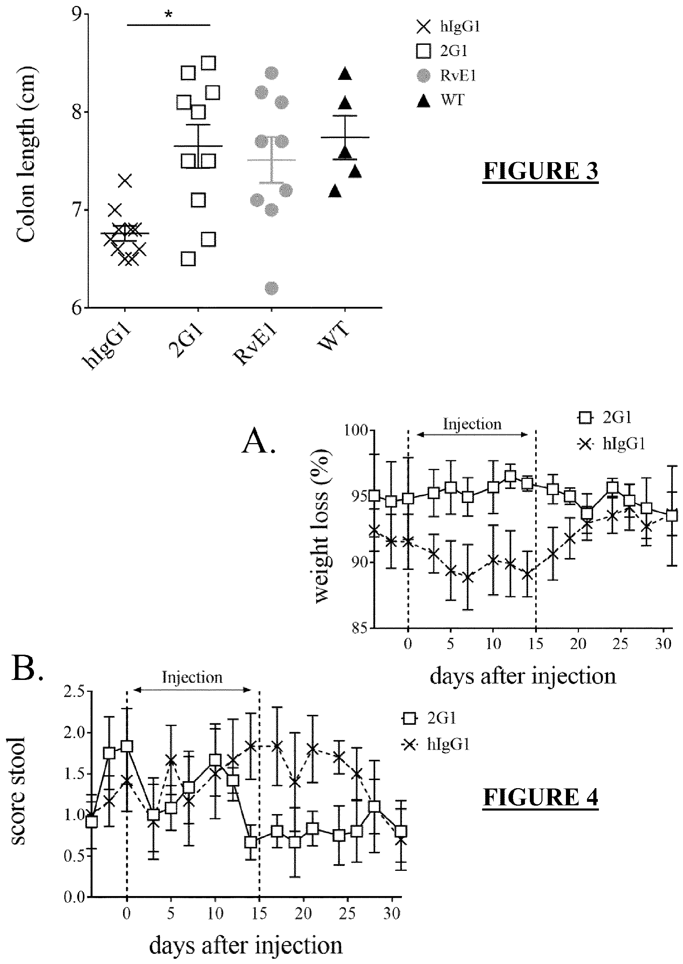

**Figure 3****. Effect of an anti-CMKLR1 antibody on a mice acute inflammatory colitis model induced by TNBS.** Mice received 200 µL of the haptenating agent TNBS at 5% in 50% ethanol on day 0 and then were injected with isotype control hIgG1 (10µg per mouse) (x), RvE1 (1µg per mouse) daily (•), or 2G1 antibody (10µg per mouse) three times (□) for 5 days, or non-treated (wild type animals) (▲). Mice were sacrificed colon length was measured in each condition.
**Figure 4****. Effect of an anti-CMKLR1 antibody on a IL10 KO mice chronic inflammatory colitis model.** Mice KO for IL10 develop a spontaneous inflammatory colitis. They were treated with an Anti-CMKLR1 antibody (2G1) (□) or isotype control (hIgG1) (x) intra-peritoneally (25µg/injection, 3 times a week). A. Animal weight loss during and after treatment. **B.** Animal score stool.

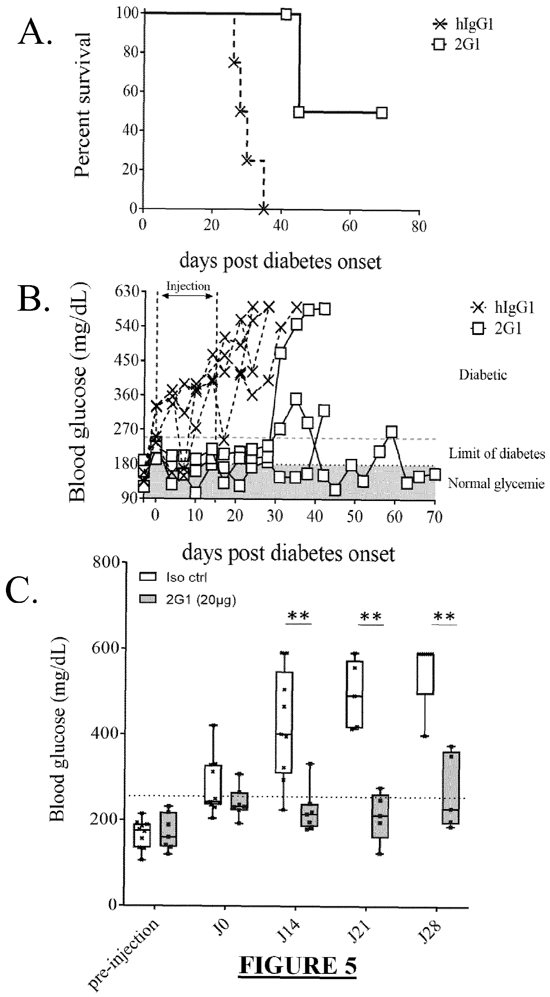

**Figure 5****. Effect of an anti-CMKLR1 antibody on a mice type1 non-obese diabetes model.** Mice develop spontaneous diabetes. When glycaemia was between 180 and 234 mg/dL, they were treated with anti-CMKLR1 antibody (□ in A and B, ■ in C) or isotype control antibody (x in A and B; □ in C) intraperitoneously at 20µg/injection three times a week for 2 weeks. **A.** Percentage of survival, **B.** individual representation of blood glucose concentration in mg/dL. **C.** pooled representation of blood glucose concentration in mg/dL.

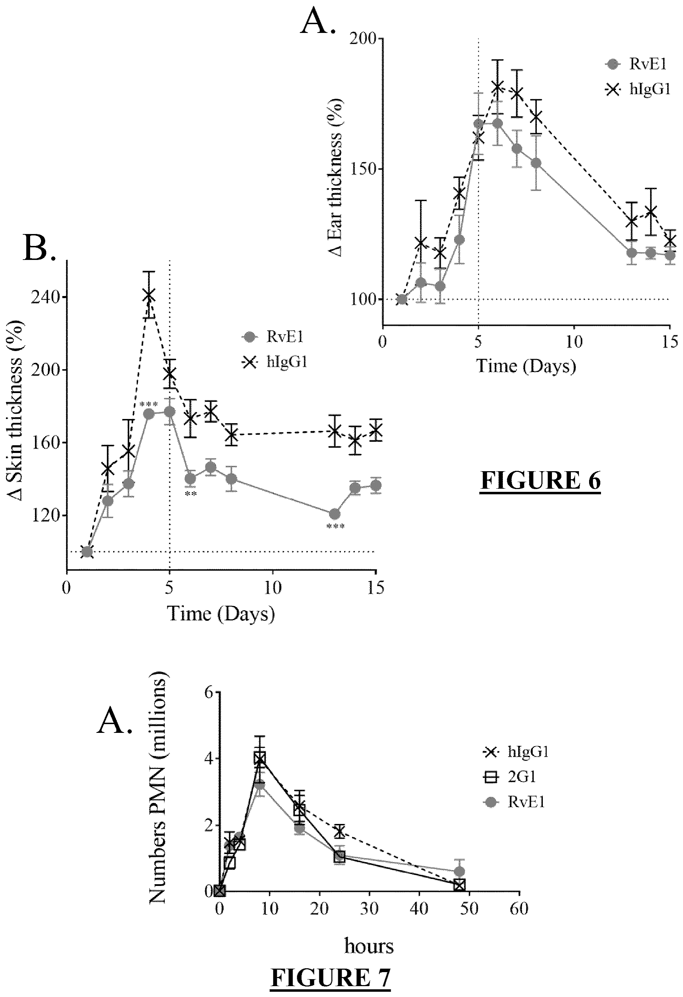

**Figure 6****. Effect of Resolvin E1 on an autoimmune disease such as mice psoriasis model.** Aldara treated mice for 6 consecutive days were injected intra-peritoneally with: RvE1 (1µg) (•) and PBS daily, and isotype control antibody (10µg) (x) at day 0, 2 and 4. **A.** Ear thicknesses and **B.** skin thicknesses were measured daily until the recovery.

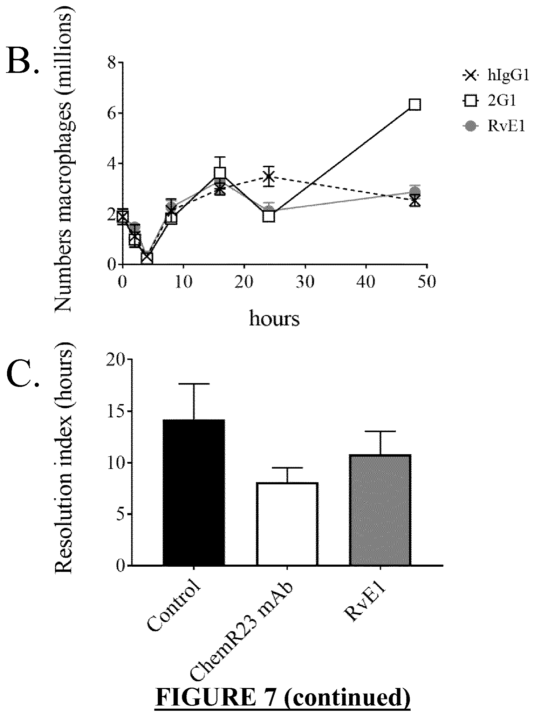

**Figure 7****. Effect of an anti-CMKLR1 antibody on a mice peritonitis model.** Preventive injection of anti-CMKLR1 was performed before Zymosan A injection (1mg per mouse in 1mL).: RvE1 (1µg per mouse) (•), 2G1 antibody (10µg per mouse) (□) or isotype control antibody (x). **A.** PMN numbered during the first 50 hours after Zymosan A injection. **B.** Macrophages numbered during the first 50 hours after Zymosan A injection. **C.** Resolution index.

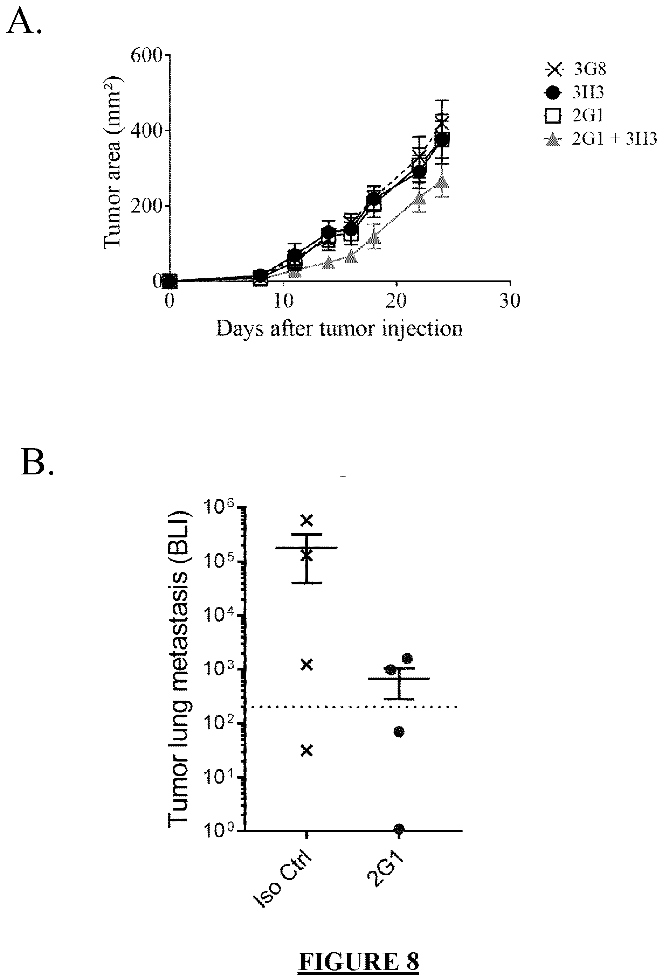

**Figure 8****. Effect of an anti-CMKLR1 antibody on a 4T1 breast tumor model.** Mice were inoculated with 4T1 cells (0,25 millions) in the mammary gland. Then the anti-CMKLR1 antibody (2G1) (A. □ B. •) or an anti-41BB antibody (3H3) (•) or both antibodies (▲) were injected twice at day 4 and day 7 (10µg/injection) or a control antibody (IgG1 isotype antibody clone 3G8) (x A. and B.) was injected three time a week for three weeks. A. The tumor area was measured 8 days after tumor injection and then every 2 days. B. Tumor lung metastasis was measured by Bioluminescence imaging (BLI) in animals treated with isotype control and anti-CMKLR1 antibody (n=4).

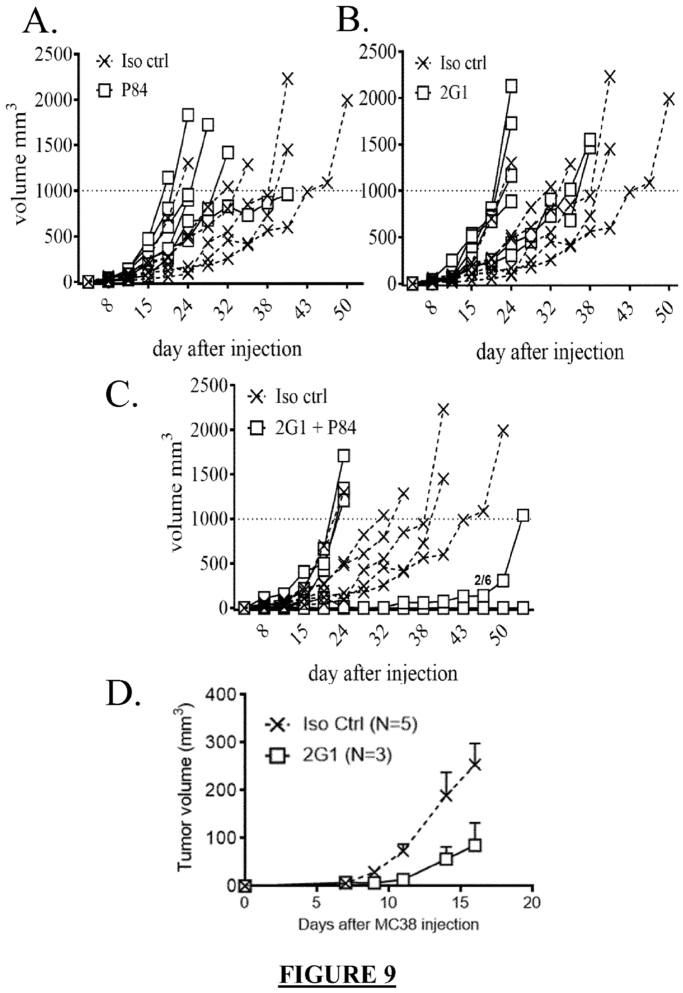

**Figure 9****. Effect of an anti-CMKLR1 antibody on 2 different colon carcinoma mice models.** Two mice models were studied, and animals received isotype control antibody (3G8) (x) or anti-CMKLR1 (2G1) (□) at 20µg/injection intraperitoneally for 3 weeks. **A-C.** Results in the CT26 colon carcinoma model comparing single treatment with p84 (A) and 2G1 (B) with combined therapy with both antibodies (C). D: Results in the MC38 colon carcinoma model.

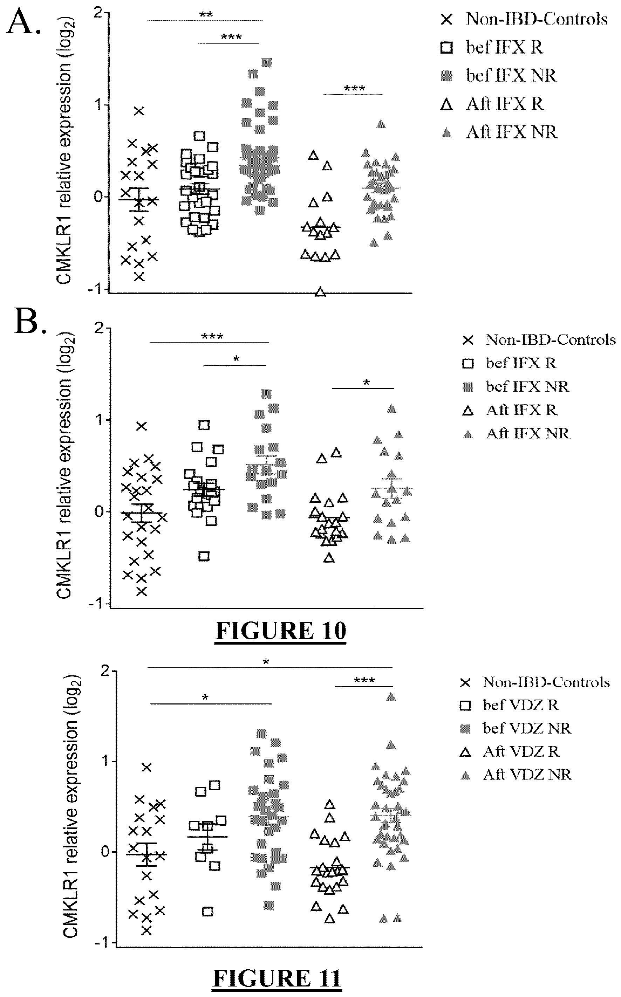

**Figure 10****. Expression of CMKLR1 on Ulcerative Colitis (UC) or Crohn's Disease patient biopsies before and after anti-TNFa treatment.** x represents controls; □ represents CMKLR1 expression in patient responding to corticosteroids treatment and/or immunosuppression treatment before Infliximab treatment; ■ represents CMKLR1 expression in patient non-responding to corticosteroids treatment and/or immunosuppression treatment before Infliximab treatment; Δ represents CMKLR1 expression in patient responding to corticosteroids treatment and/or immunosuppression treatment after Infliximab treatment; ▲ represents CMKLR1 expression in patient non-responding to corticosteroids treatment and/or immunosuppression treatment after Infliximab treatment. **A.** CMKLR1 transcript expression in Ulcerative colitis patients before and after Infliximab treatment. **B.** CMKLR1 transcript expression in Crohn's Disease patients before and after Infliximab treatment in inflamed colon biopsies.
**Figure 11****. Expression of CMKLR1 on UC patient biopsies before and after anti α4β7 (VDZ) treatment.** A. Represents the CMKLR1 (CMKLR1) transcript expression in Ulcerative colitis patients before and after Vedolizumab treatment in inflamed colon biopsies. R corresponds to patient responsive to VDZ treatment, NR corresponds to patient non-responsive to VDZ treatment.

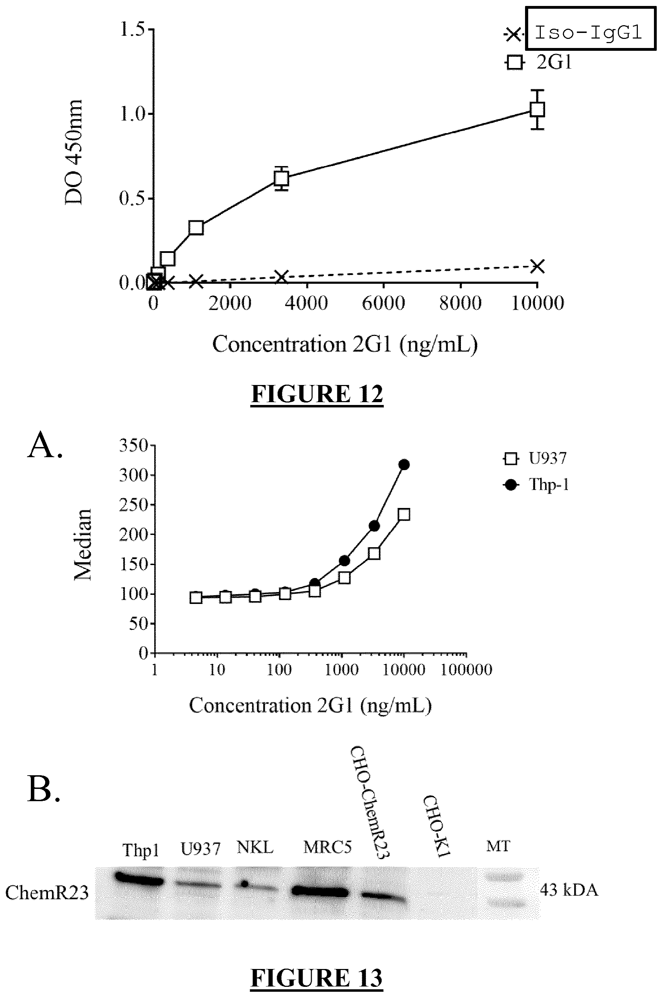

**Figure 12****. Binding analysis of the anti-CMKLR1 antibody on a CMKLR1 peptide by ELISA.** The binding of the 2G1 antibody (□) on the CMKLR1 EL3 loop (SEQ ID No: 18) was compared to the isotype control antibody (3G8) (x) by measuring the OD at 450nm by ELISA.
**Figure 13****. Study of CMKLR1 expression on different cell lines by FACS and western blot. A.** The protein expression at cell surface of CMKLR1 was determined using the 2G1 antibody at different concentration (ng/ml) on two human different T cell lines Thp1 and U937. **B.** the CMKLR1 protein expression was tested by western blot on T cell lines (Thp1 and U937), fibroblast cell line MRC5, NK cell lines NKL and negative and transduced CHO cells for CMKLR1.

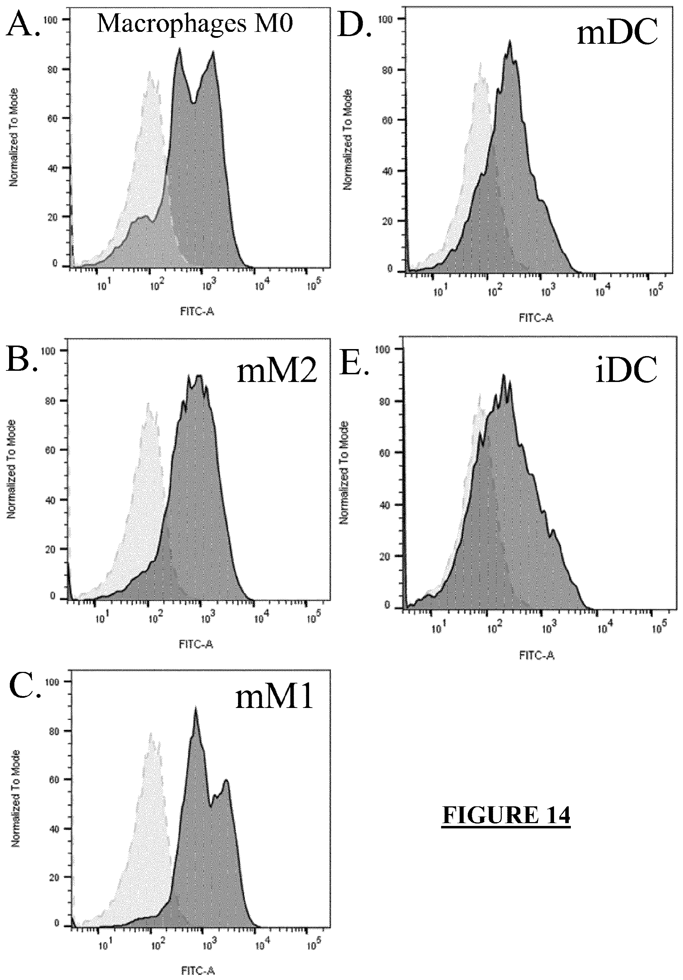

**Figure 14****. Expression of CMKLR1 on mice myeloid cells by FACS.** After differentiation, mice myeloid lineage cells were analyzed for their CMKLR1 expression at cell surface. **A.** expression on macrophage monocytes (M0). **B** and **C.** expression on macrophages (mM1 and mM2). **D** and **E.** expression on Dendritic cells (mDC and iDC).

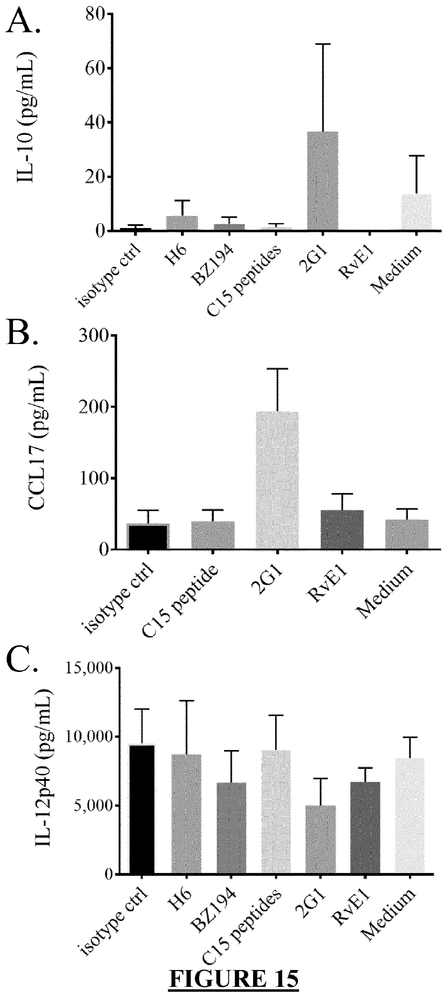

**Figure 15****. Study of the inflammatory cytokine secretion by human macrophages during CMKLR1 activation.** After differentiation of human macrophages M1 or M2, cells were incubated in presence of medium, isotype control, anti-CMKLR1 antibodies (H6 and BZ194), C15 peptides, 2G1 anti-CMKLR1 antibody or RvE1. Secretion of IL10, CCL17 and IL12p40 were assessed by ELISA. Cytokines secretion is measured in the supernatant by using an ELISA kit from BD. Supernatant were diluted at 1/10 for IL10 cytokines, 1/50 for CCL17 cytokines and 1/100 for IL12p40 cytokines. **A.** IL10 cytokine secretion by M2 cells. **B.** CCL17 cytokine secretion by M2 cells. **C.** represents IL12 cytokine secretion by M1 cells.

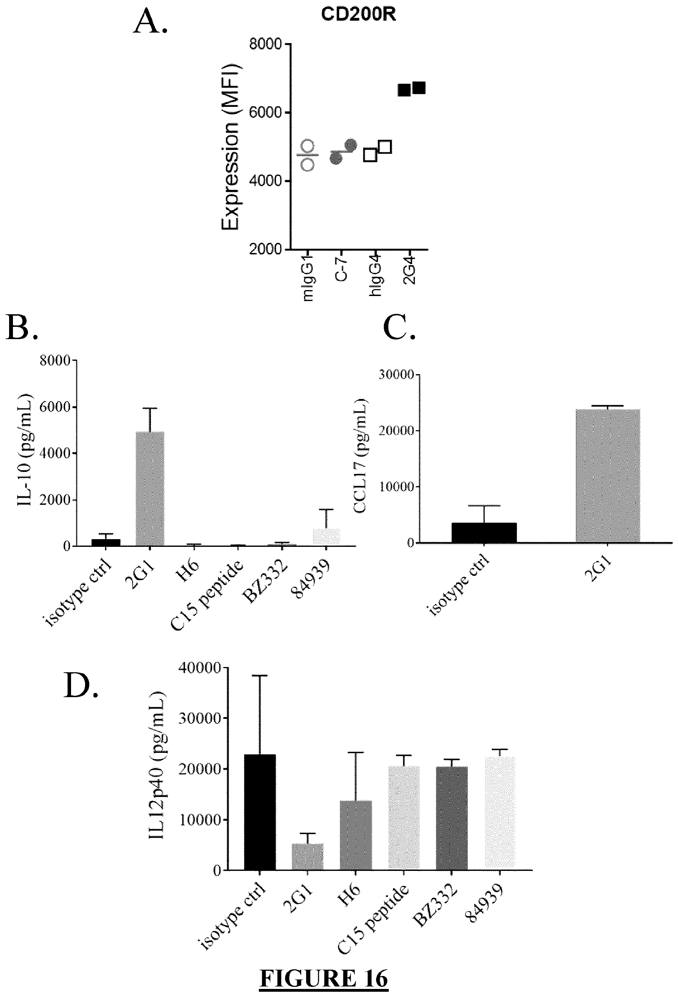

**Figure 16****. Study of the inflammatory cytokine secretion by human macrophages during CMKLR1 activation.** After differentiation of human monocytes, cells were incubated in presence of different isotype controls (mlgG4; hIgG4 or hIgG1 at 2 µg/ml); C7 commercial anti-CMKLR1 antibody (2 µg/ml); 2G4 or 2G1 antibodies (2 µg/ml), or other commercial anti-CMKLR1 antibodies (H6 (2µg/ml), BZ332 (2µg/ml) or 84939 (2 µg/ml), C15 peptides (10nM), or RvE1 (10ng/ml). **A.** Expression of the CD200R marker measured by FACS. **B.** IL10 cytokine secretion by M2 cells. **C.** CCL17 cytokine secretion by M2 cells. **D.** represents IL12 cytokine secretion by M1 cells. Secretion of IL10, CCL17 and IL12p40 were assessed by ELISA.

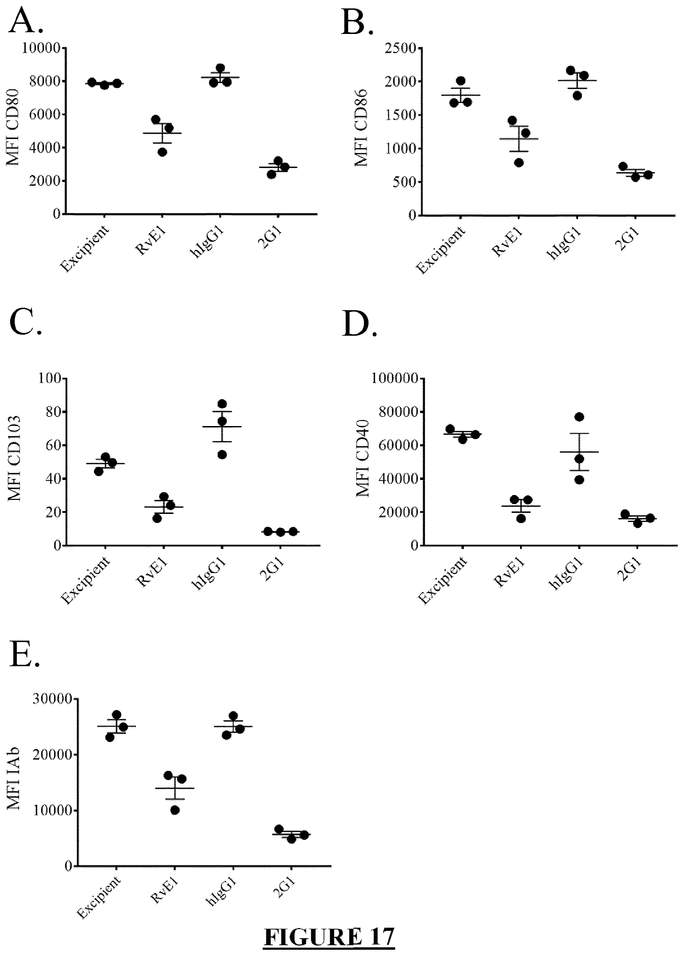

**Figure 17****. Study of the myeloid cell activation markers after CMKLR1 pathway activation.** Dendritic cells incubated in excipient, RvE1, 2G1 or isotype control (hlgG1) were then stained for FACS analysis with marker antibodies: **A.** CD80-PE. **B.** CD86-FITC. **C.** CD103-PerCPCy5.5. **D.** CD40-PeCy7. **E.** I/Ab-APC. Mean of Fluorescence was determined in each condition.

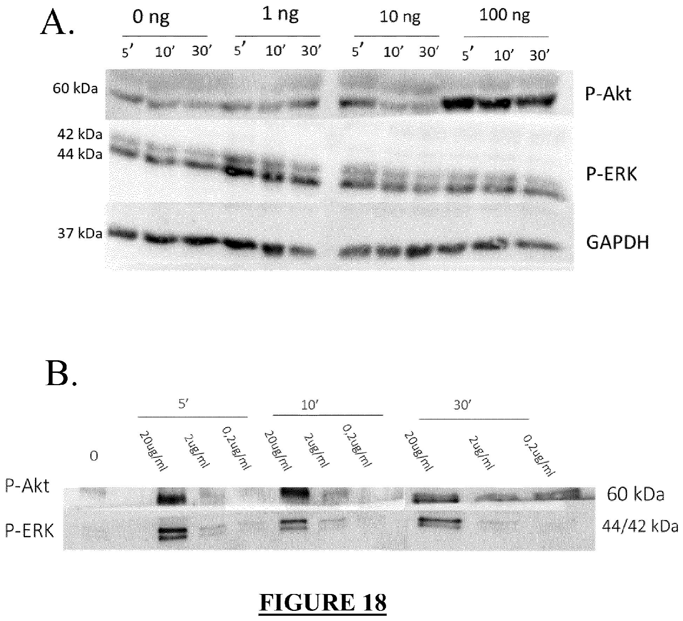

**Figure 18****. Study of the CMKLR1 pathway activation: Akt and Erk phosphorylation.** Western blot analysis of the ERK and AKT activation pathway after 5, 10 and 30 minutes of mice M1 cell incubation with 2G1 or RvE1. The Phosphorylated protein Akt or Erk were assessed using P-Akt antibody or P-Erk antibody (p44/42). **A.** Erk and Akt activation with RvE1. **B.** Erk and Akt activation with 2G1.

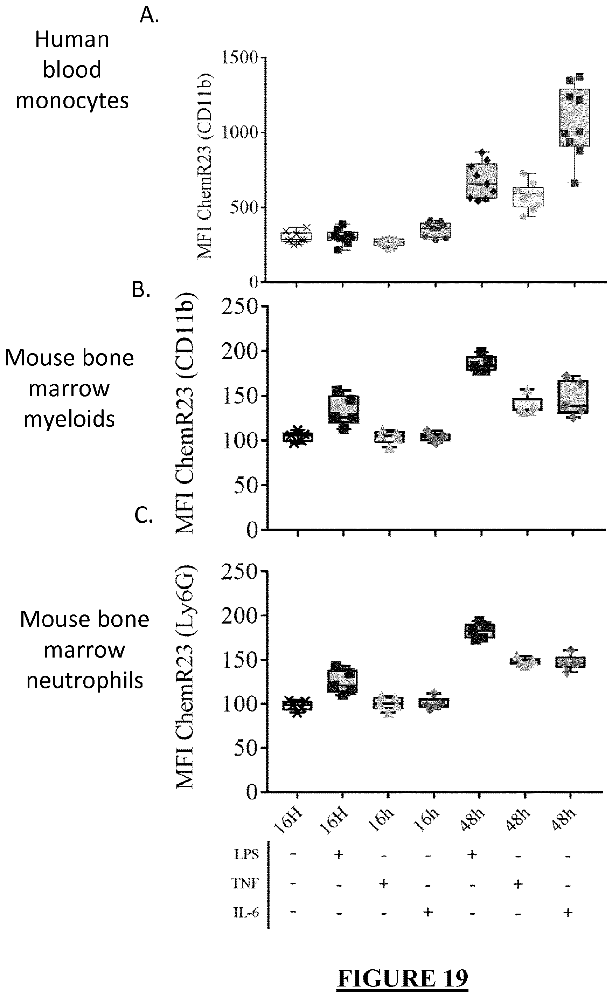

**Figure 19****. Expression of CMKLR1 on human monocytes and mice bone marrow cells stimulated with pro-inflammatory stimuli.**
   After 16 or 48 hours of stimulation of the cells with LPS, TNFa or IL6, expression of the CMKLR1 (ChemR23) was measured by FACS. A. Results on human blood monocytes, B. and C. Results on myeloid and neutrophil cells from mice bone marrow.

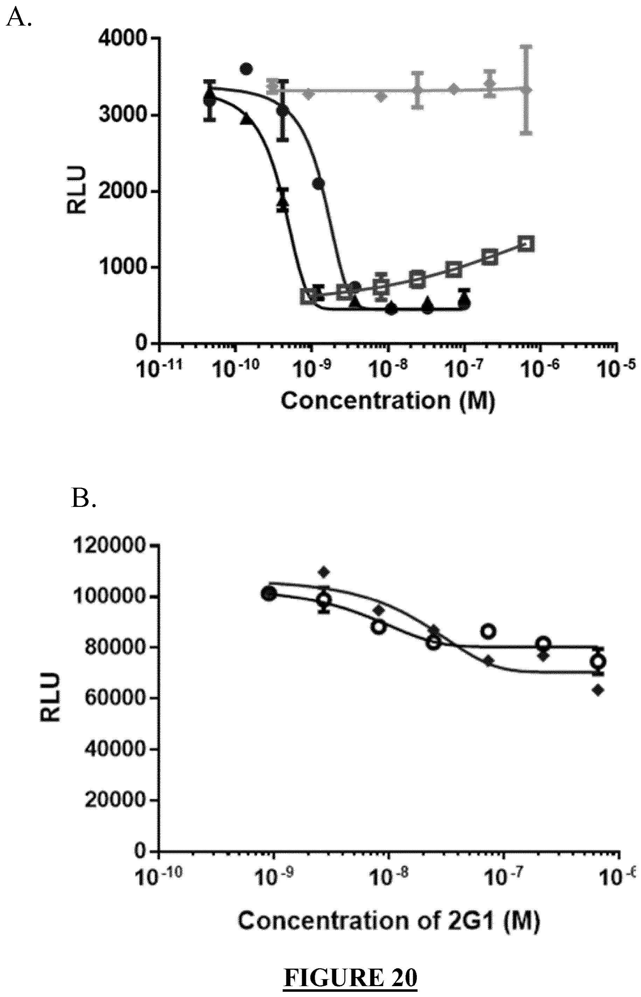

**Figure 20****. Competition study on chemerin-CMLKR1 interaction with anti-CMKLR1 antibodies.**
   A. Inhibition of the cAMP by Chemerin from two different providers were tested from DiscoverX (•) or R&D System (▲) or combined with anti-CMKLR1 antibody (2G1) alone ( ◊) or combined with chemerin from DiscoverX (□) .
   B. beta Arrestin activation in presence of anti-CMKLR1 antibody at different concentrations from 1µM to 1nM and Chemerin 2nM (◊) or 6nM (•)

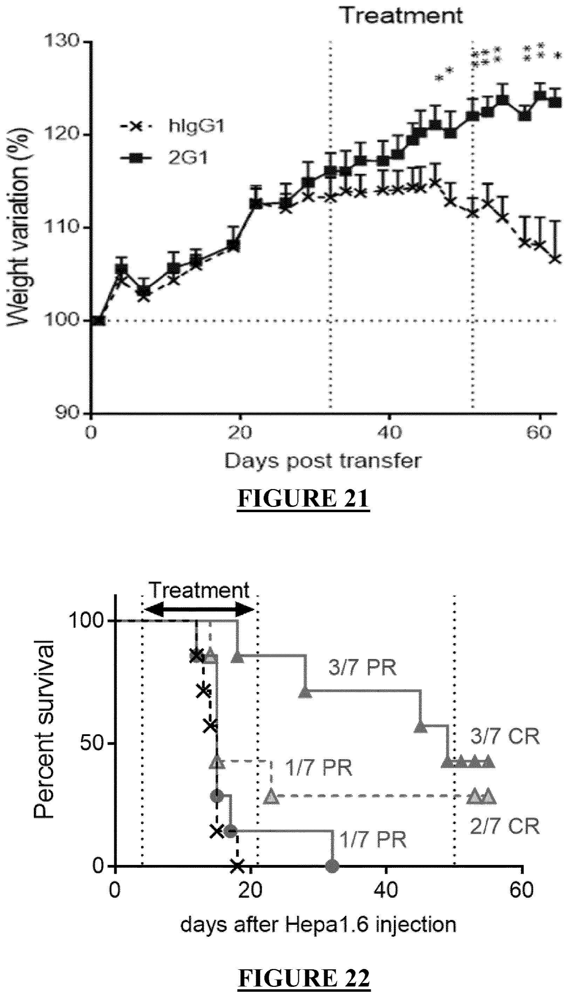

**Figure 21****. Effect of an anti-CMKLR1 antibody in the CD45Rb^{high} T-cell transfer chronic colitis mouse model.**
   Weight variation of treated animals was followed up to sixty days. Animals were treated with isotype control hIgG1 (x) or anti-CMKLR1 antibody (■).
**Figure 22****. Effect of an anti-CMKLR1 antibody on an Hepatocarcinoma mice model (HCC model).** Anti-tumor effect of anti-CMKLR1 antibody (2G1, 0.8mg/kg) i.p. administration three times a week for 2 weeks in combination (▲) or not (•) with two injections on day 4 and 8 of anti-PD1 mAb (*RMP1-14* clone, 8mg/kg) or with injections (twice a week) of anti-PD-1 antibody alone (Δ). Mice have been treated during 2 weeks in an orthotropic model of murine hepatoma (2.5.10^6 of Hepa 1.6 cells injected through the portal vein on day 0). Isotype control antibody was used at 0,8 mg/kg three times a week for 2 weeks. Mice response were considered partial (PR) when mice survive few days to 1 month after stop of treatment or complete (CR) when mice survive over 1 month or cured when they survive three times longer than the time necessary to all control mice dye.

### EXAMPLES

### Production and Selection of anti-CMKLR1 antibody

Several antibodies having different CDR sequences within their heavy and light chain variable domains have been synthetized. The distinct antibodies were tested for their ability to induce maturation and differentiation of dendritic cells towards a pro-inflammatory pathway or an anti-inflammatory pathway. Considering the divergent results, the inventors compared obtained amino acid sequences of the heavy and light chain variable domains and determined that the amino acid difference in HCDR3 might be material to said experimental results. They selected antibody 2G1 (SEQ ID No: 10 and SEQ ID No: 17) to assess its properties in resolving inflammatory status of at least influencing said status in resolution phase.

As shown in **Fig. 1****;** the 2G1 and 1G1 (another synthetized antibody) antibodies were able to inhibit DCs activation and/or maturation in a stronger manner than other synthetized antibodies (3G1 and 4G1) cells towards a pro-inflammatory pathway since level of detection of DCs expressing CD103 and IAb treated with 2G1 was lower than DCs cells treated with C7 antibody (the method used in this assay is described in point 10.2 of the examples). As shown in Fig. 1E and 1F, the viability of cells is enhanced after treatment with 1G1 or 2G1 antibodies as compared to cells treated with others antibodies, including C7, or Resolvin E1.

The 2G1 was humanized using *in silico* CDR grafting method, a method of humanization well known in the art. The resulting humanized sequences from the CDR and FR regions, as well as from the variable heavy chain and light chain are described in the following tables.

**Table 1: Sequences of the wild type (SEQ ID No: 4, SEQ ID No: 6 and SEQ ID No: 144) and humanized CDRs other SEQ ID) of the heavy chain variable domain of an anti-CMKLR1 compound.**

| SEQ ID No | | Sequences |
|---|---|---|
| 4 | VHCDR1 WT | GFTFSSYGMS |
| 6 | VHCDR2 WT | TINRYGGSTYYPDSVKG |
| 144 | VHCDR3 WT | LIYYGNEGDS |
| 62 | VHCDR1 | GFTFSSYAMS |
| 63 | VHCDR1 | GYTFTSYGMS |
| 64 | VHCDR1 | GYTFTSYAMS |
| 65 | VHCDR1 | GYTFTSYAMN |
| 66 | VHCDR2 | TINRYGGSTYYAASVKG |
| 67 | VHCDR2 | TISRSGGSTYYAASVKG |
| 68 | VHCDR2 | TINRYGGSTYYPDSFKG |
| 69 | VHCDR2 | TINRYGGSTYYAQKFQG |
| 70 | VHCDR2 | IINRNGGSTYYAQKFQG |
| 71 | VHCDR2 | TINRYGGSPYYAQGFTG |
| 72 | VHCDR2 | TINRYGGNPYYAQGFTG |
| 145 | VHCDR3 | LIYYGNEGES |
| 146 | VHCDR3 | LIYYGNEGDT |
| 147 | VHCDR3 | LIYYGNEGET |

**Table 2: Sequences of the wild type SEQ ID No: 12; SEQ ID No: 14 and SEQ ID No: 15) and humanized CDRs (from SEQ ID No: 76 to SEQ ID No: 89) of the light chain variable domain of an anti-CMKLR1 compound.**

| SEQ ID No | | Sequences |
|---|---|---|
| 12 | VLCDR1 WT | SASSSVSFMH |
| 14 | VLCDR2 WT | DTTKLTS |
| 15 | VLCDR3 WT | QQWNSKPPLT |
| 76 | VLCDR1 | RASQSVSFMH |
| 77 | VLCDR1 | RASQSVSFLH |
| 78 | VLCDR1 | RASQGISFLA |
| 79 | VLCDR1 | RASQSVSFLA |
| 80 | VLCDR1 | RASQSISFLH |
| 81 | VLCDR2 | DATKLTS |
| 82 | VLCDR2 | DASKLTS |
| 83 | VLCDR2 | DASKLES |
| 84 | VLCDR2 | DATKSTS |
| 85 | VLCDR2 | DASKSFS |
| 86 | VLCDR2 | DATK RTT |
| 87 | VLCDR2 | DAS KSTS |
| 88 | VLCDR2 | DASKRTT |
| 89 | VLCDR3 | QQWQSKPPLT |

**Table 3: Sequences of the wild type, humanized and consensus FRs of the heavy chain variable domain of an anti-CMKLR1 compound.**

| SEQ ID No | | SEQUENCES |
|---|---|---|
| 90 | *HFR1 WT* | EVQLVASGGGLVQPGGSLKLSCAAS |
| 91 | *HFR1* /*1* | EVQLVESGGGLVQPGGSLRLSCAAS |
| 92 | *HFR1* /*2* | QVQLVQSGAEVKKPGASVKVSCKAS |
| 93 | *HFR1* /*3* | QVQLVQSGSELKKPGASVKVSCKAS |
| 94 | *HFR2 WT* | WVRQTPDRRLELVA |
| 95 | *HFR2* /*1* | WVRQAPGKGLELVA |
| 96 | *HFR2* /*2* | WVRQAPGQGLELVA |
| 97 | *HFR2* /*3* | WVRQAPGQGLELVA |
| 98 | *HFR2* /*4* | WVRQAPGKGLELVS |
| 99 | *HFR2* /*5* | WVRQAPGQGLELVG |
| 100 | *HFR2* /*6* | WVRQAPGQGLELMG |
| 101 | *HFR3 WT* | RFTISRDNAKNTLYLQMSSLKSEDTAMYYCPR |
| 102 | *HFR3* /*1* | RFTISRDNSKNTLYLQMNSLRAEDTAVYYCPK |
| 103 | *HFR3* /*2* | RVTITRDNSTSTLYMELSSLRSEDTAVYYCPR |
| 104 | *HFR3* /*3* | RFVISRDNSVSTLYLQISSLKAEDTAVYYCPR |
| 105 | *HFR3* /*4* | RVTITRDTSTSTVYMELSSLRSEDTAVYYCPR |
| 106 | *HFR3* /*5* | RFVISRDTSVSTAYLQISSLKAEDTAVYYCPR |
| 107 | *HFR4 WT* | WGQGTTLTVSS |
| 108 | *HFR4* | WGQGTLVTVSS |
| 19 | HFR1 con. | XVQLVXSGXGXXXXPGXSXXXSCXAS |
| 125 | HFR2 con. | WVRXTPXXXLELVA |
| 126 | HFR3 con | RXXIXRDNXXXTLYXXXSSLXXEDTAXYYCPR |
| 127 | HFR4 con. | WGQGTXXTVSS |

**Table 4: Sequences of the wild type, humanized and consensus FRs of the light chain variable domain of an anti-CMKLR1 compound.**

| SEQ ID No | | Sequences |
|---|---|---|
| 109 | *LFR1 WT*/*1* | QIVLTQSPAIMSASPGEKVTMTC |
| 110 | *LFR1* /*1* | AIQLTQSPSSLSASVGDRVTITC |
| 111 | *LFR1* /*2* | EIVLTQSPDFQSVTPKEKVTITC |
| 112 | *LFR1* /*3* | EIVLTQSPATLSLSPGERATLSC |
| 113 | *LFR2 WT* /*1* | WYQQKSGTSPKRWIY |
| 114 | *LFR2* /*1* | WYQQKPGKAPKRWIY |
| 115 | *LFR2* /*2* | WYQQKPDQSPKRWIY |
| 116 | *LFR2* /*3* | WYQQKPGQAPRRWIY |
| 117 | *LFR2* /*4* | WYQQKPGKAPKRLIY |
| 118 | *LFR3 WT* /*1* | GVPARFSGSGSGTFYSLTISSMEAEDAATYYC |
| 119 | *LFR3* /*1* | GVPSRFSGSGSGTDYTLTISSLQPEDFATYYC |
| 120 | *LFR3* /*2* | GVPSRFSGSGSGTDYTLTINSLEAEDAATYYC |
| 121 | *LFR3* /*3* | GIPARFSGSGSGTDYTLTISSLEPEDFAVYYC |
| 122 | *LFR3* /*4* | GVPSRFSGSGSGTDFTLTISSLQPEDFATYYC |
| 132 | *LFR3* /*5* | GVPSRFSGSGSGTDFTLTINSLEAEDAATYYC |
| 123 | *LFR4 WT* /*1* | FGPGTKLELK |
| 124 | *LFR4* /*1* | FGGGTKVEIK |
| 128 | *LFR1 consensus* /*1* | XIXLTQSPXXXSXXXXXXXTXTC |
| 129 | *LFR2 consensus* /*1* | WYQQKXXXXPXRWIY |
| 130 | *LFR3 consensus* /*1* | GXPXRFSGSGSGTXYXLTIXSXXXEDXAXYYC |
| 131 | *LFR4 consensus* /*1* | FGXGTKXEXKR |

**Table 5: Sequences of the heavy chain of an anti-CMKLR1 compound**

| SEQ ID No | | Sequences |
|---|---|---|
| 20 | 23_VHvA | EVQLVESGGGLVQPGGSLRLSCAASGFTFSSYGMSWVRQAPGKGLELVATINRYGGSTYYPDSV KGRFTISRDNSKNTLYLQMNSLRAEDTAVYYCPRLIYYGNEGDSWGQGTLVTVSS |
| 21 | 23_VHvB | EVQLVESGGGLVQPGGSLRLSCAASGFTFSSYGMSWVRQAPGKGLELVATINRYGGSTYYAAS VKGRFTISRDNSKNTLYLQMNSLRAEDTAVYYCPKLIYYGNEGDSWGQGTLVTVSS |
| 22 | 23_VHvC | EVQLVESGGGLVQPGGSLRLSCAASGFTFSSYAMSWVRQAPGKGLELVATINRYGGSTYYAAS VKGRFTISRDNSKNTLYLQMNSLRAEDTAVYYCPKLIYYGNEGDSWGQGTLVTVSS |
| 23 | 23_VHvD | EVQLVESGGGLVQPGGSLRLSCAASGFTFSSYAMSWVRQAPGKGLELVSTISRSGGSTYYAASV KGRFTISRDNSKNTLYLQMNSLRAEDTAVYYCPKLIYYGNEGDSWGQGTLVTVSS |
| 24 | 23_VHvE | EVQLVESGGGLVQPGGSLRLSCAASGFTFSSYAMSWVRQAPGKGLELVSTISGSGGSTYYAASV KGRFTISRDNSKNTLYLQMNSLRAEDTAVYYCAKLIYYGNEGDSWGQGTLVTVSS |
| 25 | 23_VHvF | EVQLVESGGGLVQPGGSLRLSCAASGFTFSSYAMSWVRQAPGKGLEWVSTISGSGGSTYYAAS VKGRFTISRDNSKNTLYLQMNSLRAEDTAVYYCAKLIYYGNEGDSWGQGTLVTVSS |
| 26 | 23_VHvG | EVQLVESGGGLVQPGGSLRLSCAASGFTFSSYAMSWVRQAPGKGLEWVSTISGSGGSTYYAAS VKGRFTISRDNSKNTLYLQMNSLRAEDTAVYYCAKLIYYGNEGESWGQGTLVTVSS |
| 27 | 23_VHvH | EVQLVESGGGLVQPGGSLRLSCAASGFTFSSYAMSWVRQAPGKGLEWVSTISGSGGSTYYAAS VKGRFTISRDNSKNTLYLQMNSLRAEDTAVYYCAKLIYYGNEGDTWGQGTLVTVSS |
| 28 | 46_VHvA | QVQLVQSGAEVKKPGASVKVSCKASGFTFSSYGMSWVRQAPGQGLELVATINRYGGSTYYPD SFKGRVTITRDNSTSTLYMELSSLRSEDTAVYYCPRLIYYGNEGDSWGQGTLVTVSS |
| 29 | 46_VHvB | QVQLVQSGAEVKKPGASVKVSCKASGFTFSSYGMSWVRQAPGQGLELVGTINRYGGSTYYAQ KFQGRVTITRDNSTSTLYMELSSLRSEDTAVYYCPRLIYYGNEGDSWGQGTLVTVSS |
| 30 | 46_VHvC | QVQLVQSGAEVKKPGASVKVSCKASGYTFTSYGMSWVRQAPGQGLELMGIINRNGGSTYYAQ KFQGRVTITRDTSTSTVYMELSSLRSEDTAVYYCPRLIYYGNEGDSWGQGTLVTVSS |
| 31 | 46_VHvD | QVQLVQSGAEVKKPGASVKVSCKASGYTFTSYGMSWVRQAPGQGLELMGIINPNGGSTYYAQ KFQGRVTITRDTSTSTVYMELSSLRSEDTAVYYCARLIYYGNEGDSWGQGTLVTVSS |
| 32 | 46_VHvE | QVQLVQSGAEVKKPGASVKVSCKASGYTFTSYGMSWVRQAPGQGLEWMGIINPNGGSTYYA QKFQGRVTITRDTSTSTVYMELSSLRSEDTAVYYCARLIYYGNEGDSWGQGTLVTVSS |
| 33 | 46_VHvF | QVQLVQSGAEVKKPGASVKVSCKASGYTFTSYYMHWVRQAPGQGLEWMGIINPNGGSTSYA QKFQGRVTMTRDTSTSTVYMELSSLRSEDTAVYYCARLIYYGNEGDSWGQGTLVTVSS |
| 34 | 46_VHvG | QVQLVQSGAEVKKPGASVKVSCKASGYTFTSYYMHWVRQAPGQGLEWMGIINPNGGSTSYA QKFQGRVTMTRDTSTSTVYMELSSLRSEDTAVYYCARLIYYGNEGESWGQGTLVTVSS |
| 35 | 46_VHvH | QVQLVQSGAEVKKPGASVKVSCKASGYTFTSYYMHWVRQAPGQGLEWMGIINPNGGSTSYA QKFQGRVTMTRDTSTSTVYMELSSLRSEDTAVYYCARLIYYGNEGDTWGQGTLVTVSS |
| 36 | 41_VHvA | QVQLVQSGSELKKPGASVKVSCKASGFTFSSYGMSWVRQAPGQGLELVATINRYGGSTYYPDS FKGRFVISRDNSVSTLYLQISSLKAEDTAVYYCPRLIYYGNEGDSWGQGTLVTVSS |
| 37 | 41_VHvB | QVQLVQSGSELKKPGASVKVSCKASGFTFSSYGMSWVRQAPGQGLELVGTINRYGGSPYYAQ GFTGRFVISRDNSVSTLYLQISSLKAEDTAVYYCPRLIYYGNEGDSWGQGTLVTVSS |
| 38 | 41_VHvC | QVQLVQSGSELKKPGASVKVSCKASGYTFTSYAMSWVRQAPGQGLELVGTINRYGGNPYYAQ GFTGRFVISRDNSVSTLYLQISSLKAEDTAVYYCPRLIYYGNEGDSWGQGTLVTVSS |
| 39 | 41_VHvD | QVQLVQSGSELKKPGASVKVSCKASGYTFTSYAMNWVRQAPGQGLELMGTINRYGGNPYYA QGFTGRFVISRDTSVSTAYLQISSLKAEDTAVYYCPRLIYYGNEGDSWGQGTLVTVSS |
| 40 | 41_VHvE | QVQLVQSGSELKKPGASVKVSCKASGYTFTSYAMNWVRQAPGQGLELMGTINTYTGNPYYAQ GFTGRFVISRDTSVSTAYLQISSLKAEDTAVYYCARLIYYGNEGDSWGQGTLVTVSS |
| 41 | 41_VHvF | QVQLVQSGSELKKPGASVKVSCKASGYTFTSYAMNWVRQAPGQGLEWMGWINTYTGNPTY AQGFTGRFVISLDTSVSTAYLQISSLKAEDTAVYYCARLIYYGNEGDSWGQGTLVTVSS |
| 42 | 41_VHvG | QVQLVQSGSELKKPGASVKVSCKASGYTFTSYAMNWVRQAPGQGLEWMGWINTYTGNPTY AQGFTGRFVISLDTSVSTAYLQISSLKAEDTAVYYCARLIYYGNEGETWGQGTLVTVSS |

**Table 6: Sequences of the light chain of an anti-CMKLR1 compound**

| SEQ ID No | | Sequences |
|---|---|---|
| 43 | 13VLvA | AIQLTQSPSSLSASVGDRVTITCSASSSVSFMHWYQQKPGKAPKRWIYDTTKLTSGVPSRFSGS GSGTDYTLTISSLQPEDFATYYCQQWNSKPPLTFGGGTKVEIK |
| 44 | 13VLvB | AIQLTQSPSSLSASVGDRVTITCRASQSVSFMHWYQQKPGKAPKRWIYDATKLTSGVPSRFSGS GSGTDYTLTISSLQPEDFATYYCQQWNSKPPLTFGGGTKVEIK |
| 45 | 13VLvC | AIQLTQSPSSLSASVGDRVTITCRASQSVSFLHWYQQKPGKAPKRWIYDATKLTSGVPSRFSGS GSGTDFTLTISSLQPEDFATYYCQQWNSKPPLTFGGGTKVEIK |
| 46 | 13VLvD | AIQLTQSPSSLSASVGDRVTITCRASQSVSFLHWYQQKPGKAPKRWIYDATKLTSGVPSRFSGS GSGTDFTLTISSLQPEDFATYYCQQWQSKPPLTFGGGTKVEIK |
| 47 | 13VLvE | AIQLTQSPSSLSASVGDRVTITCRASQSVSFLHWYQQKPGKAPKRWIYDASKLTSGVPSRFSGS GSGTDFTLTISSLQPEDFATYYCQQWQSKPPLTFGGGTKVEIK |
| 48 | 13VLvF | AIQLTQSPSSLSASVGDRVTITCRASQGISFLAWYQQKPGKAPKRLIYDASKLESGVPSRFSGSGS GTDFTLTISSLQPEDFATYYCQQWQSKPPLTFGGGTKVEIK |
| 49 | 13VLvG | AIQLTQSPSSLSASVGDRVTITCRASQGISSLAWYQQKPGKAPKRLIYDASSLESGVPSRFSGSGS GTDFTLTISSLQPEDFATYYCQQFQSYPPLTFGGGTKVEIK |
| 50 | 21_VLvA | EIVLTQSPDFQSVTPKEKVTITCSASSSVSFMHWYQQKPDQSPKRWIYDTTKLTSGVPSRFSGS GSGTDYTLTINSLEAEDAATYYCQQWNSKPPLTFGGGTKVEIK |
| 51 | 21_VLvB | EIVLTQSPDFQSVTPKEKVTITCRASQSVSFMHWYQQKPDQSPKRWIYDATKSTSGVPSRFSGS GSGTDYTLTINSLEAEDAATYYCQQWNSKPPLTFGGGTKVEIK |
| 52 | 21_VLvC | EIVLTQSPDFQSVTPKEKVTITCRASQSVSFLHWYQQKPDQSPKRWIYDATKSTSGVPSRFSGS GSGTDFTLTINSLEAEDAATYYCQQWNSKPPLTFGGGTKVEIK |
| 53 | 21_VLvD | EIVLTQSPDFQSVTPKEKVTITCRASQSVSFLHWYQQKPDQSPKRWIYDATKSTSGVPSRFSGS GSGTDFTLTINSLEAEDAATYYCQQWQSKPPLTFGGGTKVEIK |
| 54 | 21_VLvE | [?] EIVLTQSPDFQSVTPKEKVTITCRASQSISFLHWYQQKPDQSPKRLIYDASKSTSGVPSRFSGSGS GTDFTLTINSLEAEDAATYYCQQWQSKPPLTFGGGTKVEIK |
| 55 | 21_VLvF | EIVLTQSPDFQSVTPKEKVTITCRASQSISSLHWYQQKPDQSPKLLIYYASQSFSGVPSRFSGSGS GTDFTLTINSLEAEDAATYYCHQSQSLPPLTFGGGTKVEIK |
| 56 | 11_VLvA | EIVLTQSPATLSLSPGERATLSCSASSSVSFMHWYQQKPGQAPRRWIYDTTKLTSGIPARFSGSG SGTDYTLTISSLEPEDFAVYYCQQWNSKPPLTFGGGTKVEIK |
| 57 | 11_VLvB | EIVLTQSPATLSLSPGERATLSCRASQSVSFMHWYQQKPGQAPRRWIYDATKLTTGIPARFSGS GSGTDYTLTISSLEPEDFAVYYCQQWNSKPPLTFGGGTKVEIK |
| 58 | 11_VLvC | EIVLTQSPATLSLSPGERATLSCRASQSVSFLHWYQQKPGQAPRRWIYDATKRTTGIPARFSGSG SGTDFTLTISSLEPEDFAVYYCQQWNSKPPLTFGGGTKVEIK |
| 59 | 11_VLvD | [?] EIVLTQSPATLSLSPGERATLSCRASQSVSFLHWYQQKPGQAPRRWIYDATKRTTGIPARFSGSG SGTDFTLTISSLEPEDFAVYYCQQWQSKPPLTFGGGTKVEIK |
| 60 | 11_VLvE | EIVLTQSPATLSLSPGERATLSCRASQSVSFLAWYQQKPGQAPRRLIYDASKRTTGIPARFSGSGS GTDFTLTISSLEPEDFAVYYCQQWQSKPPLTFGGGTKVEIK |
| 61 | 11_VLvF | EIVLTQSPATLSLSPGERATLSCRASQSVSYLAWYQQKPGQAPRLLIYDASNRATGIPARFSGSGS GTDFTLTISSLEPEDFAVYYCHQRQNWPPLTFGGGTKVEIK |

### Examples of therapeutic efficacy of anti-CMKLR1 antibody treatment on preclinical models of autoimmune and inflammatory diseases

### EXAMPLE 1. Induction of colitis by DSS

Colitis was induced in 8-10 weeks-old-C57Bl/6 male mice by adding 2% (wt/vol) of DSS to the sterile drinking ad libitum water for 6 days. Treatments were injected intra-peritoneally: Isotype control hIgG1 (10µg per mouse), RvE1 (1µg per mouse) daily, or 2G1 antibody (10µg per mouse) three times for 5 days. Colitis follow-up consisting of body weight and stool score (0: normal stool; 4: blood in stool) parameters were performed daily. When mice were euthanized, colon length representing the pathology severity was measured. Resolution index was determined in the different conditions as described in Bannenberg et al., 2005.

RESULTS: The DSS animal model presented on **Figure 2** is an acute inflammatory model. Figure 1 shows a better overall state of the animals treated with the anti-CMKLR1 antibody than states of animals receiving the control antibody or the resolvin RvE1. The anti-CMKLR1 treated mice lost significantly less weight (**Fig. 2A**) and the score stool (**Fig. 2B**) was significantly better. Regarding the colon length and resolution index (**Figure 2C and 2D**), the animals receiving the anti-CMKLR1 or RvE1 presented similar results.

### EXAMPLE 2. Induction of colitis by TNBS

Colitis was induced in 8-10 weeks-old-C57BI/6 male mice by intrarectal injection of 200 µL of the haptenating agent TNBS at 5% in 50% ethanol on day 0. Treatments were injected intra-peritoneally; RvE1 (1µg per mouse) daily for three days, or 2G1 antibody (10µg per mouse) twice for 3 days. Colitis follow-up consisting of body weight and stool score (0: normal stool; 4: blood in stool) parameters were performed daily (data not represented). When mice are euthanized, colon length representing the pathology severity was measured.

RESULTS: Colitis induced by TNBS is another model of acute inflammation. **Figure 3** shows that animals treated with anti-CMKLR1 or RvE1 have a colon length which is the same as normal animal (wt). However, those treated with the isotype control presented a shorten colon length. These results confirmed the therapeutic potential of an anti-CMKLR1 antibody acting like RvE1 on acute inflammatory mice models.

### EXAMPLE 3. IL10-KO model - Spontaneous colitis model

IL-10KO mice develop a spontaneous colitis from 20 weeks of age mostly due to the absence of regulatory T cells function through IL-10 secretion in the intestine. IL-10KO mice were followed-up three times a week from 18-week old for their weight loss and stool consistency which are clinical hallmarks of this pathology. Anti-CMKLR1 antibody (2G1) or isotype control (hlgG1) were injected intra-peritoneally when the weight loss was superior to 5% and the stool score was superior or equal to 1 for 2 weeks (25µg/injection, 3 times a week).

RESULTS: A chronic inflammatory model was used to study the efficacy of anti-CMKLR1 antibody treatment. **Figure 4** shows the analysis of the percentage of weight loss (**Fig. 4A**) and the score stool (**Fig. 4B**) when animals were treated with isotype control or anti-CMKLR1 antibody. Results show that animals lost less weight when treated with the anti-CMKLR1 antibody and had a better score stool than those receiving the isotype control. Anti-CMKLR1 antibody seems hence to present a therapeutic potential on chronic inflammatory diseases.

### EXAMPLE 4. Preclinical model of Type 1 diabetes: the mice NOD model

8 weeks-old NOD female mice were obtained from Charles River laboratory. These mice develop a spontaneous type 1 diabetes at age between 12 to 20 weeks. The diabetes initiation can be measured by the high glycaemia. When the glycaemia was between 180 and 234 mg/dL, anti-CMKLR1 and isotype control were given intra-peritoneally at 20µg/injection three times a week for 2 weeks. Mice were euthanized when the glycaemia was superior to 600mg/dL corresponding to an irreversible diabetes.

RESULTS: this type 1 diabetes model is considered as a mice autoimmune disease model as well. Results presented **Figure 5A-C** show that animals treated with an anti-CMKLR1 antibody presented a better survival percentage and an almost normal glycaemia as indicated by measurement of the blood glucose concentration. This recovery seems to be stable over time and indicate a potential total recovery of animals that were previously ill. Anti-CMKLR1 antibody restored a tolerated state against glucose.

### EXAMPLE 5. Imiquimod-induced psoriasis-like skin inflammation

Aldara^{®} cream which is known to induce psoriasis in mice was used on male C57Bl/6 mice (8-10-week old). Mice received a daily topical dose of Aldara on the shaved back and the left ear for 6 consecutive days. Treatments were injected intra-peritoneally: RvE1 (1µg per mouse) and PBS daily. Ear (**Fig. 6A**) and back (**Fig. 6B**) thicknesses were measured daily until the recovery.

RESULTS: Animals treated with the resolvin RvE1 had a lower thickness of the ears and skin than animals treated with a control molecule. These results suggest a potential application for agonist anti-CMKLR1 antibody therapy on psoriasis mice model, illustrative of an autoimmune disease.

### EXAMPLE 6. Therapeutic efficacy of anti-CMKLR1 antibody on preclinical model of sepsis: mice peritonitis model

Common peritonitis is induced by intraperitoneal injection of Zymosan A^{®} (1mg per mouse in 1mL). Preventive injection of anti-CMKLR1 was performed 5 minutes before Zymosan A injection: RvE1 (1µg per mouse), 2G1 antibody (10µg per mouse). Murine peritoneal polymorphonuclear neutrophil (PMN) and macrophages were collected at 2-4-8-16-24 and 48h after Zymosan A injection and numbered by flow cytometry analysis to determinate the resolution index (Bannenberg et al., 2005).

RESULTS: Results presented on **Figure 7** on PMNs (**7A**) and Macrophages (**7B**) numbers and on resolution index (**7C**) show that animals treated with RvE1 or an anti-CMKLR1 antibody presented identical results and a slightly less PMNs and macrophages cells along with a better resolution index compared to the isotype control. In sepsis, even a slight difference could be of importance for therapy. Hence, these results are very positive for a potential application of an anti-CMKLR1 antibody in sepsis.

### EXAMPLE 7. Therapeutic efficacy of anti-CMKLR1 antibody treatment on preclinical models of cancers:

### Example 7.1. Effect of an anti-CMKLR1 antibody on the growth of a primary tumor and its lung metastasis development in an orthotopic mammary carcinoma model.

Mice were anesthetized with 3% of isoflurane. Mice were shaved on the abdomen and 4T1 cells (0,25 millions) were injected in the mammary gland with an insulinic syringe (30 Gauges) in 50µL of PBS. The anti-CMKLR1 antibody (2G1) or an anti-41BB antibody (3H3) or both antibodies were injected twice at day 4 and day 7 (10µg/injection); a control antibody was injected three times a week for three weeks intraperitoneally in PBS (100µg/injection). In a second study to measure the lung metastasis following mammary carcinoma development, animals were treated with anti-CMKLR1 antibody at 0.8mg/kg or control antibody (100µg/injection) three times a week for three weeks.

RESULTS: As shown in **Figure 8A****.,** animals treated by a single compound (2G1 or 3H3) did not show any improvement in the tumor growth compared to the animals receiving isotype control antibody. However, the animals treated with the combination of anti-CMKLR1 antibody and anti-41BB antibody show significant (p<0.01) reduction in the growth of the tumor in the mammary carcinoma model. Since this model of mammary carcinoma is a pretty aggressive model, results are considered positive. As shown on **Figure 8B****,** which illustrates the effects of an anti-CMKLR1 compound on the lung metastasis by bioluminescence imaging, it can be seen than the anti-CMKLR1 treatment reduces the lung metastasis as compared to animals treated with the control antibody. The analysis of the lymph node metastasis shows that no animals treated with the anti-CMKLR1 compound has metastasis while two animals in the control have metastasis (data not shown). Those results indicate that anti-CMLKR1 antibody with agonist activity mimicking RvE1 has an anti-metastatic effect. In this model, the antibody of the invention does not present any significant efficacy on primary tumor development. However, results show an improvement when animals are treated by a combination of anti-CMKLR1 and anti-41BB antibodies.

### Example 7.2. Therapeutic effect on tumor growth in a colon carcinoma model

8-week old C57bl/6J male mice were anesthetized with 3% of isoflurane. Mice were shaved on the flank and MC38 cells (0,5.10^6 cells/mouse) cell lines were injected subcutaneously with an insulinic syringe (30 Gauges) in 50µL of PBS. Another model was used, in which 8-week old Balb/c male mice were anesthetized with 3% of isoflurane. Mice were shaved on the flank and CT26 cells (1.10^6 cells/mouse) were injected subcutaneously with an insulinic syringe (30 Gauges) in 50µL of PBS.

The agonistic anti-CMKLR1 antibody (2G1) or an anti-SIRPa antibody (p84- anti-mouse SIRPa from Merck Millipore) (SIRPa is a new checkpoint inhibitor) were injected once a week (20µg/injection) intraperitoneally for 3 weeks starting at d4 after tumor inoculation alone or combined.

RESULTS: As shown in **Figure 9A-C,** in the CT26 carcinoma model, the anti-CMKLR1 antibody on its own (**Fig. 9B**) did not demonstrate a clinical effect on the tumor development compared to the control group nor did the anti-SIRPa alone (**Fig. 9A**). However, and surprisingly, the combination of both compounds enabled an inhibition of the tumor growth in a timely manner (**Fig. 9C**). In another mice colon carcinoma model presented on **Figure 9D****,** the anti-CMKLR1 did show an efficacy in the inhibition of the tumor growth compared to the isotype control. All together, these results for two different colon carcinoma models indicate that an agonist anti-CMKLR1 antibody can prevent tumor development on its own, or in combination with another therapeutic agent.

### EXAMPLE 8. Meta-analysis of the CMKLR1 expression on UC or CD human patient biopsies treated with an anti-TNFa or anti-α4β7 antibody therapies.

Signaling networks perpetuating chronic gastrointestinal inflammation in Crohn's disease (CD) and ulcerative colitis (UC), the two main forms of inflammatory bowel diseases (IBD), remain unclear in human. According to an analysis of nearly 500 patients with IBD and 100 controls, inventors report here that CMKLR1 transcript are accumulated in inflamed colon tissues of severe IBD patients who were not responding to immunosuppressive/corticosteroids and immunotherapies such as anti-TNFα (infliximab) or anti-α4β7 integrin (vedolizumab) therapies.

The inventors first analyzed the mucosal CMKLR1 transcript expression by performing a meta-analysis of publicly available transcriptional datasets of three cohorts of UC patients (GSE16879(Arijs et al., 2009a) and GSE12251(Arijs et al., 2009b), and GSE73661 with colon mucosa biopsies performed before anti-TNF treatment (within a week) in patients refractory to corticosteroids and/or immunosuppression. In these three cohorts, anti-TNF response was defined as histological healing analyzed 4-6 weeks after their first anti-TNF infusion (altogether: n=18 non-IBD controls, n=41 UC non-responders and n=28 UC responders).

RESULTS: The analysis showed that CMKLR1 transcript expression is significantly increased in colon biopsies of primary UC non-responder patients before and after treatment with anti-TNF therapy as compared to non-IBD controls or patients with UC before anti-TNF and who will respond to anti-TNF therapy (**Figure 10A**). Mucosal CMKLR1 expression is also significantly increased in colon or ileal biopsies of Crohn's Disease patients (n=24 non-IBD controls, n=17 CD non-responders and n=20 CD responders; GSE16879(Arijs et al., 2009a)) before and after anti-TNF therapy in patients who will not respond to anti-TNF as compared to non-IBD controls or future responders (**Figure 10B**). Finally, colon mucosa gene expression analysis in a cohort of UC patients (GSE7366146) treated with anti-α4β7 (vedolizumab) therapy confirmed also that CMKLR1 expression is also significantly increased in non-responders before and after treatment with Vedolizumab (**Figure 11**).

Altogether, the meta-analysis shows that CMKLR1 is over-expressed in inflamed tissues of IBD patients, in particular in patients non-responding to current immunosuppressive or immunotherapies even before initiation of the treatment. Our meta-analysis provide evidence that the CMKLR1 expression in the colon, or rather ileon for CD, from UC or CD patients who are treatment refractory may in contrast qualify these patients as being responsive to an agonist anti-CMKLR1 antibody treatment such as an antibody of the invention.

### EXAMPLE 9. CMKLR1 expression and antibody binding study

### - ELISA binding CMKLR1 (Figure 12)

CMKLR1 peptide (273NH2-PYHTLNLLELHHTAMPGSVFSLGLPLATALAIA-COOH305) (SEQ ID No: 18) (5µg/ml) was coated in borate buffer overnight. Saturation was performed with PBS-Tween 0,1%-Gelatin 0,25% for 2 hours at 37°C. Then, 2G1 or hIgG1 antibodies were added at different concentrations for 2 hours at 37°C. Then peroxidase-conjugated secondary antibody (0,8µg/ml) was added for 1 hour at 37°C and revealed by a TMB substrate. The colorimetric reaction was read with TECAN.

### - CMKLR1 expression by FACS (Figure 13A)

Cells were resuspended in PBS-FBS-EDTA and incubated with Fc block (1/50) for 30 minutes on ice. Staining on monocytes, macrophages and dendritic cells were performed using A488-labeled 2G1 (5µg) or A488-labeled hIgG1 (5µg).

### - Western blot analysis CMKLR1 (Figure 13B)

After protein migration and transfer as described previously, 2G1 antibody (10µg/membrane) was incubated overnight at 4°C and revealed with a peroxidase-conjugated secondary antibody (1:2000). The CMKLR1 expression was then detected by using Chemiluminescence and Image Reader. Western blot Images were quantified by Multi Gauge software.

RESULTS: the results illustrated on **Figure 12** confirm that the anti-CMKLR1 antibody clone 2G1 is able to bind the polypeptide forming the loop EL3 of CMKLR1. Expression of CMKLR1 on different cell lines assessed using 2G1 antibody by FACS and Western blot showed that human Tumor T cell lines Trp1 and U937 express CMKLR1 as well as transduced CMKLR1 CHO cells and human lung fibroblast cell line MRC5 and human NK cell line NKL (**Fig. 13**).

### EXAMPLE 10. Study of the CMKLR1 expression on myeloid lineage

### Example 10.1. Human monocytes differentiation and polarization

Monocytes were collected from PBMC of buffy coat of healthy volunteers and isolated by magnetic separation or by elutriation. Then, monocytes were cultured with different cocktails of cytokines to generate differentiated unpolarized macrophages or polarized macrophages. This protocol allowed to generate polarized differentiated macrophages in order to have pro-(M1) or anti-(M2) inflammatory macrophages in different wells. Monocytes were plated at 0,5.10⁶cells/mL in complete RPMI (RPMI with 10% FBS, 1% glutamine, 1% antibiotics) and 500µL of the cell suspension was plated per well in a 24-well plate. 100ng/mL of M-CSF was added with medium for differentiation of the cells. Cells were incubated 5 days and medium was replaced with fresh medium complemented with 100ng/mL of M-CSF at day 3. For the polarization phases, solution of LPS-IFNg at 100ng/mL of LPS and 20ng/mL of IFNg complemented with isotype controls (mIgG1 or higG4) (2µg/ml) or anti-CMKLR1 antibodies (2µg/ml) (2G1 or 2G4, H6, BZ332 or 84939) or with C15 peptide (10nM) or RvE1 (10ng/ml) during 3 days to generate M1 macrophages. M1-IFNg macrophages could also be generated by adding only IFNg (20ng/mL) in culture medium. For M2 polarization cells were incubated with IL-4 at 20ng/mL. Following differentiation and/or polarization, phenotype and functional cytokines/chemokines release was studied by FACS analysis, ELISA and Western blot.

### Example 10.2. Mice macrophages and DCs isolation and differentiation

### -Isolation of murine bone marrow derived macrophages

Bone marrow cells were harvested and cultured in the RPMI medium supplemented with 10% FBS, glutamine and antibiotics containing macrophages colony-stimulating factor (M-CSF) at 100ng/mL for 5 days inducing the macrophages differentiation. The macrophages were harvested and cultured for 2 days with either IFNg (20ng/ml) and LPS (100ng/ml) inducing the M1 polarization or with IL-4 (20ng/ml) inducing the M2 polarization. Treatments were added during macrophage polarization at 2µg/ml.

### -Bone marrow derived dendritic cells generation

Bone marrow cells were harvested and cultured in RPMI medium supplemented with 10% FBS, glutamine and antibiotics and the dendritic cell differentiation was induced by GM-CSF at 20ng/ml for 7 days. Then, immature dendritic cells (iDC) were collected and cultured for 24 hours with LPS (100ng/ml) to induce the maturation from iDC to mDC. Treatments were added during differentiation and maturation at 2µg/ml.

After differentiation of mice M1 or M2 as explain above, cells were incubated in presence of medium, isotype control, anti-CMKLR1 antibodies: clones H6 and BZ194, C15 peptides, 2G1 the anti-CMKLR1 antibody of interest or RvE1 were used. Then the secretion of IL10, CCL17 and IL12p40 was assessed by ELISA. Cytokines secretion was measured in the supernatant using an ELISA kit from BD. Supernatants were diluted at 1/10 for IL10 cytokines, 1/50 for CCL17 cytokines and 1/100 for IL12p40 cytokines.

### - ELISA cytokines secretion study

Cytokines secretion was detected by ELISA according to the BD manufacturer instructions. Briefly, supernatants were diluted in the appropriate buffer and incubated for 2 hours after overnight coating with capture antibody and saturation. Then, cytokines were revealed with a detection biotin-coupled antibody and the signal was amplified with the biotin-streptavidin-coupled peroxidase system. TMB supplied by BD Bioscience was used as substrate and colorimetric reaction was read with TECAN.

### - Activation cell markers analyzed by FACS

Dendritic cells were resuspended in PBS-FBS-EDTA and incubated with live and dead (LIVE/DEAD^{®} Fixable Dead Cell Stains Yellow-Life Technologies) for 30 minutes on ice. Staining of CD11c-BV711, CD11b-APCCy7, I/Ab-APC, CD103-PerCPCy5.5, CCR7-V450, CD40-PeCy7, CD80-PE, CD86-FITC (all provided by BD Pharmingen) were performed.

### - Western blot analysis ERK/Akt

Mice pro-inflammatory macrophages (M1) were generated from bone marrow with M-CSF and polarized with IFN-gamma (IFNg) and LPS. Briefly, bone marrow cells were collected by flushing the femoral bone and cultured with 100ng/mL of mM-CSF for 5 days and then polarized with 20ng/mL of IFNg and 100ng/mL of LPS for 24h. Then, they were deprived of FBS for 24 hours with RPMI FBS 2% medium. Finally, mice M1 were treated with 2µg/mL of 2G1 antibody at different times: 5, 10 and 30 minutes. Cells were collected in a RIPA buffer. The protein concentration was measured by a BCA protein kit assay. Proteins were denatured by heating at 95°C for 5 minutes and diluted in DTT and Laemmli solution. After migration and transfer, the nitrocellulose membrane was blocked with 5% BSA in TBS-T for 2 hours. Anti-phospho-ERK antibody and anti-phospho-Akt antibody (1:1000) were incubated with the membrane overnight at 4°C and revealed with a peroxidase-conjugated secondary antibody (1:2000). Western blot Images were quantified by Multi Gauge software

### Example 10.3: CMLKR1 expression after inflammatory stimuli on Human blood monocytes and mouse bone marrow myeloid and neutrophils cells.

Human monocytes were collected from PBMC of buffy coat of healthy volunteers and isolated by magnetic separation or by elutriation. Then the monocytes (CD14 positive cells) were cultured in medium and treated with different pro-inflammatory stimuli 16 hours or 48 hours: LPS (100 ng/ml) or TNFa (10.000 U/ml) or IL6 (20 ng/ml).

Mouse monocytes (CD11b+ Ly6G- SSClow) and neutrophils (CD11b+ Ly6G- SSClow) were obtained from bone marrow cells harvested and cultured in RPMI medium supplemented with 10% FBS, glutamine and antibiotics. Cells were then cultured in medium and treated with different pro-inflammatory stimuli 16 hours or 48 hours: LPS (100 ng/ml) or TNFa (10.000 U/ml) or IL6 (20 ng/ml).

The expression of CMLKR1 was measured by FACS using commercial anti-CMKLR1 antibodies (Human anti-ChemR23: clone 84939 and Mouse anti-ChemR23 : clone 477806).

RESULTS: Analysis of the expression of CMKLR1 in mice myeloid lineage illustrated on **Figure 14** revealed a good expression of the protein on monocytes and macrophages from type 1 and 2 and as well as on dendritic cells. On **Figure 19****,** the expression of CMKLR1 on human monocyte and a mice bone marrow myeloid and neutrophil cells is illustrated. This expression is clearly increased by inflammatory stimuli such as LPS, TNFa or IL6 (at least twice as compared to the control after 48h) confirming that CMKLR1 expression on myeloid cell lineage and overexpression during inflammation could represent a therapeutic approach to downregulate and/or induce resolution of inflammation. The study of the inflammatory cytokines secreted by mice M1 or M2 macrophages illustrated on **Figure 15** revealed that only the 2G1 clone, an anti-CMKLR1 antibody, induced anti-inflammatory cytokines IL10 and CCL17 by M2 type macrophages (**Figure 15A** and **15B**) and decreased the pro-inflammatory cytokines from M1 type macrophages (**Figure 15C**). The result was different for cells treated with RvE1 or with C15 peptide from C-terminal part of the Chemerin (Cash *et al.,* 2008) or even with other commercial anti-CMKLR1 antibodies, indicating a very interesting characteristic of the 2G1 antibody as an anti-inflammatory compound. Then inventors studied the cytokines expression in human myeloid cells obtained from donors and incubated with the different compounds. Results presented on **Figure 16** show that 2G4 induces the M2 polarization of the cells compared to the control and the commercial CMKLR1 antibody C7, confirming the results obtained in mice cells. Only the 2G1 antibody was able to induce anti-inflammatory cytokines IL10 and CCL17 by M2 type macrophages (**Figure 16A** and **16B**) and was able to decrease the secretion of the IL12p40 cytokine by M1 type macrophages (**Figure 16C**). Finally, the DC activation markers were analyzed by FACS and results illustrated on **Figure 17** shows a strong decrease of the expression of CD80, CD86, CD103, CD40 and IAb when cells were treated with RvE1 lipid or with 2G1 antibody compared to the excipient or isotype control. These results indicate that 2G1 antibody is as active on CMKLR1 pathway on DCs as RVE1. Then inventors analyzed the CMKLR1 activation pathway on mice macrophages by Western blot. **Figure 18** shows that anti-CMKLR1 antibody 2G1 was able to induce the activation of both Akt and Erk proteins after 10 to 30 minutes of incubation. These results indicate that the 2G1 antibody is able to exhibit an agonist property against CMKLR1 receptor as does RvE1 lipid.**EXAMPLE 11.** Competition study on Chemerin-induced CMKLR1 activation with anti-CMKLR1 antibody

### Methods.

### Competition assay to measure Chemerin-dependent B-arrestin recruitment by CMKLR1 receptor in presence of anti-CMKLR1 antibody:

The day before the assay, CHO-K1 CMKLR1 cells (Discover'X ref 93-0313E2) were plated in pre-warmed cell reagent then plated in a 96-well plate at 100 µl/well of cells as (Discover'X ref 15-103) and incubated 48 hours at 37°C, in a 5% CO2 humidified incubator. The anti-CMLKR1 antibody was diluted (22X in 7-point series of 3-fold dilutions from 1µM to 1nM) and cells were incubated 30 min at 37°C with the antibody. Then cells were stimulated with Chemerin (2 or 6 nM) according to provider protocol (Discover'x ref 92-1036) 90 min at 37°C. The luminescence was measured after Working Detection Solution addition to the cells with a plate reader with 0,5 s integration.

### Measurement of the competition of the anti-CMKLR1 antibody with Chemerin in the production of AMPc by CMKLR1 receptor:

The day before the experiment CHO-K1 CMKLR1 Gi cells (Discover'X ref 95-0080C2) were plated in pre-warmed cell reagent then plated in a 96-well plate at 100 µl/well of cells as (Discover'X ref 15-103) and incubated 24 hours at 37°C, in a 5% CO2 humidified incubator for 24 hours.

A mix of Chemerin agonist (6x in 7-point series of 3-fold dilutions from 10⁻⁷µM to 10⁻¹⁰M ) (Discover'x ref 92-1036 or 2324-CM-025 from R&D Systems) and forskolin (40µM) (a cAMP activator) (Discover'x ref 92-0005) was added to the cells during 30 min at 37°C; or cells were pre-incubated 30 min at 37°C with anti-CMKLR1 antibody (serial dilution : 6X in 7-point series of 3-fold dilutions from 1µM to 1nM). Then a mix of chemerin (2nM) + forskolin (60nM) was added to the cells 30 min at 37°C. For the detection cAMP, antibody reagent and cAMP working detection solution was added to the plate for 1 hour at room temperature, then cAMP solution A was added, and cells incubated 3 hours at room temperature in the dark. Bioluminescence was read with a plate reader with 0,5s integration.

**Results:** In order to test if the antibody of the invention is an antagonist of the Chemerin-induced CMKLR1 activation, two assays were performed, and the results are presented on **Figure 20****.** Chemerin-induced inhibition of forskolin-dependent cAMP production is shown on Figure 20A (**black circles or white squares**); anti-CMLKR1 antibody of the invention could not revert this inhibition of production (**black circles or white squares**) as compared to the control (grey diamonds). Chemerin-induced activation of the beta-arrestin presented Figure 20B, show that the anti-CMKLR1 antibody of the invention did not significantly modify chemerin-dependent activation of the beta-arrestin (white circles as compared to black diamonds). The antibodies of the invention do not have an antagonist activityof the CMLKR1-Chemerin interaction. Furthermore, the antibodies of the invention are not able to induce chemerin-induced CMKLR1 signaling pathway, confirming that these antibodies are not agonist of chemerin of the CMLKR1 pathway.

### EXAMPLE 12. CD45Rb^{high} T-cell transfer chronic colitis mouse model

**Method:** CD45Rb^{high} CD4 T cells were isolated from the spleen of naive mice and sorted on an ARIA FACS after a negative selection of the CD4 T cells by magnetic sorting, then injected intraperitoneally at 0,5.10⁶ cells in 100µL of PBS into 6-weeks old female Rag1 knock-out mice. Anti-CMKLR1 antibody (2G1) or an isotype control were administered from day 32 after the CD45Rb^{high} CD4 T cell transfer for 3 weeks three times a week at 1mg/kg. The follow-up of weight was evaluated three times a week and the weight variation was determined over the initial weight. * p < 0.05, ** p < 0.01.

**Results:** **Figure 21** presents the percentage of weight variation over the time post antibody administration, of animals treated with anti-CMLKR1 antibody or an isotype control. Both groups presented the same initial weight evolution over the first 30 days after treatment and started to differentiate at day 35. Mice treated with the anti-CMKLR1 continue to gain weight while, in contrast, control mice start to lose weight indicating development of chronic colitis as anticipated in this control group. The inventors confirm in a third model of colitis, here in a chronic model of inflammation, that anti-CMKLR1 antibodies of the invention could be of interest to treat chronic inflammatory and autoimmune diseases such as colitis.

### Example 13. Anti-tumor effect on the overall survival of mice hepatocarcinoma tumor model

**Method:** Mice were anesthetized with a cocktail of xylazine/ketamine. After a laparotomy, tumoral Hepa 1.6 cells were injected in PBS through the portal vein (2,5.10⁶ cells/100 µL) in PBS. The treatment was started 4 days after tumor injection. The anti-CMKLR1 antibody (2G1clone) and the hIgG1 istotype control were injected at 0.8mg/kg three times per week during 2 weeks. The anti-PD1 monoclonal antibody was injected twice a week during 2 weeks intraperitoneally in PBS (8mg/kg). Combination anti-CMKLR1 and anti-PD1 antibodies was tested as well (0.8 mg/kg and 8mg/kg respectively). The Overall survival was followed during sixty days and the percentage of survival in each condition was reported Figure 22.

**Results:** As shown in **Figure 22****,** animals treated with the anti-CMKLR1 or the anti-PD1 antibodies had seen their survival rate prolonged only for 1 animal on 7 treated (15% of treated animals) indicating a partial response (PR). However, animals treated with a combination of anti-PD1 and anti-CMKLR1 antibodies allows a significant increase of the survival rate (from 15% to 45%) with animals alive 60 days after treatment, indicating a complete response (CR). This result indicates an unexpected efficiency of the therapeutic combination (anti-PD1/antiCMKLR1 antibodies) on HCC tumor model.

### Embodiments

**1.** An anti-CMKLR1 compound selected from the group of an antibody or an antigen-binding fragment thereof or a chimeric, humanized or modified antibody which specifically binds to CMKLR1, said compound comprising:
   - an antibody heavy chain variable domain comprising the three CDRs VHCDR1, VHCDR2 and VHCDR3, wherein:
      - VHCDR1 comprises or consists of the amino acid sequences set forth in SEQ ID No: 4; SEQ ID No: 62; SEQ ID No: 63; SEQ ID No: 64 or SEQ ID No: 65; and
      - VHCDR2 comprises or consists of the amino acid sequences set forth in SEQ ID No: 6; SEQ ID No: 66; SEQ ID No: 67; SEQ ID No: 68; SEQ ID No: 69; SEQ ID No: 70; SEQ ID No: 71 or SEQ ID No: 72; and
      - VHCDR3 comprises or consists of the amino acid sequences set forth in SEQ ID No: 8; SEQ ID No: 73; SEQ ID No: 74 or SEQ ID No: 75; and
   - an antibody light chain variable domain comprising the three CDRs VLCDR1, VLCDR2 and VLCDR3, wherein:
      - VLCDR1 comprises or consists of the amino acid sequences set forth in; SEQ ID No: 12; SEQ ID No: 76; SEQ ID No: 77; SEQ ID No: 78; SEQ ID No: 79 or SEQ ID No: 80; and
      - VLCDR2 comprises or consists of the amino acid sequences set forth in SEQ ID No: 14; SEQ ID No: 81; SEQ ID No: 82; SEQ ID No: 83; SEQ ID No: 84; SEQ ID No: 85; SEQ ID No: 86; SEQ ID No: 87 or SEQ ID No: 88; and
      - VLCDR3 comprises or consists of the amino acid sequences set forth in SEQ ID No: 15 or SEQ ID No: 89.
**2.** The anti-CMKLR1 compound according to embodiment 1, which is a Resolvin E1-like agonist of CMKLR1, in particular wherein the anti-CMKLR1 compound is a pro-resolution factor, in particular on myeloid cell lineages, in particular wherein the anti-CMKLR1 compound is an anti-human CMKLR1 compound.
**3.** The anti-CMKLR1 compound according to embodiment 1 or 2, which binds specifically to an epitope localized within the third extra-cellular loop (EL3) of CMKLR1, in particular wherein the compound binds specifically to a polypeptide comprising amino acid residues of sequence SEQ ID No: 2.
**4.** The anti-CMKLR1 compound according to any one of embodiments 1 to 3, wherein the VHCDR3 comprises or consists of the amino acid sequences set forth in SEQ ID No: 144 or SEQ ID No: 148.
**5.** The anti-CMKLR1 compound according to any one of embodiments 1 to 4 wherein:
   - VHCDR1 comprises or consists of the amino acid sequences set forth in SEQ ID No: 4; SEQ ID No: 62; or SEQ ID No: 63; and
   - VHCDR2 comprises or consists of the amino acid sequences set forth in SEQ ID No: 67; SEQ ID No: 70 or SEQ ID No: 72; and
   - VHCDR3 comprises or consists of the amino acid sequence set forth in SEQ ID No: 144 or SEQ ID No: 148.
**6.** The anti-CMKLR1 compound according to any one of embodiments 1 to 5, wherein:
   - VLCDR1 comprises or consists of the amino acid sequence set forth in SEQ ID No: 77; and
   - VLCDR2 comprises or consists of the amino acid sequences set forth in SEQ ID No: 14; SEQ ID No: 81 or SEQ ID No: 84; and
   - VLCDR3 comprises or consists of the amino acid sequences set forth in SEQ ID No: 15 or SEQ ID No: 89.
**7.** The anti-CMKLR1 compound according to any one of embodiments 1 to 6, wherein the heavy chain variable domain comprises or consists of the amino acid sequences set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41 or SEQ ID No: 42; and wherein the light chain variable domain comprises or consists of the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.
**8.** The anti-CMKLR1 compound according to any one of embodiments 1 to 7, which enhances *in vitro* and/or *in vivo* the secretion of anti-inflammatory cytokines, in particular IL10 and/or CCL17; more particularly IL10; and/or which inhibits or reduces *in vitro* and/or *in vivo* the secretion of pro-inflammatory cytokines, in particular IL12; in particular by myeloid cells expressing CMKLR1.
**9.** The anti-CMKLR1 compound according to any one of embodiments 1 to 8, which enhances the macrophage polarization to favor anti-inflammatory M2 type macrophages.
**10.** The anti-CMKLR1 compound according to any one of embodiments 1 to 9, which inhibits the activation and/or the proliferation of dendritic cells.
**11.** The anti-CMKLR1 compound according to any one of embodiments 1 to 10, which has the capability *in vitro* and/or *in vivo* to induce the phosphorylation of Akt and/or Erk after activation of CMKLR1, in particular the phosphorylation of both Akt and Erk induced after activation of CMKLR1.
**12.** The anti-CMKLR1 compound according to any one of embodiments 1 to 11, which does not compete with Chemerin for the binding to CMKLR1, or which does not interfere with the binding of Chemerin to CMKLR1.
**13.** An anti-CMKLR1 compound selected from the group of an antibody, an antigen-binding fragment thereof or a chimeric, modified or humanized antibody, which specifically binds to CMKLR1, said compound comprising an antibody heavy chain variable domain comprising a VHCDR3 comprising or consisting of the amino acid sequences set forth in SEQ ID No: 8 or a mutated sequence thereof wherein amino acid residue(s) is(are) substituted providing amino acid residues at position 1 and 2 of SEQ ID No: 8 are respectively L and I or L; and
   wherein the anti-CMKLR1 compound specifically binds to an epitope localized within the third extra-cellular loop (EL3) of CMKLR1, in particular wherein the compound binds specifically to a polypeptide comprising or consisting of amino acid residues of sequence SEQ ID No: 2; and
   wherein the anti-CMKLR1 compound is a Resolvin E1-like agonist of CMKLR1, in particular wherein the anti-CMKLR1 compound is a pro-resolution factor, in particular on myeloid cell lineages; and
   wherein said compound competes with an antibody comprising the heavy variable domain corresponding to SEQ ID No: 9 and the light chain variable domain corresponding to SEQ ID No: 16, more particularly with antibody 2G1, for the binding to a polypeptide comprising or consisting of amino acid residues of sequence SEQ ID No: 2 or to a polypeptide comprising or consisting of the third loop (EL3) of the extracellular domain of CMKLR1.
**14.** The anti-CMKLR1 compound according to embodiment 13, wherein:
   - the antibody heavy chain variable domain comprises the three CDRs VHCDR1, VHCDR2 and VHCDR3, wherein:
      - VHCDR1 comprises or consists of the amino acid sequences set forth in SEQ ID No: 4; SEQ ID No: 62; SEQ ID No: 63; SEQ ID No: 64 or SEQ ID No: 65; and
      - VHCDR2 comprises or consists of the amino acid sequences set forth in SEQ ID No: 6; SEQ ID No: 66; SEQ ID No: 67; SEQ ID No: 68; SEQ ID No: 69; SEQ ID No: 70; SEQ ID No: 71 or SEQ ID No: 72; and
      - VHCDR3 comprises or consists of the amino acid sequences set forth in SEQ ID No: 8; SEQ ID No: 73; SEQ ID No: 74 or SEQ ID No: 75; and
   further comprising an antibody light chain variable domain comprising the three CDRs VLCDR1, VLCDR2 and VLCDR3, wherein:
   - VLCDR1 comprises or consists of the amino acid sequences set forth in; SEQ ID No: 12; SEQ ID No: 76; SEQ ID No: 77; SEQ ID No: 78; SEQ ID No: 79 or SEQ ID No: 80; and
   - VLCDR2 comprises or consists of the amino acid sequences set forth in SEQ ID No: 14; SEQ ID No: 81; SEQ ID No: 82; SEQ ID No: 83; SEQ ID No: 84; SEQ ID No: 85; SEQ ID No: 86; SEQ ID No: 87 or SEQ ID No: 88; and
   - VLCDR3 comprises or consists of the amino acid sequences set forth in SEQ ID No: 15 or SEQ ID No: 89.
**15.** A nucleic acid molecule, or a set of nucleic acid molecules, more particularly an isolated nucleic acid molecule(s) and/or a recombinant nucleic acid molecule(s), which encode(s) an anti-CMKLR1 compound according to any one of embodiments 1 to 14, more particularly a nucleic acid molecule or a set of nucleic acid molecules which encode(s) a heavy chain variable domain comprising or consisting of the amino acid residues of sequences set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41 or SEQ ID No: 42 and/or a light chain variable domain comprising or consisting of the amino acid residues of sequences set forth in SEQ ID No: SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.
**16.** The nucleic acid molecule or set of nucleic acid molecules according to embodiment 15, wherein the nucleic acid molecule(s) further comprise(s) regulation sequences for transcription and expression of the encoded heavy chain variable domain and/or the light chain variable domain.
**17.** A vector comprising or consisting of the operably linked nucleic acid molecule or set of nucleic acid molecules according to embodiment 15 or 16, the vector being in particular a recombinant vector and/or an isolated vector.
**18.** The vector according to embodiment 17, which is a plasmid, an artificial chromosome, a cosmid or a viral vector.
**19.** A host cell comprising the acid molecules according to embodiment 15 or 16, or a vector according to embodiment 17 or 18.
**20.** The host cell according to embodiment 19 which is a mammalian cell, in particular a Chinese Hamster Ovary (CHO) cell.
**21.** An anti-CMKLR1 compound selected from the group consisting of an anti-CMKLR1 according to any one of embodiments 1 to 14, a nucleic acid molecule or a set of nucleic acid molecules according to embodiment 15 or 16, a vector according to embodiment 17 or 18, and/or a host cell according to embodiment 19 or 20;
   for use in the prevention and/or the treatment of an inflammatory disease, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis, in particular wherein the resolution of inflammation is delayed or disrupted.
**22.** The anti-CMKLR1 compound for use according to embodiment 21, in the treatment of an inflammatory bowel disease, in particular colitis or Crohn's disease, in a subject whose myeloid cells over-express CMKLR1, in particular in a subject who is non-responder to corticosteroids and/or immunosuppressive treatment.
**23.** The anti-CMKLR1 compound for use according to embodiment 21 or 22 in the prolonged treatment of colitis and/or the treatment of advanced colitis.
**24.** An anti-CMKLR1 compound selected from the group consisting of an anti-CMKLR1 according to any one of embodiments 1 to 14, a nucleic acid molecule or a set of nucleic acid molecules according to embodiment 15 or 16, a vector according to embodiment 17 or 18, and/or a host cell according to embodiment 19 or 20;
   for use in the prevention and/or the treatment of an autoimmune disease such as diabetes, in particular type I diabetes, psoriasis, lupus, rheumatoid arthritis, multiple sclerosis, Sjögren's syndrome, celiac disease, vasculitis, myasthenia gravis, or an infection disease such as sepsis, peritonitis, degenerative diseases, wound healing disorders or dry eye syndrome, in particular wherein the resolution of inflammation is delayed or disrupted.
**25.** An anti-CMKLR1 compound selected from the group consisting of an anti-CMKLR1 according to any one of embodiments 1 to 14, a nucleic acid molecule or a set of nucleic acid molecules according to embodiment 15 or 16, a vector according to embodiment 17 or 18, and/or a host cell according to embodiment 19 or 20;
   for use in the prevention and/or the treatment of a cancer, metastatic cancers, in particular solid and liquid cancers such as carcinoma, more particularly hepatocarcinoma, in particular mammary carcinoma or colon carcinoma, or lung cancer or myeloid cancer such as leukemia, in particular a cancer wherein cancer cells express CMKLR1 or where the microenvironment of the tumor is invaded by cells expressing or overexpressing CMKLR1, in particular wherein the resolution of inflammation is delayed or disrupted.
**26.** An anti-CMKLR1 compound selected from the group consisting of an anti-CMKLR1 according to any one of embodiments 1 to 14, a nucleic acid molecule or a set of nucleic acid molecules according to embodiment 15 or 16, a vector according to embodiment 17 or 18, and/or a host cell according to embodiment 19 or 20;
   for use in the prevention and/or the treatment of a cancer, metastatic cancers, in particular solid and liquid cancers such as carcinoma, more particularly hepatocarcinoma, in particular mammary carcinoma or colon carcinoma, or lung cancer or myeloid cancer such as leukemia, in a cancer wherein cancer cells express CMKLR1 or where the microenvironment of the tumor is invaded by cells expressing or overexpressing CMKLR1.
**27.** A combination product comprising:
   - at least one anti-human CMKLR1 compound as defined in any one of embodiments to 14, a nucleic acid molecule or a set of nucleic acid molecules according to embodiment 15 or 16, a vector according to embodiment 17 or 18, and/or a host cell according to embodiment 19 or 20; and
   - at least one second therapeutic agent selected from the group consisting of chemotherapeutic agents, radiotherapy agents, immunotherapeutic agents, cell therapy agents, antibiotics and probiotics; in particular immunotherapeutic agents selected from the group consisting of checkpoint blocker or activator of adaptive immune cells, particularly selected from the group consisting of anti-PDL1, anti-PD1, anti-CTLA4, anti-SIRPa, anti-CD137, anti-CD2, anti-CD28, anti-CD40, anti-HVEM, anti-BTLA, anti-CD160, anti-TIGIT, anti-TIM-1/3, anti-LAG-3, anti-2B4, and anti-OX40, anti-CD40 agonist, CD40-L, TLR agonists, anti-ICOS, ICOS-L and B-cell receptor agonists, in particular anti-PD1, anti-PDL1, anti-SIRPa and/or anti-CD137; for simultaneous, separate or sequential use as a medicament.
**28.** A combination of compound comprising an anti-CMKLR1 compound as defined in any one of embodiments 1 to 14, a nucleic acid molecule or a set of nucleic acid molecules according to embodiment 15 or 16, a vector according to embodiment 17 or 18, and/or a host cell according to embodiment 19 or 20;
   and an anti-PD1 or anti-PDL1 compound selected among the group consisting of an antibody, an antigen-binding fragment thereof, a humanized antibody, a chimeric antibody, a modified antibody or antigen-binding antibody mimetic.
**29.** The combination of compound according to embodiment 28 comprising an anti-CMKLR1 compound according to any one of embodiments 1 to 11, and an anti-PD1 antibody, more particularly wherein the anti-PD1 compound is a monoclonal anti-PD1 antibody.
**30.** A combination of compound comprising an anti-CMKLR1 compound as defined in any one of embodiments 1 to 14, a nucleic acid molecule or a set of nucleic acid molecules according to embodiment 15 or 16, a vector according to embodiment 17 or 18, and/or a host cell according to embodiment 19 or 20;
   and an anti-SIRPa compound selected among the group consisting of an antibody, an antigen-binding fragment thereof, a humanized antibody, a chimeric antibody, a modified antibody or antigen-binding antibody mimetic, more particularly an anti-SIRPa antibody or an antigen-binding fragment thereof.
**31.**A method for selecting an anti-CMKLR1 compound, said method comprising the following steps:
   a) Providing a compound selected from the group of an antibody or an antigen-binding fragment thereof or a chimeric or humanized antibody; said compound comprising:
      - an antibody heavy chain variable domain comprising the three CDRs VHCDR1, VHCDR2 and VHCDR3, wherein:
         - VHCDR1 comprises or consists of the amino acid sequences set forth in SEQ ID No: 4 or a mutated sequence thereof wherein amino acid residue(s) is(are) substituted; and
         - VHCDR2 comprises or consists of the amino acid sequences set forth in SEQ ID No: 6 or a mutated sequence thereof wherein amino acid residue(s) is(are) substituted; and
         - VHCDR3 comprises or consists of the amino acid sequences set forth in SEQ ID No: 8 or a mutated sequence thereof wherein amino acid residue(s) is(are) substituted providing amino acid residues at position 1 and 2 of SEQ ID No: 8 are respectively L and I or L; and
      - an antibody light chain variable domain comprising the three CDRs VLCDR1, VLCDR2 and VLCDR3, wherein:
         - VLCDR1 comprises or consists of the amino acid sequences set forth in SEQ ID No: 12 or a mutated sequence thereof wherein amino acid residue(s) is(are) substituted; and
         - VLCDR2 comprises or consists of the amino acid sequences set forth in SEQ ID No: 14 or a mutated sequence thereof wherein amino acid residue(s) is(are) substituted; and
         - VLCDR3 comprises or consists of the amino acid sequences set forth in SEQ ID No: 15 or a mutated sequence thereof wherein the amino acid residue(s) is(are) substituted;
   b) Testing the capability of the compound to be a RvE1-like agonist of CMKLR1, in particular testing the capability of the compound to favor the resolution of inflammation; in particular on myeloid cells; and optionally
   c) Testing the binding capability by Biosensor of the compound to an epitope localized within the third loop E3 of CMKLR1, in particular the binding capability of the compound to a polypeptide comprising the amino acid residues of SEQ ID No: 2 or SEQ ID No 152; and optionally
   d) Testing the ability of the compound to not compete with the binding of chemerin to CMKLR1;
   e) And when the binding capability of the compound tested in step c) is at least 10E-8 KD,
      i) Testing the capability of the compound to induce the phosphorylation of Akt and/or the phosphorylation of Erk after binding of the compound to CMKLR1, in particular the phosphorylation of both Akt and Erk;
      ii) Testing the capability of the compound to enhance the secretion of anti-inflammatory cytokines, in particular IL10 and/or CCL17; and/or the capability of the compound to inhibit or reduce the secretion of pro-inflammatory cytokines, in particular IL12; in particular on myeloid cells expressing CMKLR1; and/or
      iii) Testing the capability of the compound to enhance the macrophage polarization to favor anti-inflammatory M2 type macrophages; in particular the capability of the compound to enhance the secretion of the phenotype marker CD200R; and/or
      iv) Testing the capability of the compound to inhibit the dendritic cells activation and/or proliferation.
**32.** An agonist compound of CMKLR1 having a RvE1-like agonist capability, which induces the phosphorylation of Akt and/or the phosphorylation of Erk after binding of the compound to CMKLR1, in particular on myeloid cells, said compound binding specifically to the third loop E3 of CMKLR1, in particular to an epitope localized within the third loop E3 and comprised within amino acid residues of sequence SEQ ID No: 2 or SEQ ID No: 152;
   said compound being an antibody, an antigen-binding antibody, an antigen-binding antibody mimetic; a modified antibody, an extracellular ligand, a peptide or a polypeptide.
**33.** The agonist compound of embodiment 32, which enhances the macrophage polarization to favor anti-inflammatory M2 type macrophages, and/or which inhibits the proliferation and of the activation of dendritic cells, which enhances *in vitro* and/or *in vivo* the secretion of anti-inflammatory cytokines, in particular IL10 and/or CCL17;
   and/or which inhibits or reduces *in vitro* and/or *in vivo* the secretion of pro-inflammatory cytokines, in particular IL12; in particular by myeloid cells expressing CMKLR1.

## Claims

1. An anti-CMKLR1 compound selected from the group of an antibody or an antigen-binding fragment thereof or a chimeric, humanized, or modified antibody which specifically binds to CMKLR1, said compound comprising an antibody heavy chain variable domain and an antibody light chain variable domain, wherein the heavy chain variable domain comprises the amino acid sequences set forth in SEQ ID No: 20; SEQ ID No: 21; SEQ ID No: 22; SEQ ID No: 23; SEQ ID No: 24; SEQ ID No: 25; SEQ ID No: 26; SEQ ID No: 27; SEQ ID No: 28: SEQ ID No; 29; SEQ ID No: 30; SEQ ID No: 31; SEQ ID No: 32; SEQ ID No: 33; SEQ ID No: 34; SEQ ID No: 35; SEQ ID No: 36; SEQ ID No: 37; SEQ ID No: 38; SEQ ID No: 39; SEQ ID No: 40; SEQ ID No: 41, or SEQ ID No: 42; and wherein the light chain variable domain comprises the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60, or SEQ ID No: 61.

2. The anti-CMKLR1 compound according to claim 1, wherein the heavy chain variable domain comprises the amino acid sequences set forth in SEQ ID No: 24; and wherein the light chain variable domain comprises the amino acid sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

3. The anti-CMKLR1 compound according to claim 1 or claim 2, which binds specifically to a polypeptide comprising the sequence of amino acid residues of SEQ ID No: 2.

4. The anti-CMKLR1 compound according to any one of claims claim 1 to 3, which is an agonist of the interaction between RvE1 and CMKLR1, in particular wherein the anti-CMKLR1 compound is an anti-human CMKLR1 compound.

5. A nucleic acid molecule, or a set of nucleic acid molecules, more particularly an isolated nucleic acid molecule(s) and/or a recombinant nucleic acid molecule(s), which encode(s) an anti-CMKLR1 compound according to any one of claims 1 to 4.

6. The nucleic acid molecule, or a set of nucleic acid molecules, according to claim 5, which encode(s) a heavy chain variable domain comprising the amino acid residues of sequences set forth in SEQ ID No: 24 and/or a light chain variable domain comprising the amino acid residues of sequences set forth in SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51: SEQ ID No: 52; SEQ ID No: 53; SEQ ID No: 54; SEQ ID No: 55; SEQ ID No: 56; SEQ ID No: 57; SEQ ID No: 58; SEQ ID No: 59; SEQ ID No: 60 or SEQ ID No: 61.

7. The nucleic acid molecule or set of nucleic acid molecules according to claim 5 or 6, wherein the nucleic acid molecule(s) further comprise(s) regulation sequences for transcription and expression of the encoded heavy chain variable domain and/or the light chain variable domain.

8. A vector comprising the operably linked nucleic acid molecule or set of nucleic acid molecules according to any one of claims 5 to 7, the vector being in particular a recombinant vector and/or an isolated vector.

9. The vector according to claim 8, which is a plasmid, an artificial chromosome, a cosmid, or a viral vector.

10. A host cell comprising the nucleic acid molecules according to any one of claims 5 to 7 or a vector according to claim 8 or 9.

11. The host cell according to claim 10 which is a mammalian cell, in particular a Chinese Hamster Ovary (CHO) cell.

12. An anti-CMKLR1 compound selected from an anti-CMKLR1 according to any one of claims 1 to 4, a nucleic acid molecule or a set of nucleic acid molecules according to any one of claims 5 to 7, a vector according to claim 8 or 9, and/or a host cell according to claim 10 or 11, for use in the prevention and/or treatment of a disease selected from inflammatory diseases, autoimmune diseases, and cancer diseases.

13. An anti-CMKLR1 compound selected from an anti-CMKLR1 according to any one of claims 1 to 4, a nucleic acid molecule or a set of nucleic acid molecules according to any one of claims 5 to 7, a vector according to claim 8 or 9, and/or a host cell according to claim 10 or 11,
for use in the prevention and/or the treatment of an inflammatory disease selected from acute inflammatory diseases, chronic inflammatory diseases such as asthma, keratoconjunctivitis, periodontal disease, eczema, inflammatory bowel disease, in particular Crohn's disease and colitis, in particular ulcerative colitis or spontaneous colitis, in particular wherein the resolution of inflammation is delayed or disrupted.

14. An anti-CMKLR1 compound selected from an anti-CMKLR1 according to any one of claims 1 to 4, a nucleic acid molecule or a set of nucleic acid molecules according to any one of claims 5 to 7, a vector according to claim 8 or 9, and/or a host cell according to claim 10 or 11;
for use in the prevention and/or the treatment of solid and liquid cancers such as carcinoma, more particularly hepatocarcinoma, in particular mammary carcinoma or colon carcinoma, or lung cancer or myeloid cancer such as leukemia, in particular a cancer wherein cancer cells express CMKLR1 or where the microenvironment of the tumor is invaded by cells expressing or overexpressing CMKLR1, in particular wherein the resolution of inflammation is delayed or disrupted.

15. An anti-CMKLR1 compound selected from an anti-CMKLR1 according to any one of claims 1 to 4, a nucleic acid molecule or a set of nucleic acid molecules according to any one of claims 5 to 7, a vector according to claim 8 or 9, and/or a host cell according to claim 10 or 11;
for use in the prevention and/or the treatment of an autoimmune disease such as diabetes, in particular type I diabetes, psoriasis, lupus, rheumatoid arthritis, multiple sclerosis, Sjögren's syndrome, celiac disease, vasculitis, myasthenia gravis, or an infection disease such as sepsis, peritonitis, degenerative diseases, wound healing disorders or dry eye syndrome, in particular wherein the resolution of inflammation is delayed or disrupted.
